(19) Europäisches Patentamt
European Patent Office
Office européen des brevets

(11) EP 4 212 503 A1

(12) # EUROPEAN PATENT APPLICATION
published in accordance with Art. 153(4) EPC

(43) Date of publication:
**19.07.2023 Bulletin 2023/29**

(21) Application number: **21866696.4**

(22) Date of filing: **06.09.2021**

(51) International Patent Classification (IPC):
***C07C 51/09*** *(2006.01)* ***C07C 53/18*** *(2006.01)*
***C07C 53/21*** *(2006.01)* ***H01G 9/035*** *(2006.01)*
***H01G 9/20*** *(2006.01)* ***H01G 11/06*** *(2013.01)*
***H01G 11/64*** *(2013.01)* ***H01M 6/16*** *(2006.01)*
***H01M 8/02*** *(2016.01)* ***H01M 10/052*** *(2010.01)*
***H01M 10/0567*** *(2010.01)*

(52) Cooperative Patent Classification (CPC):
**C07C 51/09; C07C 53/18; C07C 53/21; C07F 1/02; H01G 9/035; H01G 9/20; H01G 11/06; H01G 11/64; H01M 6/16; H01M 8/02; H01M 10/052; H01M 10/0567;** Y02E 60/10; Y02E 60/50; Y02P 70/50

(86) International application number:
**PCT/JP2021/032577**

(87) International publication number:
**WO 2022/054737 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.09.2020 JP 2020150884**

(71) Applicant: **Daikin Industries, Ltd.**
**Osaka-shi, Osaka 530-0001 (JP)**

(72) Inventors:
- **SHIRAI, Atsushi**
  **Osaka-shi, Osaka 530-0001 (JP)**
- **YAMAZAKI, Shigeaki**
  **Osaka-shi, Osaka 530-0001 (JP)**
- **KUROKI, Yoshichika**
  **Osaka-shi, Osaka 530-0001 (JP)**
- **OHTSUKA, Tatsuya**
  **Osaka-shi, Osaka 530-0001 (JP)**

(74) Representative: **Hoffmann Eitle**
**Patent- und Rechtsanwälte PartmbB**
**Arabellastraße 30**
**81925 München (DE)**

(54) **FLUORINATED CARBOXYLIC ACID SALT COMPOUND**

(57) A method for producing a compound represented by formula (P1): $(B^{1f})_{mp}(A^1)_{np}$,
wherein
$B^{1f}$ is RfCOO,
Rf is a hydrocarbon having one or more fluorine atoms,
$A^1$ is a group excluding H,
mp is (valence of $A^1$) x np and is 1 or 2,
np is mp/(valence of $A^1$) and is 1,
$A^1$ has a valence of 1 or 2,

the method comprising step A of reacting
a compound represented by formula (S1): $(B^{1f})$ $(R^1)$,
wherein
$B^{1f}$ is as defined above, and
$R^1$ is an organic group,
and
a compound represented by formula (S2): $(A^1)_{ms2}(B^2)_{ns2}$,
wherein
$A^1$ is as defined above,
$B^2$ is OH, $CO_3$, or $HCO_3$,
ms2 is (valence of $B^2$) x ns2/ (valence of $A^1$) and is 1 or 2, and
ns2 is (valence of $A^1$) x ms2/ (valence of $B^2$) and 1 or 2,
or a hydrate thereof.

Such a method is a novel production method of a fluorinated carboxylic acid salt compound (preferably a fluorinated carboxylic acid salt compound having a low water content).

EP 4 212 503 A1

**Description**

Technical Field

The present disclosure relates to a fluorinated carboxylic acid salt compound.

Background Art

The fluorinated carboxylic acid salt compound is an effective compound used by an electrochemical device etc. (Patent Literature 1)

A method for synthesizing a fluorinated carboxylic acid lithium by reacting a fluorinated carboxylic acid halide with hydroxide lithium or by reacting a fluorinated carboxylic acid with hydroxide lithium or carbonic acid lithium is well known (Patent Literature 2).

Citation List

Patent Literature

PTL 1: JP2004-22379A
PTL 2: JP2011-93808A

Summary of Invention

Technical Problem

In the previous method for producing a fluorinated carboxylic acid salt compound, water, which was difficult to remove, was produced as a by-product.

The present disclosure aims to provide a novel production method of a fluorinated carboxylic acid salt compound (preferably a fluorinated carboxylic acid salt compound having a low water content).

Solution to Problem

The above problem can be solved by a method for producing a compound represented by formula (P1): $(B^{1f})_{mp}(A^1)_{np}$, wherein

$B^{1f}$ is RfCOO,
Rf is a hydrocarbon having one or more fluorine atoms, $A^1$ is a group excluding H,
mp is the number of (valence of $A^1$) x np/ (valence of $B^1$) and is 1 or 2,
np is the number of (valence of $B^1$) x mp/ (valence of $A^1$) and is 1,
$A^1$ has a valence of 1 or 2, and
$B^{1f}$ has a valence of 1,

the method comprising step A of reacting
a compound represented by formula (S1):$(B^{1f})(R^1)$,
wherein

$B^{1f}$ is as defined above, and
$R^1$ is an organic group,
with the proviso that $R^1$ is a different group from $A^1$, and

a compound represented by formula (S2) : $(A^1)_{ms2}(B^2)_{ns2}$,
wherein

$A^1$ is as defined above,
$B^2$ is OH, $CO_3$, or $HCO_3$,
ms2 is the number of (valence of $B^2$) x ns2/ (valence of $A^1$) and is 1 or 2, and

ns2 is the number of (valence of $A^1$) x ms2/ (valence of $B^2$) and 1 or 2,

or a hydrate thereof.

The present disclosure provides the following aspects.

Item 1.

A method for producing a compound represented by formula (P1):

$$(B^{1f})_{mp}(A^1)_{np},$$

wherein

$B^{1f}$ is RfCOO,
Rf is a hydrocarbon having one or more fluorine atoms, $A^1$ is a group excluding H,
mp is the number of (valence of $A^1$) x np/ (valence of $B^{1f}$) and is 1 or 2,
np is the number of (valence of $B^{1f}$) x mp/ (valence of $A^1$) and is 1,
$A^1$ has a valence of 1 or 2, and
$B^{1f}$ has a valence of 1,

the method comprising step A of reacting
a compound represented by formula (S1):$(B^{1f})(R^1)$,
wherein

$B^{1f}$ is as defined above, and
$R^1$ is an organic group,
with the proviso that $R^1$ is a different group from $A^1$, and

a compound represented by formula (S2):$(A^1)_{ms2}(B^2)_{ns2}$,
wherein

$A^1$ is as defined above,
$B^2$ is OH, $CO_3$, or $HCO_3$,
ms2 is the number of (valence of $B^2$) x ns2/ (valence of $A^1$) and is 1 or 2, and
ns2 is the number of (valence of $A^1$) x ms2/ (valence of $B^2$) and 1 or 2,

or a hydrate thereof.

Item 2.

The production method according to Item 1, wherein $A^1$ is a metal atom or ammonium.

Item 3.

The production method according to Item 1 or 2, wherein the method comprises step B of removing a compound represented by formula (P2):$(R^1)_{op}(B^2)$, which has been produced as a by-product in step A,
wherein

$B^2$ and $R^1$ are as defined above, and
op is the number of (valence of $B^2$)/(valence of $R^1$) and is 1 or 2.

Item 4.

The production method according to any one of Items 1 to 3, wherein Rf is a $C_{1-6}$ alkyl group having one or more fluorine atoms.

Item 5.

The production method according to any one of Items 1 to 4, wherein Rf is a $C_{1-3}$ alkyl group having two or more fluorine atoms.

Item 6.

The production method according to any one of Items 1 to 5, wherein $A^1$ is an alkali metal atom.

Item 7.

The production method according to any one of Items 1 to 6, wherein $A^1$ is Li.

Item 8.

The production method according to any one of Items 1 to 7, wherein $B^2$ is OH.

Item 9.

An additive for an electrochemical device, comprising

a compound represented by formula (P1A):$RfCOOA^d$,
wherein
Rf is an organic group having one or more fluorine atoms, and
$A^d$ is a metal atom, and
water in an amount of 0 to 50000 mass ppm relative to the compound represented by formula (P1A).

Item 10.

An electrolyte solution comprising the additive for an electrochemical device according to Item 9.

Item 11.

An electrochemical device, comprising the electrolyte solution according to Item 10.

Item 12.

A lithium ion secondary battery, comprising the electrolyte solution according to Item 10.

Item 13.

A compound represented by formula (P1A): $RfCOOA^d$,
wherein

Rf is an organic group having one or more fluorine atoms, and $A^d$ is a Li atom,
wherein the water content is 50000 mass ppm or less.

Item 14.

A composition comprising

a compound represented by formula (P1A): $RfCOOA^d$,
wherein
Rf is an organic group having one or more fluorine atoms, and $A^d$ is a Li atom, and
water in an amount of 50000 mass ppm or less relative to the compound represented by formula (P1).

Item 15.

The compound according to Item 13, which is produced by the production method according to any one of Items 1 to 8.

Item 16.

The composition according to Item 14, which is produced by the production method according to any one of Items 1 to 8.

Advantageous Effects of Invention

In the production method, which is an embodiment of the present disclosure, water is not produced as a by-product. By this, preferably a fluorinated carboxylic acid salt compound having a low water content, and more preferably a fluorinated carboxylic acid salt compound substantially free of water, can be industrially advantageously provided.
The electrochemical device or the lithium ion secondary battery according to one embodiment of the present disclosure, using an electrolyte solution containing such a fluorinated carboxylic acid salt compound may have industrial advantages (relatively low production costs, relatively high performance (e.g., relatively high cycle capacity retention rate, or relatively low resistance increase rate).

Description of Embodiments

Terms

The symbols and abbreviations in the present specification can be understood in the sense commonly used in the technical field to which the present invention pertains in the context of the present specification, unless otherwise specified.
In the present specification, the terms "comprise" and "contain" are used with the intention of including the phrases "consisting essentially of" and "consisting of.
Unless otherwise specified, the steps, treatments, or operations described in the present specification can be performed at room temperature.
In the present specification, room temperature can mean a temperature in the range of 10 to 40°C.
In the present specification, the phrase "Cn-Cm" (wherein n and m each represent a number) indicates that the number of carbon atoms is n or more and m or less, as a person skilled in the art would generally understand.
In the present specification, unless otherwise specified, examples of the "halo (group)" may include fluoro (group), chloro (group), bromo (group), and iodine (group).
In the present specification, unless otherwise specified, examples of the "halogen (atom)" may include fluorine (atom), chlorine (atom), bromine (atom), and iodine (atom).
In the present specification, the "organic group" refers to a group containing one or more carbon atoms (or a group formed by removing one hydrogen atom from an organic compound).
In the present specification, unless otherwise specified, a "hetero atom" can mean an atom other than hydrogen and carbon.
In the present specification, unless otherwise specified, examples of the "hetero atom" include a nitrogen atom, an oxygen atom, and a sulfur atom.
In the present specification, unless otherwise specified, the "organic group" refers to a group containing one or more carbon atoms as a constituent element.
In the present specification, unless otherwise specified, examples of the "organic group" include

(1) hydrocarbyl (which may have one or more substituent groups), or
(2) a group in which one or more heteroatoms are inserted to hydrocarbyl (which may have one or more substituent groups) (in the present specification, hereinbelow sometimes referred to as "heterohydrocarbyl").

Examples of the substituent include halo, nitro, cyano, oxo, thioxo, sulfo, sulfamoyl, sulfinamoyl, and sulfenamoyl.
In the present specification, unless otherwise specified, the "hydrocarbyl (group)" include a group having one or more carbon atoms and one or more hydrogen atoms, as constituent elements, and preferably a group consisting of one or more carbon atoms and one or more hydrogen atoms, as constituent elements.
Examples of "(1) hydrocarbyl" include aliphatic hydrocarbyl groups (e.g., benzyl group) optionally substituted

with one or more aromatic hydrocarbyl groups, and aromatic hydrocarbyl groups optionally substituted with one or more aliphatic hydrocarbyl groups (e.g., aryl groups in the narrow sense).

In the present specification, the (hetero)aryl group include aryl groups in the narrow sense, and heteroaryl groups.

Examples of "(2) group in which one or more heteroatoms are inserted to a hydrocarbyl group (heterohydrocarbyl group)" include a 5- to 6-membered heteroaryl group, and groups in which a benzene ring is condensed to the 5- to 6-membered heteroaryl group, alkoxy group, ester group, ether group, and heterocyclic group.

In the present specification, unless otherwise specified, the "aliphatic hydrocarbyl (group)" can be a linear aliphatic hydrocarbyl group, a branched aliphatic hydrocarbyl group, a cyclic aliphatic hydrocarbyl group, or a combination thereof.

In the present specification, unless otherwise specified, the aliphatic hydrocarbyl group can be a saturated or unsaturated aliphatic hydrocarbyl group.

In the present specification, unless otherwise specified, examples of the "aliphatic hydrocarbyl (group)" include an alkyl group, an alkenyl group, an alkynyl group, and a cycloalkyl group.

In the present specification, unless otherwise specified, examples of the "alkyl (group)" include linear or branched alkyl groups having 1 to 10 carbon atoms, such as methyl, ethyl, propyl, isopropyl, butyl, isobutyl, sec-butyl, tert-butyl, pentyl, isopentyl, neopentyl, and hexyl.

In the present specification, unless otherwise specified, examples of the "alkenyl (group)" include linear or branched alkenyl groups having 1 to 10 carbon atoms, such as vinyl, 1-propenyl, isopropenyl, 2-methyl-1-propenyl, 1-butenyl, 2-butenyl, 3-butenyl, 2-ethyl-1-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, and 5-hexenyl.

In the present specification, unless otherwise specified, examples of the "alkynyl (group)" include linear or branched alkynyl groups having 2 to 6 carbon atoms, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, and 5-hexynyl.

In the present specification, unless otherwise specified, examples of the "cycloalkyl (group)" include cycloalkyl groups having 3 to 8 carbon atoms, such as cyclopentyl, cyclohexyl, and cycloheptyl.

In the present specification, unless otherwise specified, examples of the aromatic hydrocarbyl (group) (aryl (group)) include phenyl, naphthyl, phenanthryl, anthryl, and pyrenyl.

In the present specification, unless otherwise specified, the term "aralkyl (group)" can mean an alkyl group substituted with one aryl group, as is usually understood.

In the present specification, unless otherwise specified, an "alkoxy (group)" refers to, for example, a group represented by RO- (wherein R represents an alkyl group).

In the present specification, unless otherwise specified, an "ester group" refers to an organic group having an ester bond (i.e., $-CO_2-$, (specifically, -C(=O)-O- or -O-C(=O)-)).

Examples thereof include a group represented by formula: $RCO_2-$ (wherein R represents an alkyl group) and a group represented by formula: $R_a-CO_2-R_b-$ (wherein $R_a$ represents an alkyl group, and $R_b$ represents an alkylene group).

In the present specification, unless otherwise specified, the "ether group" refers to a group having an ether bond (-O-).

Examples of the "ether group" include polyether groups.

Examples of the "polyether group" include a group represented by formula: $R_a-(O-R_b)_n-$ (wherein $R_a$ represents an alkyl group, $R_b$, in each occurrence, may be the same or different and represents an alkylene group, and n represents an integer of 1 or more).

An alkylene group refers to a divalent group formed by removing one hydrogen atom from an alkyl group.

Examples of the "ether group" include hydrocarbyl ether groups.

A hydrocarbyl ether group refers to a hydrocarbyl group having one or more ether bonds. The "hydrocarbyl group having one or more ether bonds" can be a hydrocarbyl group into which one or more ether bonds are inserted. Examples thereof include a benzyloxy group.

Examples of the "hydrocarbyl group having one or more ether bonds" include an alkyl group having one or more ether bonds.

The "alkyl group having one or more ether bonds" can be an alkyl group having one or more ether bonds inserted. In the present specification, such a group may be referred to as "alkyl ether group."

In the present specification, unless otherwise specified, the "acyl (group)" includes an alkanoyl group. In the present specification, unless otherwise specified, an "alkanoyl (group)" is, for example, a group represented by RcCO- (wherein Rc represents an alkyl group).

In the present specification, unless otherwise specified, examples of 5- or 6-membered heteroaryl groups include 5- or 6-membered heteroaryl groups having one or more (e.g., one, two, or three) hetero atoms selected from the group consisting of oxygen, sulfur, and nitrogen (e.g., one, two, or three atoms) as a ring-constituting atom, such as pyrrolyl (e.g., 1-pyrrolyl, 2-pyrrolyl, and 3-pyrrolyl), furyl (e.g., 2-furyl, and 3-furyl), thienyl (e.g., 2-thienyl, and 3-thienyl),

pyrazolyl (e.g., 1-pyrazolyl, 3-pyrazolyl, and 4-pyrazolyl), imidazolyl (e.g., 1-imidazolyl, 2-imidazolyl, and 4-imidazolyl), isooxazolyl (e.g., 3-isooxazolyl, 4-isooxazolyl, and 5-isooxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-4-yl, and 1,2,4-triazol-3-yl), oxadiazolyl (e.g., 1,2,4-oxadiazol-3-yl, and 1,2,4-oxadiazol-5-yl), thiadiazolyl (e.g., 1,2,4-thiadiazol-3-yl, and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), and pyrazinyl.

Production Method of Compound

Step A

The production method according to one embodiment of the present disclosure is a method for producing a compound represented by formula (P1):$(B^{1f})_{mp}(A^1)_{np}$,
wherein

$B^{1f}$ is RfCOO,
Rf is a hydrocarbon group having one or more fluorine atoms,
$A^1$ is a group excluding H,
mp is the number of (valence of $A^1$) x np/(valence of B1) and is 1 or 2,
np is the number of (valence of $B^1$) x mp/ (valence of $A^1$) and is 1, $A^1$ has a valence of 1 or 2, and
$B^{1f}$ has a valence of 1,
the method comprising step A of reacting a compound represented by formula (S1):$(B^{1f})(R^1)$,
wherein
$B^{1f}$ is as defined above,
$R^1$ is an organic group,
with the proviso that $R^1$ is a different group from $A^1$, and a compound represented by formula (S2) : $(A^1)_{ms2}(B^2)_{ns2}$,
wherein
$A^1$ is as defined above,
$B^2$ is OH, $CO_3$, or $HCO_3$,
ms2 is the number of (valence of $B^2$) x ns2/ (valence of $A^1$) and is 1 or 2, and
ns2 is the number of (valence of $A^1$) x ms2/ (valence of $B^2$) and 1 or 2,
or a hydrate thereof.

In terms of the reaction addition rate, and the selectivity and yield of the target product, $A^1$ is preferably a metal atom or ammonium.
In terms of the reaction addition rate, and the selectivity and yield of the target product, $A^1$ is preferably an alkali metal atom.
In terms of the reaction addition rate, and the selectivity and yield of the target product, the metal atom is preferably an alkali metal atom.
In terms of the reaction addition rate, and the selectivity and yield of the target product, the metal atom is more preferably Li, Na, or K.
In terms of the reaction addition rate, and the selectivity and yield of the target product, the metal atom is still more preferably Li.
In terms of the reaction addition rate, and the selectivity and yield of the target product, Rf is preferably an alkyl group having one or more fluorine atoms.
In terms of the reaction addition rate, and the selectivity and yield of the target product, Rf is more preferably a $C_{1-6}$ alkyl group having one or more fluorine atoms.
In terms of the reaction addition rate, and the selectivity and yield of the target product, Rf is still more preferably a $C_{1-3}$ alkyl group having two or more fluorine atoms.
In terms of the reaction addition rate, and the selectivity and yield of the target product, $B^2$ is preferably OH.
In formula (P1), mp is the number of (valence of $A^1$) x np/(valence of $B^1$) and is 1 or 2, and np is the number of (valence of $B^1$) x mp/ (valence of $A^1$) and is 1. Specifically, mp is the number of (valence of $A^1$)/ (valence of $B^1$) and is 1 or 2, and np is 1.
In formula (S2), ms2 is the number of (valence of $B^2$) x ns2/(valence of $A^1$) and 1 or 2, and ns2 is (valence of $A^1$) x ms2/ (valence of $B^2$) and 1 or 2.
The operations of step A may be performed by employing the conventional technique of chemical reaction.
In terms of the reaction addition rate, and the selectivity and yield of the target product, the operation can be

preferably performed, for example, by adding the compound (S2) to the compound (S1) placed in the reactor, at once or in divided portions.

In terms of the reaction addition rate, and the selectivity and yield of the target product, the operation can be preferably performed, for example, by adding the compound (S1) to the compound (S2) placed in the reactor, at once or in divided portions.

From the viewpoint of easily obtaining a fluorinated carboxylic acid salt compound having a low water content, preferably a fluorinated carboxylic acid salt compound containing substantially free of water, it is preferable to use the anhydrous salt as the compound (S2). A commercially available anhydrous salt of the compound (S2) may be used, or the compound (S2) may be used after dehydration (anhydrous chloride) by a known method.

In step A, a solvent may be used.

From the viewpoint of easily obtaining a fluorinated carboxylic acid salt compound having a low water content, and preferably, a fluorinated carboxylic acid salt compound substantially free of water, any solvent other than water can be used as the solvent.

Specific examples of the solvent include

(1) alcohol solvents, such as methanol, ethanol, n-propanol, isopropanol, n-butanol, pentanol, hexanol, ethylene glycol, diethylene glycol, triethylene glycol, tetraethylene glycol, polyethylene glycol, propylene glycol, dipropylene glycol, tripropylene glycol, polypropylene glycol, trimethylene glycol, and hexanetriol;
(2) non-aromatic hydrocarbon solvents, such as pentane, hexane, heptane, octane, cyclohexane, decahydronaphthalene, n-decane, isododecane, and tridecane;
(3) aromatic hydrocarbon solvents, such as benzene, toluene, xylene, tetralin, veratrole, ethylbenzene, diethylbenzene, methylnaphthalene, anisole, phenetole nitrobenzene, o-nitrotoluene, mesitylene, indene, diphenyl sulfide, anisole, and propiophenone;
(4) ketone solvents, such as acetone, methyl ethyl ketone, diethyl ketone, hexanone, methyl isobutyl ketone, heptanone, diisobutyl ketone, acetonylacetone, methylhexanone, acetophenone, cyclohexanone, diacetone alcohol, propiophenone, and isophorone;
(5) halogenated hydrocarbon solvents, such as dichloromethane, chloroform, and chlorobenzene;
(6) ether solvents, such as diethyl ether, tetrahydrofuran (THF), diisopropyl ether, methyl-t-butyl ether (MTBE), dioxane, dimethoxyethane, diglyme, anisole, phenetole, 1,1-dimethoxy cyclohexane, diisoamyl ether, and cyclopentyl methyl ether (CPME);
(7) ester solvents, such as ethyl acetate, isopropyl acetate, diethyl malonate, 3-methoxy-3-methylbutyl acetate, γ-butyrolactone, ethylene carbonate, propylene carbonate, dimethyl carbonate, and α-acetyl-γ-butyrolactone;
(8) nitrile solvents, such as acetonitrile, and benzonitrile;
(9) sulfoxide solvents, such as dimethyl sulfoxide, and sulfolane; and
(10) amide solvents, such as N,N-dimethylformamide (DMF), N,N-dimethylacetamide, N-methylpyrrolidone (NMP), 1,3-dimethyl-2-imidazolidinone (DMI), N,N-dimethylacrylamide, N,N-dimethylacetoacetamide (DMA), N,N-diethylformamide, and N,N-diethylacetamide.

The solvents can be used alone or in a combination of two or more.

In terms of the reaction addition rate, and the selectivity and yield of the target product, the temperature of step A is preferably 120°C or less, more preferably 110°C or less, still more preferably 100°C or less, even more preferably 90°C or less, particularly preferably 80°C or less.

In terms of the reaction addition rate, and the selectivity and yield of the target product, the temperature of step A is preferably -50°C or more, more preferably -20°C or more, still more preferably -10°C or more, even more preferably 0°C or more, and particularly preferably 5°C or more.

The time of step A is not particularly limited. For example, step A is performed until the reaction ends.

The conditions of step A can be set to high temperature and high pressure, e.g., by using a pressure cooker ((e.g., an autoclave). In such a case, the reaction time may be shortened accordingly.

Step A may be performed under an inert gas (e.g., nitrogen gas) atmosphere.

Step A may be performed under atmospheric conditions.

Of these, from the viewpoint of easily obtaining a fluorinated carboxylic acid salt compound having a low water content, and preferably, a fluorinated carboxylic acid salt compound substantially free of water, step A can be preferably performed under an inert gas atmosphere.

After step A, before step B of removing the compound (P2) represented by formula (P2):$(R^1)_{op}(B^2)$, wherein $B^2$ and $R^1$ are as defined above, and op is the number of (valence of $B^2$)/(valence of $R^1$) and is 1 or 2, wherein the compound is a by-product produced in step A. As desired, for example, for the removal of an unnecessary product other than the compound (P2), treatments such as adsorption removal, filtration, and/or decantation may be performed.

Step B

The production method according to one embodiment of the present disclosure further comprises step B of removing the compound represented by formula (P2):$(R^1)_{op}(B^2)$,
wherein $B^2$ and $R^1$ are as defined above, and op is the number of (valence of $B^2$)/(valence of $R^1$) and is 1 or 2, wherein the compound is a by-product produced in step A.
The removal of the compound (P2) in step B can be preferably performed by, for example, distillation (which may be performed under reduced pressure), filtration, or decantation, or by a combination thereof.
The distillation can be preferably performed under reduced pressure.
The conditions of the distillation can be suitably determined by a person skilled in the art according to the technical knowledge in the field of chemistry.
For example, after or during step B, if desired, recrystallization, azeotropic dehydration with the addition of any solvent, or the combination of these treatments can be performed, for example, for the purpose of reducing the water content (e.g., water derived from atmosphere, etc.).
In step A, the obtained compound (P1) may be dried at any stage and any number of times by a normally conceivable drying treatment after step A, until it is used in the additive for an electrochemical device or other devices as described below.

Additive for Electrochemical Device

In one embodiment of the present disclosure, the additive for an electrochemical device is a compound represented by formula (P1A): $RfCOOA^d$
wherein Rf is an organic group having one or more fluorine atoms, and $A^d$ is a metal atom, and comprises water in an amount of 0 to 50000 mass ppm relative to the compound represented by formula (P1).
In formula (P1A), from the viewpoint of easily obtaining a fluorinated carboxylic acid salt compound having a low water content, preferably, a fluorinated carboxylic acid salt compound substantially free of water, Rf is preferably a hydrocarbon group having one or more fluorine atoms, more preferably an alkyl group having one or more fluorine atoms, still more preferably a $C_{1-6}$ alkyl group having one or more fluorine atoms, even more preferably a $C_{1-3}$ alkyl group having two or more fluorine atoms.
From the viewpoint of easily obtaining the fluorinated carboxylic acid salt compound having a low water content, preferably, a fluorinated carboxylic acid salt compound substantially free of water, $A^d$ is preferably an alkali metal atom, more preferably Li, Na, or K, and even more preferably Li in formula (P1A).
In view of electrochemical device performance, the content of water is preferably 20000 mass ppm or less, more preferably 10000 mass ppm or less, still more preferably 7000 mass ppm or less, even more preferably 5000 mass ppm. The water content may be 0 mass ppm.
In one embodiment of the present disclosure, the additive for an electrochemical device may not contain water; however, if water is contained, the lower limit of the amount of water is, for example, 50 mass ppm, 100 mass ppm, and 500 mass ppm.
The additive for an electrochemical device can be used for the treatment of electrodes of electrochemical devices or lithium ion secondary batteries.
The additive for an electrochemical device can be contained in such electrochemical devices or lithium ion secondary batteries due to the use thereof. In this case, the additive for an electrochemical device may be varied or modified.
The present disclosure also discloses an electrochemical device comprising the additive for an electrochemical device of the present disclosure (or a variation or modification thereof).
Such an electrochemical device can be understood based on technical knowledge.

Compound

The compound according to one embodiment of the present disclosure is a compound represented by formula (P1A):$RfCOOA^d$, wherein Rf is an organic group having one or more fluorine atoms, and $A^d$ is an Li atom, wherein the compound has a water content of 50000 mass ppm or less.
From the viewpoint of easily obtaining a fluorinated carboxylic acid salt compound having a low water content, and preferably, a fluorinated carboxylic acid salt compound substantially free of water, in formula (P1A), Rf is preferably a hydrocarbon group having one or more fluorine atoms, more preferably an alkyl group having one or more fluorine atoms, still more preferably a $C_{1-6}$ alkyl group having one or more fluorine atoms, even more preferably a $C_{1-3}$ alkyl group having two or more fluorine atoms.
The water content can be measured using the Karl Fischer method.

In view of electrochemical device performance, the water content can be preferably 20000 mass ppm or less, more preferably 10000 mass ppm or less, still more preferably 7000 mass ppm or less, even more preferably 5000 mass ppm or less, particularly preferably 3000 mass ppm or less. The water content may be 0 mass ppm. Being 0 mass ppm means the measurement limit or less according to the Karl Fischer method.
In one embodiment of the present disclosure, the compound may not contain water; if water is contained, the lower limit of the content is, for example, 50 mass ppm, 100 mass ppm, or 500 mass ppm.
The compound can be understood by those skilled in the art with reference to the description in the additive for an electrochemical device in the present disclosure.

Composition

The composition according to one embodiment of the present disclosure is a compound represented by formula (P1A): $RfCOOA^d$,
wherein

Rf is an organic group having one or more fluorine atoms, and
$A^d$ is a Li atom, and contains water in an amount of 50000 mass ppm or less relative to the compound represented by formula (P1).

From the viewpoint of easily obtaining a fluorinated carboxylic acid salt compound having a low water content, and preferably, a fluorinated carboxylic acid salt compound substantially free of water, in formula (P1A), Rf is preferably a hydrocarbon group having one or more fluorine atoms, more preferably an alkyl group having one or more fluorine atoms, still more preferably a $C_{1-6}$ alkyl group having one or more fluorine atoms, even more preferably a $C_{1-3}$ alkyl group having two or more fluorine atoms.
In view of electrochemical device performance, the water content is preferably 20000 mass ppm or less, more preferably 10000 mass ppm or less, still more preferably 7000 mass ppm or less, even more preferably 5000 mass ppm or less, particularly preferably 3000 mass ppm or less.
Here, in one embodiment of the present disclosure, the lower limit of the water content in the composition is, for example, 50 mass ppm, 100 mass ppm, or 500 mass ppm.
The composition can be understood by those skilled in the art with reference to, for example, the description in the additive for an electrochemical device section in the present disclosure.

Electrolyte Solution

The electrolyte solution according to one embodiment of the present disclosure comprises the additive for an electrochemical device, compound, or composition of the present disclosure.
In view of electrochemical device performance, the amount of the additive for an electrochemical device, compound, or composition of the present disclosure is generally 0.001% by mass or more, preferably 0.01% by mass or more, more preferably 0.05% by mass or more, even more preferably 0.1% by mass or more, still more preferably 0.2% by mass or more, particularly preferably 0.5% by mass or more, relative to the entire electrolyte solution.
In view of electrochemical device performance, the amount of the additive for an electrochemical device, compound, or composition of the present disclosure is generally 10% by mass or less, preferably 5% by mass or less, more preferably 3% by mass or less, still more preferably 2% by mass or less, even more preferably 1.8% by mass or less, particularly preferably 1.5% by mass or less, relative to the entire electrolyte solution.
The electrolyte solution of the present disclosure preferably contains a solvent.
The solvent preferably contains at least one member selected from the group consisting of a carbonate and a carbonic acid ester, in view of electrochemical device performance.
The carbonate may be a cyclic carbonate or a chain carbonate.
The cyclic carbonate may be a non-fluorinated cyclic carbonate or a fluorinated cyclic carbonate.
An example of the non-fluorinated cyclic carbonate includes a non-fluorinated saturated cyclic carbonate. In view of electrochemical device performance, preferred is a non-fluorinated saturated alkylene carbonate having an alkylene group having 2 to 6 carbon atoms, and more preferred is a non-fluorinated saturated alkylene carbonate having an alkylene group having 2 to 4 carbon atoms.
Of these, in respect of easily attaining high permittivity and suitable viscosity, the non-fluorinated saturated cyclic carbonate is preferably at least one member selected from the group consisting of ethylene carbonate, propylene carbonate, cis-2,3-pentylene carbonate, cis-2,3-butylene carbonate, 2,3-pentylene carbonate, 2,3-butylene carbonate, 1,2-pentylene carbonate, 1,2-butylene carbonate, and butylene carbonate.
One of the non-fluorinated saturated cyclic carbonates may be used singly, or two or more thereof may be used

in any combination at any ratio.

When the non-fluorinated saturated cyclic carbonate is contained, the content of the non-fluorinated saturated cyclic carbonate is preferably 5 to 90% by volume, more preferably 10 to 60% by volume, still more preferably 15 to 45% by volume with respect to the solvent, in view of electrochemical device performance.

The fluorinated cyclic carbonate is a cyclic carbonate having a fluorine atom. A solvent containing a fluorinated cyclic carbonate can be suitably used at a high voltage.

The term "high voltage" in the present specification means a voltage of 4.2 V or more. The upper limit of the "high voltage" is preferably 4.9 V.

The fluorinated cyclic carbonate may be a fluorinated saturated cyclic carbonate or a fluorinated unsaturated cyclic carbonate.

The fluorinated saturated cyclic carbonate is a saturated cyclic carbonate having a fluorine atom. Specific examples thereof include a compound represented by the following general formula (A):

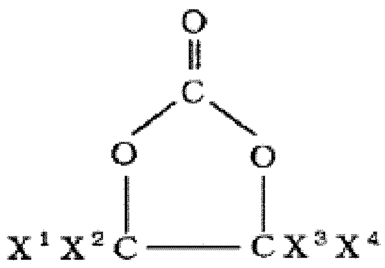

(A)

wherein $X^1$ to $X^4$ are the same or different from each other, and are each —H, $-CH_3$, $-C_2H_5$, -F, a fluorinated alkyl group optionally having an ether bond, or a fluorinated alkoxy group optionally having an ether bond; provided that at least one of $X^1$ to $X^4$ is -F, a fluorinated alkyl group optionally having an ether bond, or a fluorinated alkoxy group optionally having an ether bond (-O-). Examples of the fluorinated alkyl group include $-CF_3$, $-CF_2H$, and $-CH_2F$.

In the case where the electrolyte solution of the present disclosure, when containing the fluorinated saturated cyclic carbonate, is applied to a high-voltage lithium ion secondary battery or the like, the oxidation resistance of the electrolyte solution can be improved, and stable and excellent charge and discharge characteristics can be easily provided.

In respect of favorable permittivity and oxidation resistance, one or two of $X^1$ to $X^4$ is/are each preferably -F, a fluorinated alkyl group optionally having an ether bond, or a fluorinated alkoxy group optionally having an ether bond.

In anticipation of decrease in viscosity at low temperature, increase in the flash point, and improvement in the solubility of an electrolyte salt, $X^1$ to $X^4$ are each preferably —H, -F, a fluorinated alkyl group (a), a fluorinated alkyl group having an ether bond (b), or a fluorinated alkoxy group (c).

The fluorinated alkyl group (a) is a group obtainable by replacing at least one hydrogen atom of an alkyl group by a fluorine atom. The fluorinated alkyl group (a) has preferably 1 to 20 carbon atoms, more preferably 1 to 17 carbon atoms, still more preferably 1 to 7 carbon atoms, particularly preferably 1 to 5 carbon atoms.

Examples of the fluorinated alkyl group (a) having one carbon atom include $CFH_2$-, $CF_2H$-, and $CF_3$-. In respect of hightemperature storage characteristics, particularly preferred is $CF_2H$- or $CF_3$-, and most preferred is $CF_3$-.

In respect of favorable solubility of an electrolyte salt, among the above fluorinated alkyl groups (a), a preferred example of the group (a) having 2 or more carbon atoms includes a fluorinated alkyl group represented by the following general formula (a-1):

$R^1$-$R^2$- (a-1)

(wherein $R^1$ is an alkyl group having one or more carbon atoms and optionally having a fluorine atom; $R^2$ is an alkylene group having 1 to 3 carbon atoms and optionally having a fluorine atom;
provided that at least one of $R^1$ and $R^2$ has a fluorine atom.)

$R^1$ and $R^2$ each may further have an atom other than carbon, hydrogen, and fluorine atoms.

$R^1$ is an alkyl group having one or more carbon atoms and optionally having a fluorine atom. $R^1$ is preferably a linear or branched chain alkyl group having 1 to 16 carbon atoms. $R^1$ has more preferably 1 to 6 carbon atoms, still more preferably 1 to 3 carbon atoms.

Specific examples of linear or branched chain alkyl groups for $R^1$ include $CH_3$-, $CH_3CH_2$-, $CH_3CH_2CH_2$-, $CH_3CH_2CH_2CH_2$-,

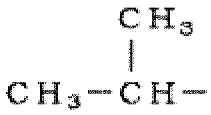 、 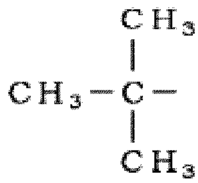

and the like.

When $R^1$ is a linear alkyl group having a fluorine atom, examples of $R^1$ include $CF_3$-, $CF_3CH_2$-, $CF_3CF_2$-, $CF_3CH_2CH_2$-, $CF_3CF_2CH_2$-, $CF_3CF_2CF_2$-, $CF_3CH_2CF_2$-, $CF_3CH_2CH_2CH_2$-, $CF_3CF_2CH_2CH_2$-, $CF_3CH_2CF_2CH_2$-, $CF_3CF_2CF_2CH_2$-, $CF_3CF_2CF_2CF_2$-, $CF_3CF_2CH_2CF_2$-, $CF_3CH_2CH_2CH_2CH_2$-, $CF_3CF_2CH_2CH_2CH_2$-, $CF_3CH_2CF_2CH_2CH_2$-, $CF_3CF_2CF_2CH_2CH_2$-, $CF_3CF_2CF_2CF_2CH_2$-, $CF_3CF_2CH_2CF_2CH_2$-, $CF_3CF_2CH_2CH_2CH_2CH_2$-, $CF_3CF_2CF_2CF_2CH_2CH_2$-, $CF_3CF_2CH_2CF_2CH_2CH_2$-, $HCF_2$-, $HCF_2CH_2$-, $HCF_2CF_2$-, $HCF_2CH_2CH_2$-, $HCF_2CF_2CH_2$-, $HCF_2CH_2CF_2$-, $HCF_2CF_2CH_2CH_2$-, $HCF_2CH_2CF_2CH_2$-, $HCF_2CF_2CF_2CF_2$-, $HCF_2CF_2CH_2CH_2CH_2$-, $HCF_2CH_2CF_2CH_2CH_2$-, $HCF_2CF_2CF_2CF_2CH_2$-, $HCF_2CF_2CF_2CF_2CH_2CH_2$-, $FCH_2$-, $FCH_2CH_2$-, $FCH_2CF_2$-, $FCH_2CF_2CH_2$-, $FCH_2CF_2CF_2$-, $CH_3CF_2CH_2$-, $CH_3CF_2CF_2$-, $CH_3CF_2CH_2CF_2$-, $CH_3CF_2CF_2CF_2$-, $CH_3CH_2CF_2CF_2$-, $CH_3CF_2CH_2CF_2CH_2$-, $CH_3CF_2CF_2CF_2CH_2$-, $CH_3CF_2CF_2CH_2CH_2$-, $CH_3CH_2CF_2CF_2CH_2$-, $CH_3CF_2CH_2CF_2CH_2CH_2$-, $CH_3CF_2CH_2CF_2CH_2CH_2$-, $HCFClCF_2CH_2$-, $HCF_2CFClCH_2$-, $HCF_2CFClCF_2CFClCH_2$-, and $HCFClCF_2CFClCF_2CH_2$.

When $R^1$ is a branched chain alkyl group having a fluorine atom, preferred examples of $R^1$ include:

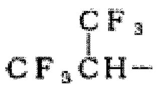、 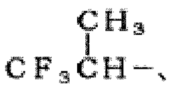、 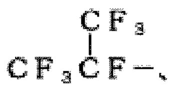、 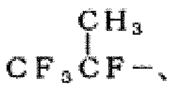、

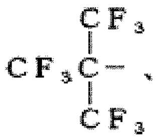 、 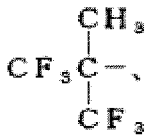、 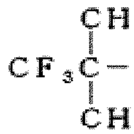 、

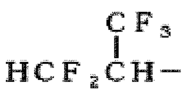、 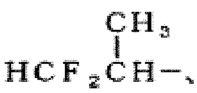、 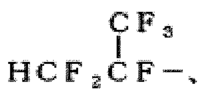、 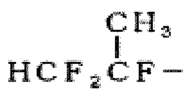、

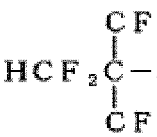、 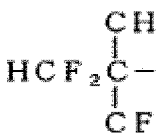、 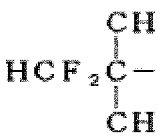 、

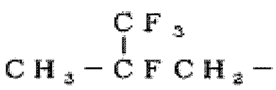 、 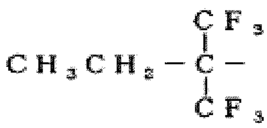 、

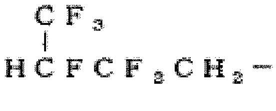 、 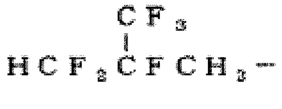 、 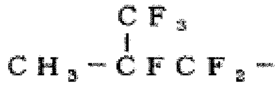 、

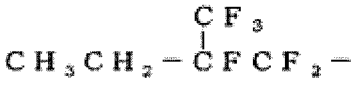 、 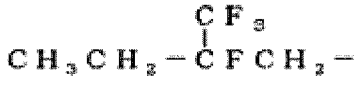 、

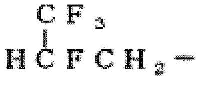 、 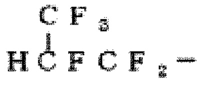 、 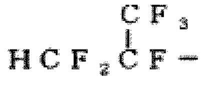 、

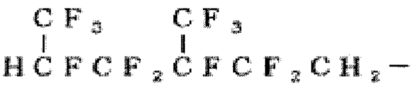 、 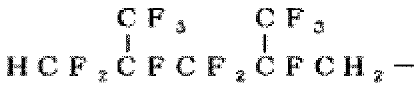 、

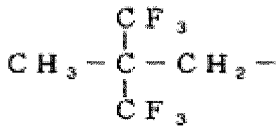

However, if a branch such as $CH_3$- or $CF_3$- is contained, the viscosity is likely to increase. Thus, the number of such branches is more preferably small (one) or zero.

$R^2$ is an alkylene group having 1 to 3 carbon atoms and optionally having a fluorine atom. $R^2$ may be linear or branched chain. Examples of a minimum structural unit constituting such a linear or branched chain alkylene group are shown below. $R^2$ is constituted by one or a combination of these units.

(i) Linear Minimum Structural Units:

$-CH_2-$, $-CHF-$, $-CF_2-$, $-CHCl-$, $-CFCl-$, $-CCl_2-$

(ii) Branched Chain Minimum Structural Units:

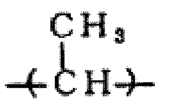 、 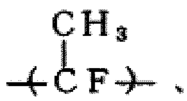 、 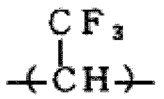 、 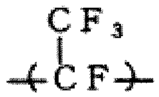 、

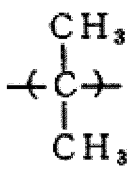 、 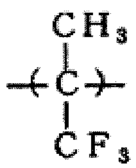 、 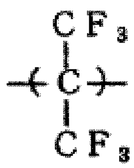 、

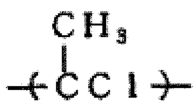 、 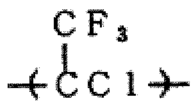

$R^2$ is preferably constituted by Cl-free structural units among these examples, because such units may not be dehydrochlorinated by a base and thus may be more stable.

When being linear, $R^2$ is composed only of any of the above linear minimum structural units, and is preferably $-CH_2-$, $-CH_2CH_2-$, or $-CF_2-$ among these. Since the solubility of an electrolyte salt can be further improved, $-CH_2-$ or $-CH_2CH_2-$ is more preferred.

When being branched chain, $R^2$ includes at least one of the above branched chain minimum structural units. A preferred example thereof is a group represented by the general formula: $-(CX^aX^b)-$, wherein $X^a$ is H, F, $CH_3$, or $CF_3$; $X^b$ is $CH_3$ or $CF_3$; provided that, when $X^b$ is $CF_3$, $X^a$ is H or $CH_3$. Such groups can much further particularly improve the solubility of an electrolyte salt.

Preferred examples of the fluorinated alkyl group (a) include $CF_3CF_2$-, $HCF_2CF_2$-, $H_2CFCF_2$-, $CH_3CF_2$-, $CF_3CHF$-, $CH_3CF_2$-, $CF_3CF_2CF_2$-, $HCF_2CF_2CF_2$-, $H_2CFCF_2CF_2$-, $CH_3CF_2CF_2$-,

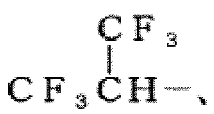 、 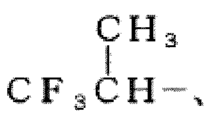 、 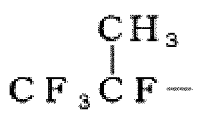 、

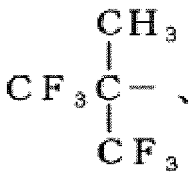 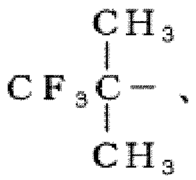

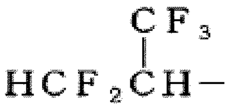 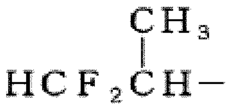 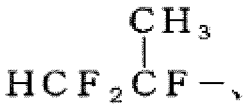

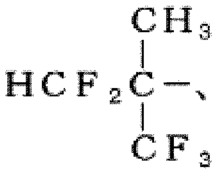 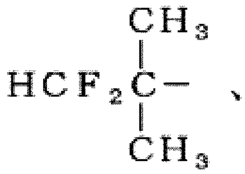

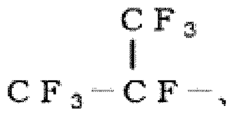 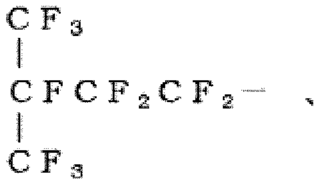

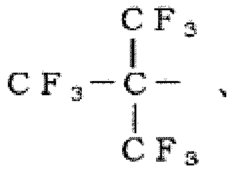

and the like.

The above fluorinated alkyl group having an ether bond (b) is a group obtainable by replacing at least one hydrogen atom of an alkyl group having an ether bond by a fluorine atom. The fluorinated alkyl group having an ether bond (b) preferably has 2 to 17 carbon atoms. An excessively large number of carbon atoms may lead to an increase in the viscosity of the fluorinated saturated cyclic carbonate and also an increase of fluorinecontaining groups. Thus, there may be observed a decrease in the solubility of an electrolyte salt due to a reduction in permittivity, and a decrease in compatibility with other solvents. From this viewpoint, the fluorinated alkyl group having an ether bond (b) has more preferably 2 to 10 carbon atoms, still more preferably 2 to 7 carbon atoms.

The alkylene group which constitutes the ether moiety of the fluorinated alkyl group having an ether bond (b) may be a linear or branched chain alkylene group. Examples of a minimum structural unit constituting such a linear or branched chain alkylene group are shown below.

(i) Linear Minimum Structural Units:

$-CH_2-$, $-CHF-$, $-CF_2-$, $-CHCl-$, $-CFCl-$, and $-CCl_2-$.

(ii) Branched Chain Minimum Structural Units:

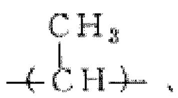 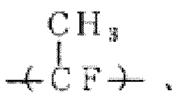 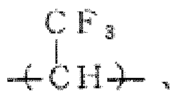 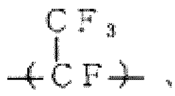

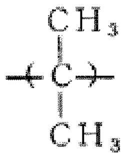 、 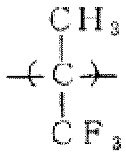 、 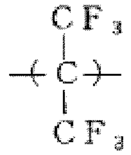 、

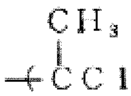 、 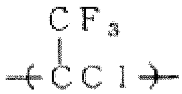

The alkylene group may be constituted by one of these minimum structural units, or may be constituted by linear units (i), by branched chain units (ii), or by a combination of a linear unit (i) and a branched chain unit (ii). Preferred specific examples will be described below in detail.

The alkylene group is preferably constituted by Cl-free structural units among these examples, because such units may not be dehydrochlorinated by a base and thus may be more stable.

A further preferred example of the fluorinated alkyl group having an ether bond (b) includes a group represented by the general formula (b-1):

$$R^3\text{-}(OR^4)_{n1}\text{-} \qquad \text{(b-1)}$$

(wherein $R^3$ is preferably an alkyl group having 1 to 6 carbon atoms and optionally having a fluorine atom; $R^4$ is preferably an alkylene group having 1 to 4 carbon atoms and optionally having a fluorine atom; n1 is an integer of 1 to 3; provided that at least one of $R^3$ and $R^4$ has a fluorine atom.)

Examples of $R^3$ and $R^4$ include the following groups; and any appropriate combination of these groups can provide, but are not limited to, the fluorinated alkyl group having an ether bond (b) represented by the general formula (b-1).

(1) $R^3$ is preferably an alkyl group represented by the general formula: $X^c{}_3C\text{-}(R^5)_{n2}\text{-}$, wherein three $X^c$s are the same or different from each other, and are each H or F; $R^5$ is an alkylene group having 1 to 5 carbon atoms and optionally having a fluorine atom; and n2 is 0 or 1.

When n2 is 0, examples of $R^3$ include $CH_3$-, $CF_3$-, $HCF_2$-, and $H_2CF$-.

When n2 is 1, specific examples of $R^3$, which is a linear group include $CF_3CH_2$-, $CF_3CF_2$-, $CF_3CH_2CH_2$-, $CF_3CF_2CH_2$-, $CF_3CF_2CF_2$-, $CF_3CH_2CF_2$-, $CF_3CH_2CH_2CH_2$-, $CF_3CF_2CH_2CH_2$-, $CF_3CH_2CF_2CH_2$-, $CF_3CF_2CF_2CH_2$-, $CF_3CF_2CF_2CF_2$-, $CF_3CF_2CH_2CF_2$-, $CF_3CH_2CH_2CH_2CH_2$-, $CF_3CF_2CH_2CH_2CH_2$-, $CF_3CH_2CF_2CH_2CH_2$-, $CF_3CF_2CF_2CH_2CH_2$-, $CF_3CF_2CF_2CF_2CH_2$-, $CF_3CF_2CH_2CF_2CH_2$-, $CF_3CF_2CH_2CH_2CH_2CH_2$-, $CF_3CF_2CF_2CF_2CH_2CH_2$-, $CF_3CF_2CH_2CF_2CH_2CH_2$-, $HCF_2CH_2$-, $HCF_2CF_2$-, $HCF_2CH_2CH_2$-, $HCF_2CF_2CH_2$-, $HCF_2CH_2CF_2$-, $HCF_2CF_2CH_2CH_2$-, $HCF_2CH_2CF_2CH_2$-, $HCF_2CF_2CF_2CF_2$-, $HCF_2CF_2CH_2CH_2CH_2$-, $HCF_2CH_2CF_2CH_2CH_2$-, $HCF_2CF_2CF_2CF_2CH_2$-, $HCF_2CF_2CF_2CF_2CH_2CH_2$-, $FCH_2CH_2$-, $FCH_2CF_2$-, $FCH_2CF_2CH_2$-, $CH_3CF_2$-, $CH_3CH_2$-, $CH_3CF_2CH_2$-, $CH_3CF_2CF_2$-, $CH_3CH_2CH_2$-, $CH_3CF_2CH_2CF_2$-, $CH_3CF_2CF_2CF_2$-, $CH_3CH_2CF_2CF_2$-, $CH_3CH_2CH_2CH_2$-, $CH_3CF_2CH_2CF_2CH_2$-, $CH_3CF_2CF_2CF_2CH_2$-, $CH_3CF_2CF_2CH_2CH_2$-, $CH_3CH_2CF_2CF_2CH_2$-, $CH_3CF_2CH_2CF_2CH_2CH_2$-, $CH_3CH_2CF_2CF_2CH_2CH_2$-, and $CH_3CF_2CH_2CF_2CH_2CH_2$-.

Examples thereof in which n2 is 1 and $R^3$ is a branched chain group include:

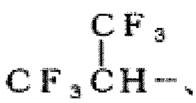 、 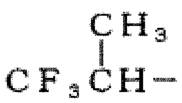 、 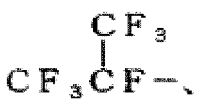 、 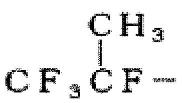 、

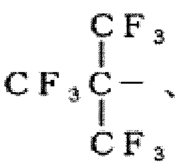 、 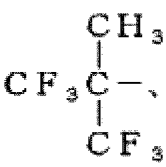 、 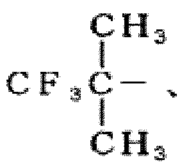 、 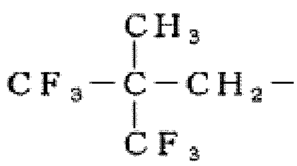 、

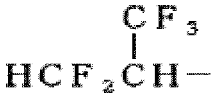、 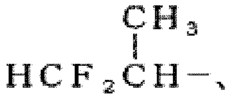、 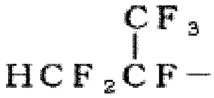、 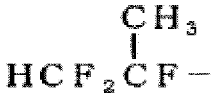、

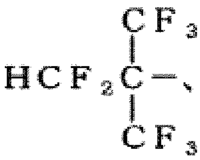、 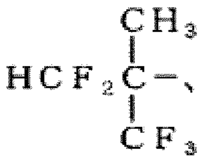、 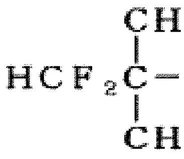

and the like.

However, if a branch such as $CH_3$- or $CF_3$- is contained, the viscosity is likely to increase. Thus, those in which $R^3$ is a linear group are more preferred.

(2) In $-(OR^4)n_1-$ of the general formula (b-1), n1 is an integer of 1 to 3, preferably 1 or 2. When n1 is 2 or 3, $R^4$s may be the same or different from each other.

Preferred specific examples of $R^4$ include the following linear or branched chain groups.

Examples of $R^4$, which is a linear group include $-CH_2-$, $-CHF-$, $-CF_2-$, $-CH_2CH_2-$, $-CF_2CH_2-$, $-CF_2CF_2-$, $-CH_2CF_2-$, $-CH_2CH_2CH_2-$, $-CH_2CH_2CF_2-$, $-CH_2CF_2CH_2-$, $-CH_2CF_2CF_2-$, $-CF_2CH_2CH_2-$, $-CF_2CF_2CH_2-$, $-CF_2CH_2CF_2-$, and $-CF_2CF_2CF_2-$.

Examples of $R^4$, which is a branched chain group include:

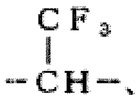、 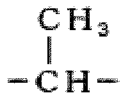、 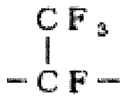、 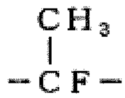、

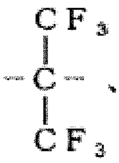、 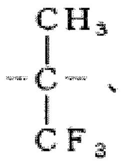、 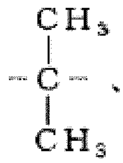、

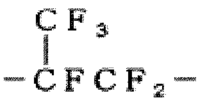、 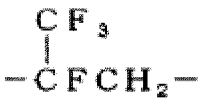、 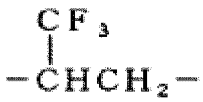、 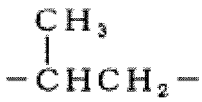、

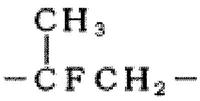、 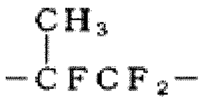、 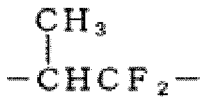、 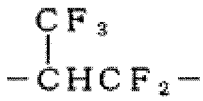

and the like.

The fluorinated alkoxy group (c) is a group obtainable by replacing at least one hydrogen atom of an alkoxy group by a fluorine atom. The fluorinated alkoxy group (c) has preferably 1 to 17 carbon atoms, more preferably 1 to 6 carbon atoms.

The fluorinated alkoxy group (c) is particularly preferably a fluorinated alkoxy group represented by the general formula: $X^d_3C-(R^6)_{n3}-O-$, wherein three $X^d$s are the same or different from each other, and are each H or F; $R^6$ is an alkylene group having 1 to 5 carbon atoms and optionally having a fluorine atom; n3 is 0 or 1; provided that any of the three $X^d$s contains a fluorine atom.

Specific examples of the fluorinated alkoxy group (c) include fluorinated alkoxy groups in which an oxygen atom binds to an end of an alkyl group, mentioned as an example for $R^1$ in the general formula (a-1).

The fluorinated alkyl group (a), the fluorinated alkyl group having an ether bond (b), and the fluorinated alkoxy group (c) in the fluorinated saturated cyclic carbonate each preferably have a fluorine content of 10% by mass or more, preferably 12% by mass, and more preferably 15% by mass in terms of an effect of reducing the viscosity at low

temperature and an effect of increasing the flash point. The upper limit thereof is usually 76% by mass.

The fluorine content of each of the fluorinated alkyl group (a), the fluorinated alkyl group having an ether bond (b), and the fluorinated alkoxy group (c) is a value calculated based on each structural formula thereof by:

[(Number of fluorine atoms×19)/(Formula weight of each group)]×100 (% by mass).

In view of favorable permittivity and oxidation resistance, the fluorine content in the total fluorinated saturated cyclic carbonate is preferably 10% by mass or more, more preferably 15% by mass or more. The upper limit thereof is usually 76% by mass.

The fluorine content in the fluorinated saturated cyclic carbonate is a value calculated based on the structural formula of the fluorinated saturated cyclic carbonate by:

[(Number of fluorine atoms×19)/(Molecular weight of fluorinated saturated cyclic carbonate)]×100 (% by mass).

Specific examples of the fluorinated saturated cyclic carbonate include the following.

Specific examples of the fluorinated saturated cyclic carbonate in which at least one of $X^1$ to $X^4$ is -F include:

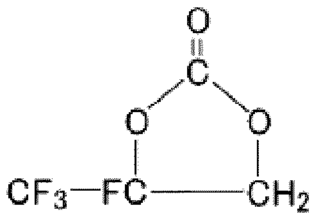 , 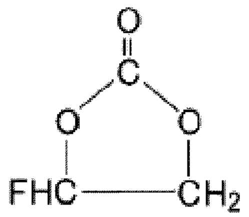 ,

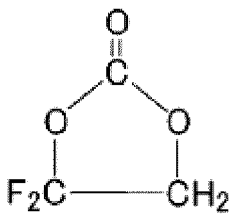 , 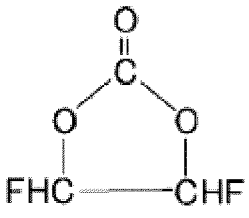 ,

These compounds have a high withstand voltage and also give favorable solubility of an electrolyte salt.

Alternatively,

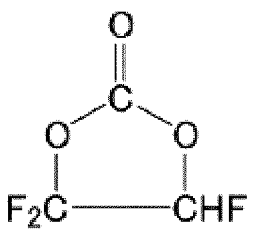 , 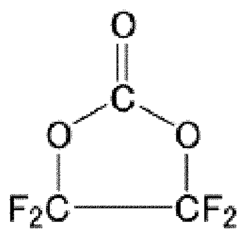 , 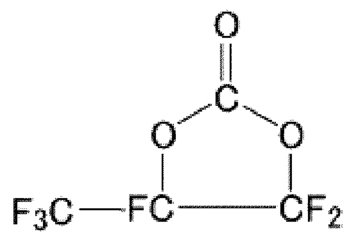 ,

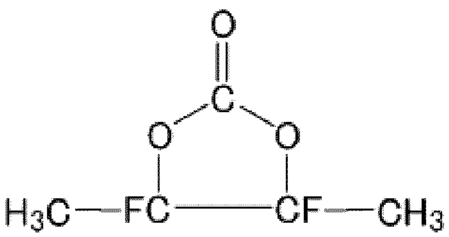 , 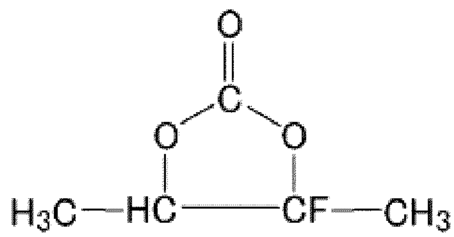

and the like may be used.

Specific examples of the fluorinated saturated cyclic carbonate in which at least one of $X^1$ to $X^4$ is a fluorinated alkyl group (a) and the others are —H include

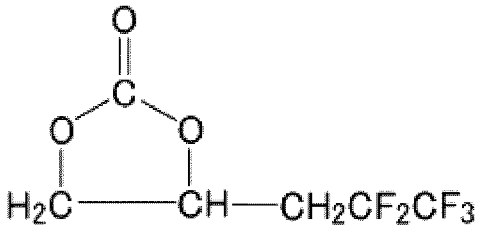
,
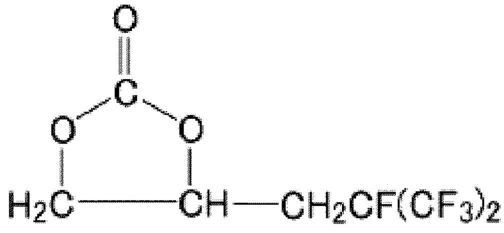
,

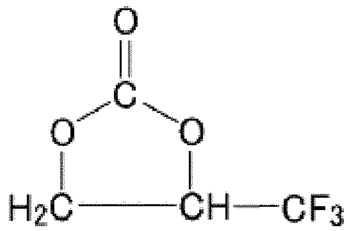
,
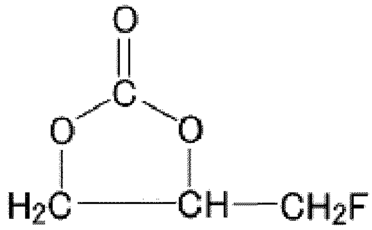
,

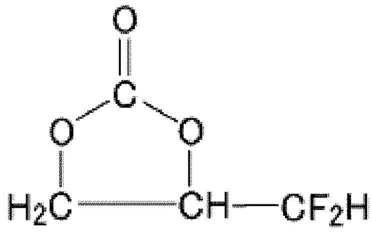
,
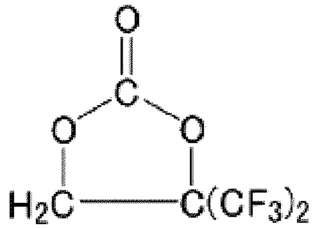
,

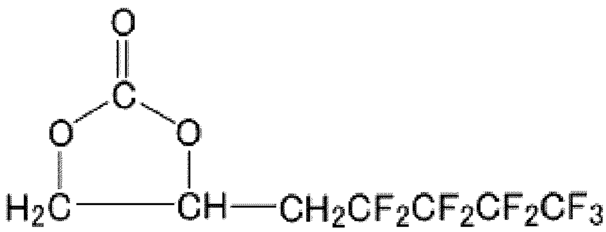
,
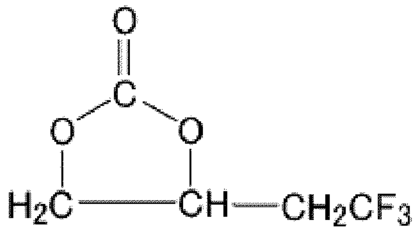

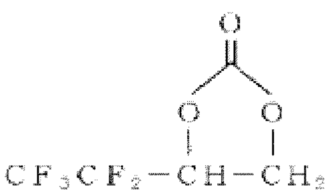
、

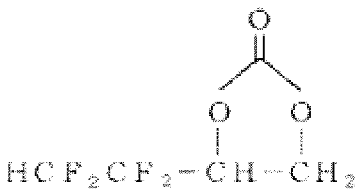
、
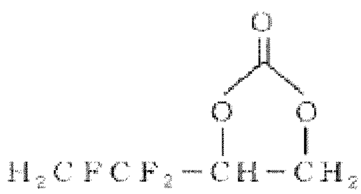
、

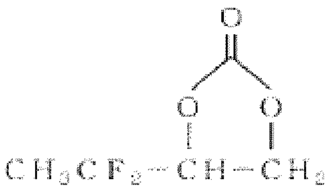

,

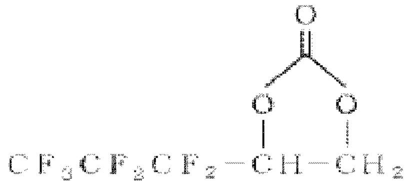

,

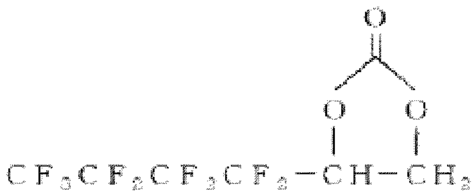

,

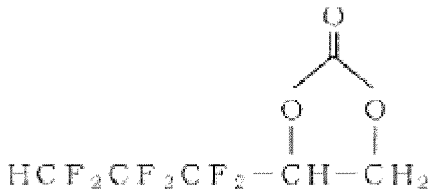

,

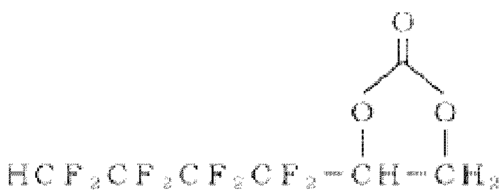

,

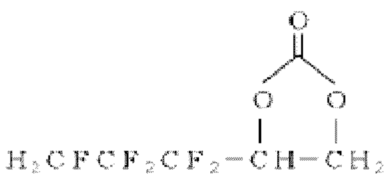

,

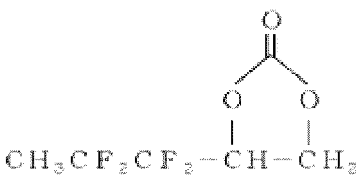

,

and the like.

Specific examples of the fluorinated saturated cyclic carbonate in which at least one of $X^1$ to $X^4$ is a fluorinated alkyl group having an ether bond (b) or a fluorinated alkoxy group (c) and the others are —H include

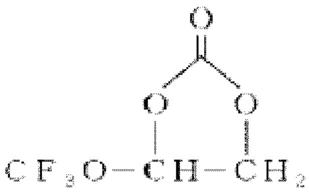 、 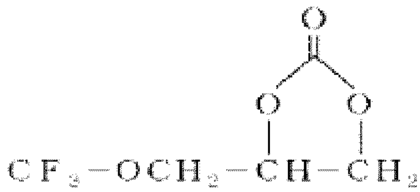 、

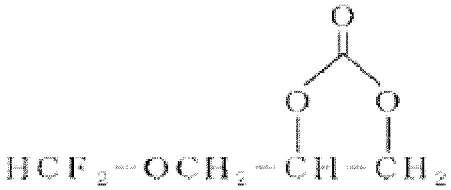 、 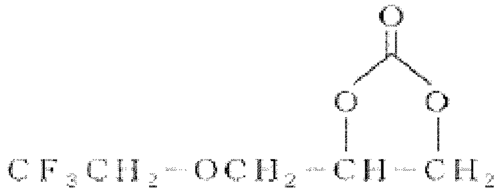 、

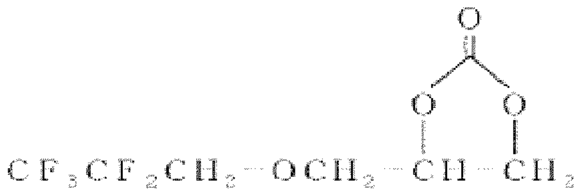 、

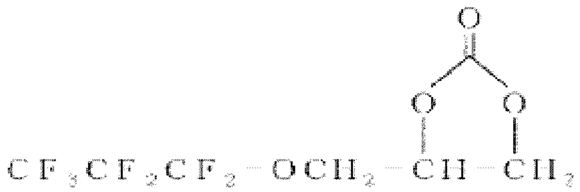 、

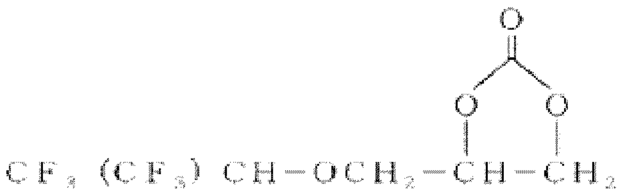 、

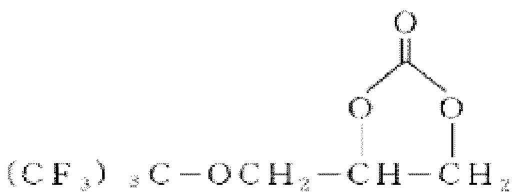 、

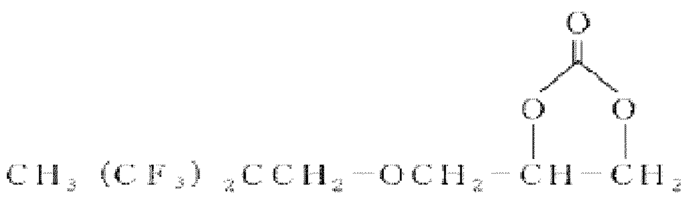 、

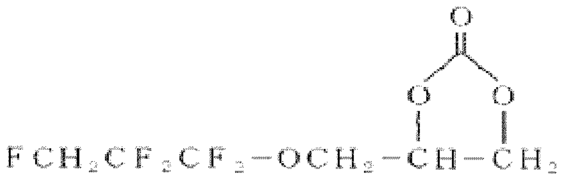

、

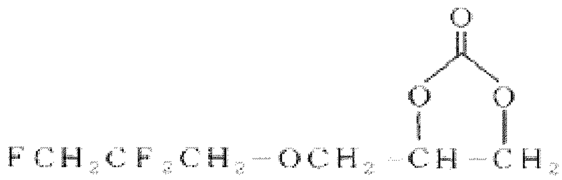

、

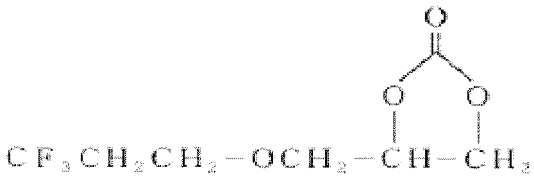

、

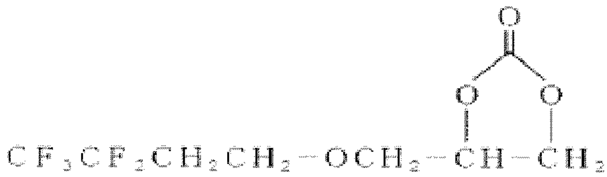

、

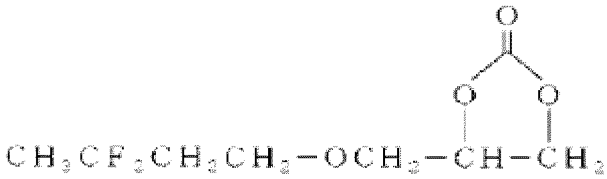

、

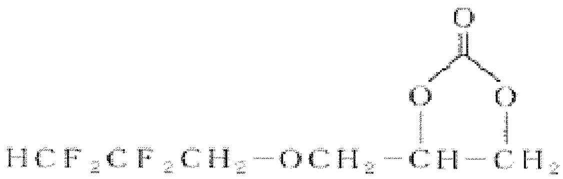

、

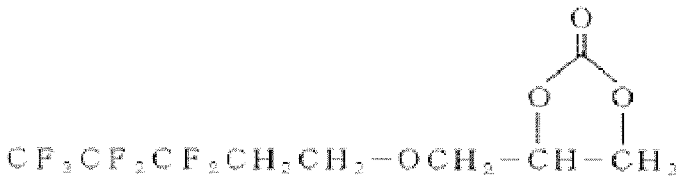

、

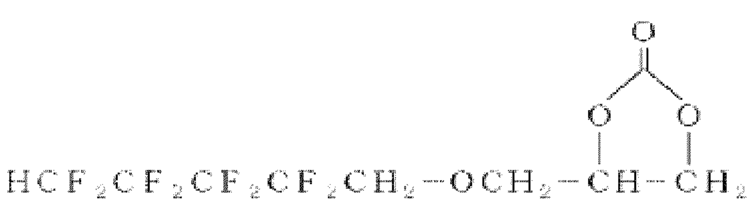

、

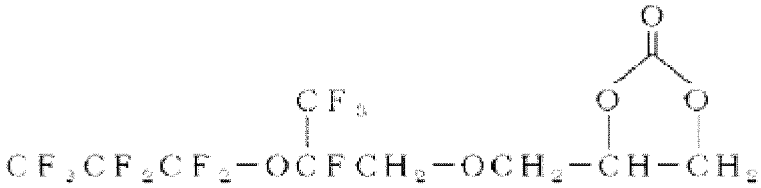 、

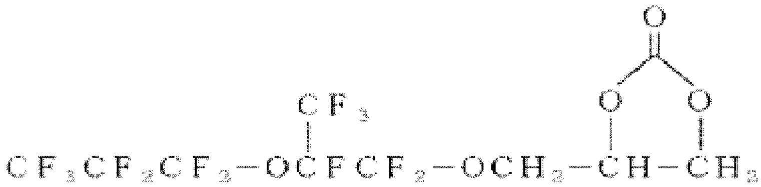 、

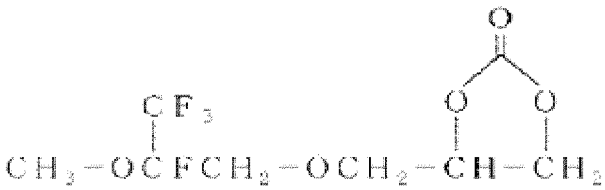 、

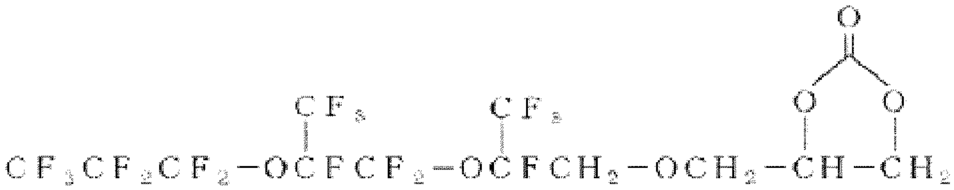 、

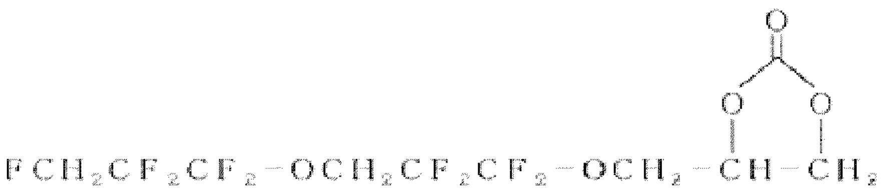 ,

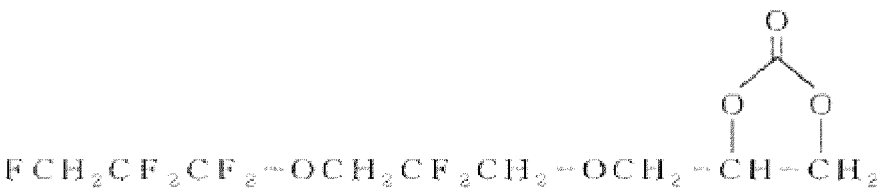 ,

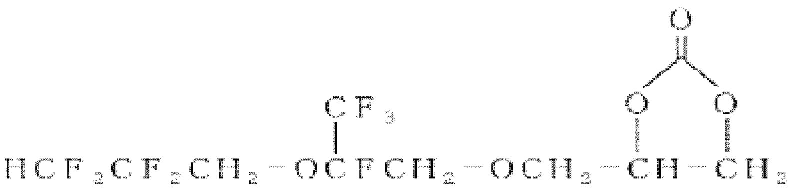 、

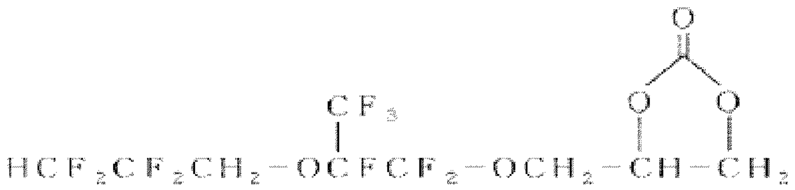
、

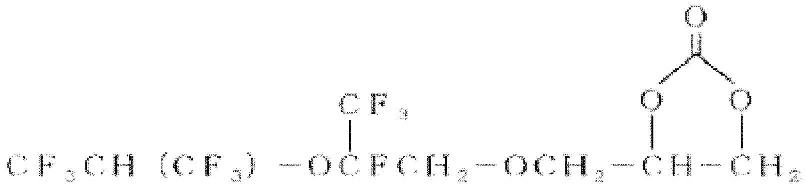
、

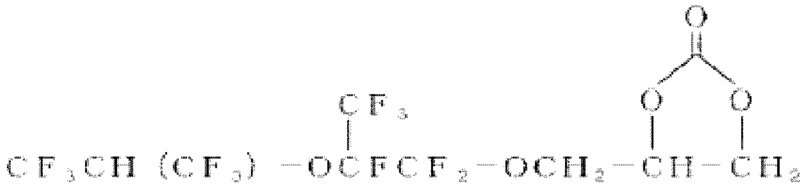
、

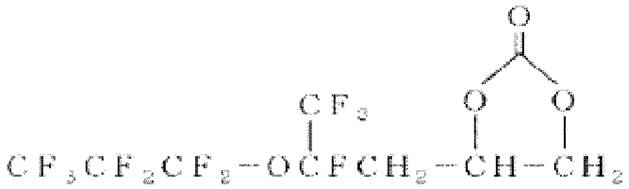
、

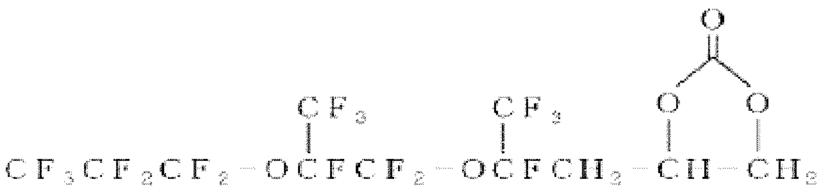
、

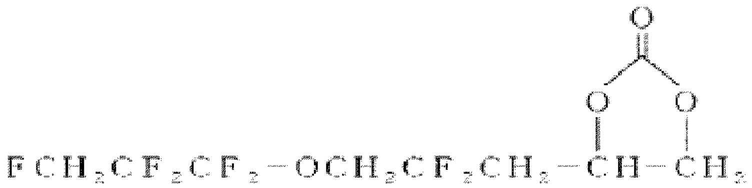
、

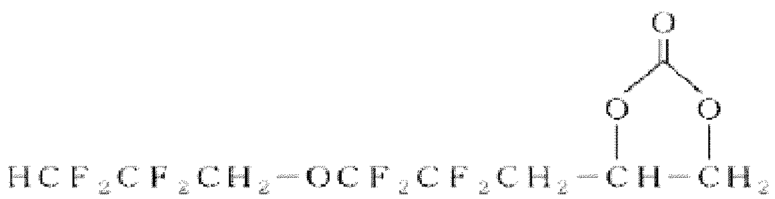
、

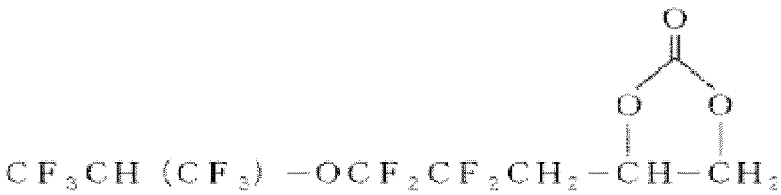

、

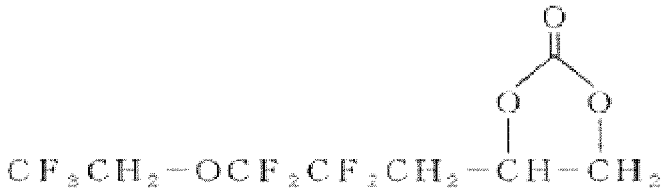

、

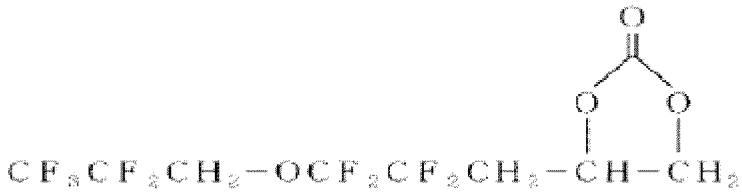

、

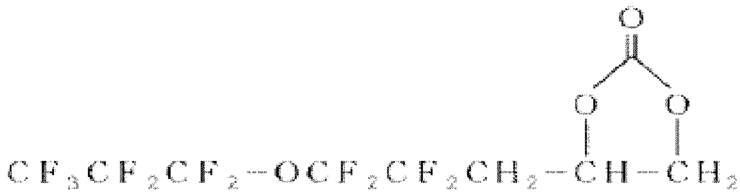

、

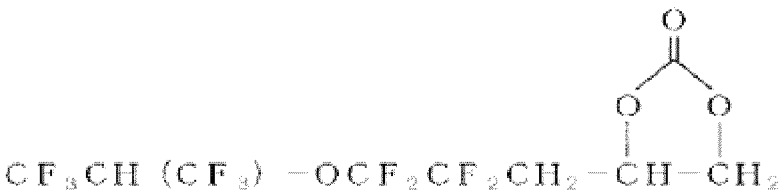

、

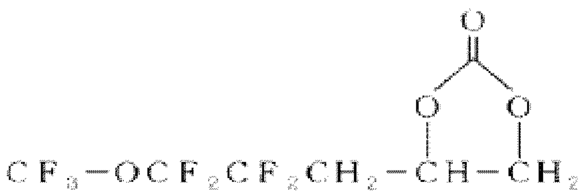

、

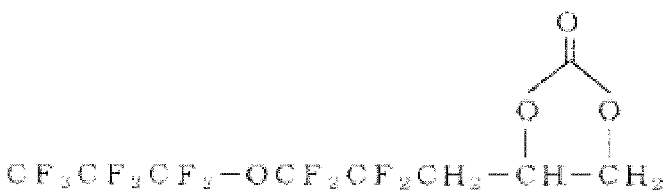

、

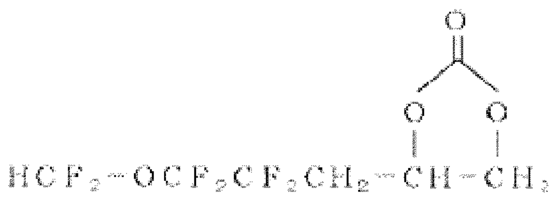 、

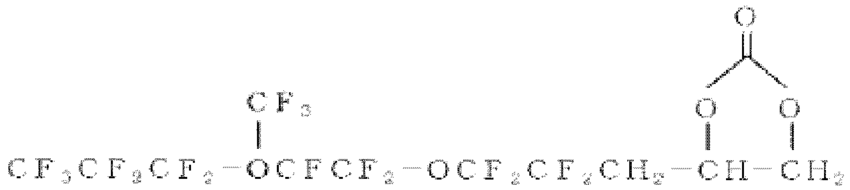 、

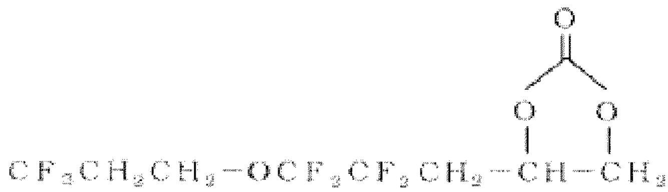 、

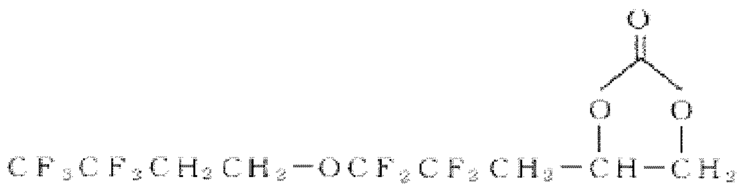 、

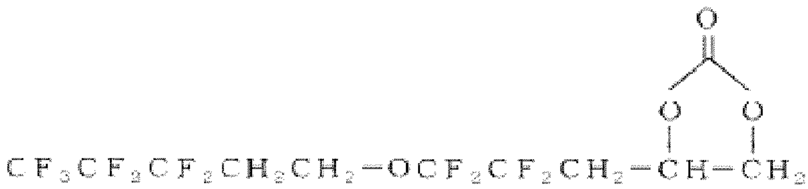 、

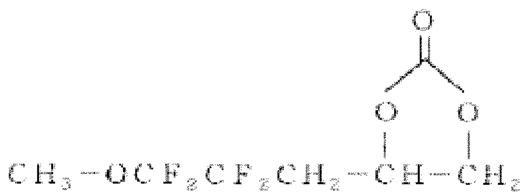

and the like.

Among these, the fluorinated saturated cyclic carbonate is preferably any of the following compounds.

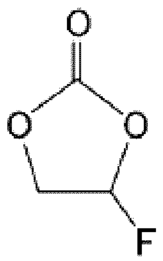
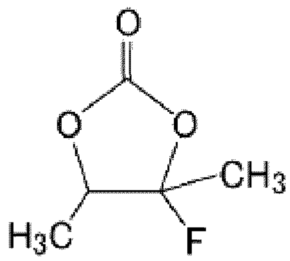
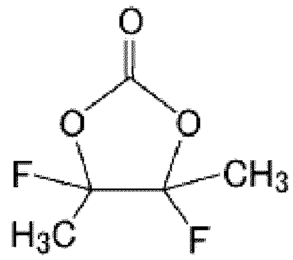
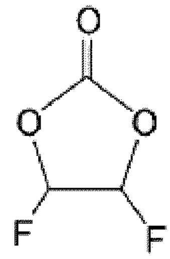

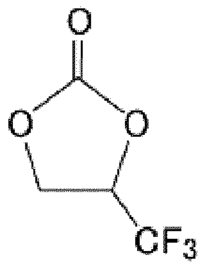
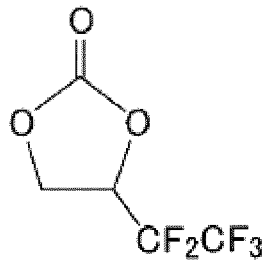
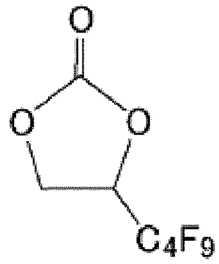
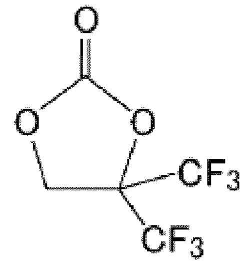

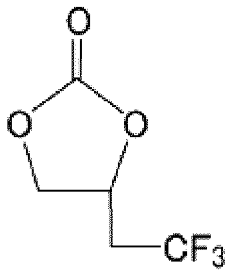
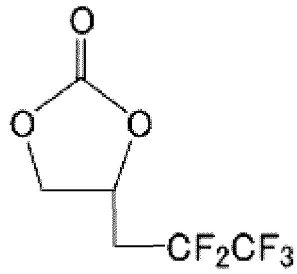
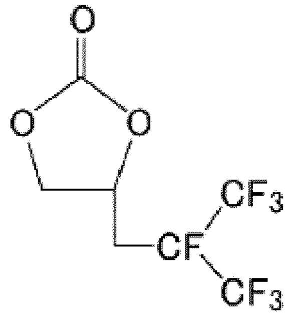
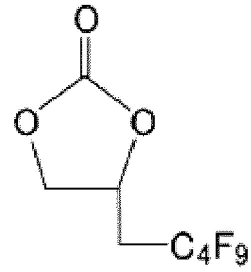

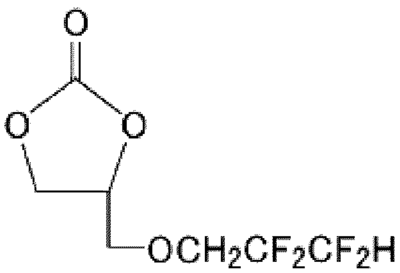
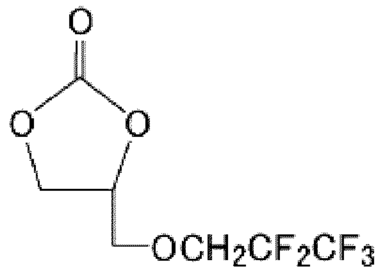
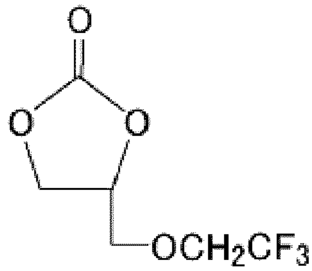

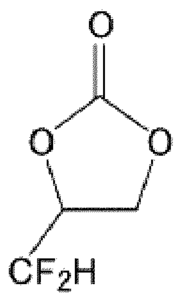
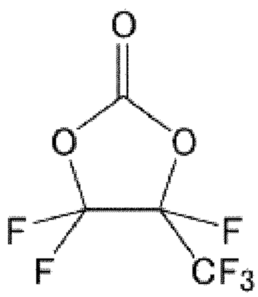
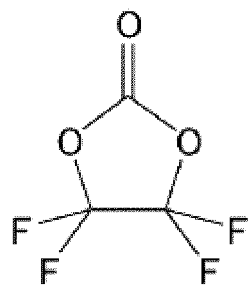
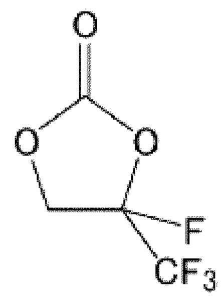

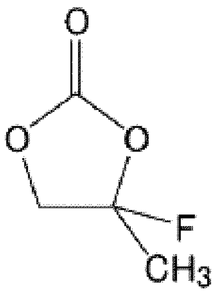 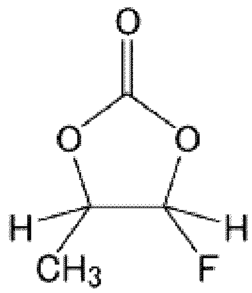 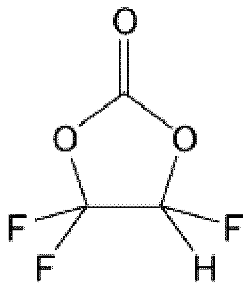 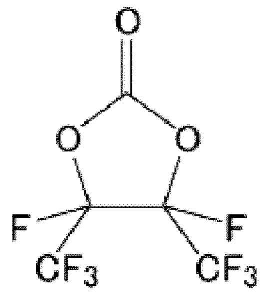

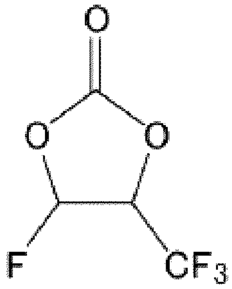

Examples of the fluorinated saturated cyclic carbonate also include trans-4,5-difluoro-1,3-dioxolan-2-one, 5-(1,1-difluoroethyl)-4,4-difluoro-1,3-dioxolan-2-one, 4-methylene-1,3-dioxolan-2-one, 4-methyl-5-trifluoromethyl-1,3-dioxolan-2-one, 4-ethyl-5-fluoro-1,3-dioxolan-2-one, 4-ethyl-5,5-difluoro-1,3-dioxolan-2-one, 4-ethyl-4,5-difluoro-1,3-dioxolan-2-one, 4-ethyl-4,5,5-trifluoro-1,3-dioxolan-2-one, 4,4-difluoro-5-methyl-1,3-dioxolan-2-one, 4-fluoro-5-methyl-1,3-dioxolan-2-one, 4-fluoro-5-trifluoromethyl-1,3-dioxolan-2-one, and 4,4-difluoro-1,3-dioxolan-2-one.

More preferred among these as the fluorinated saturated cyclic carbonate are fluoroethylene carbonate, difluoroethylene carbonate, trifluoromethylethylene carbonate (3,3,3-trifluoropropylene carbonate), and 2,2,3,3,3-pentafluoropropylethylene carbonate, in view of electrochemical device performance.

The fluorinated unsaturated cyclic carbonate is a cyclic carbonate having an unsaturated bond and a fluorine atom, and is preferably a fluorinated ethylene carbonate derivative substituted with a substituent having an aromatic ring or a carbon-carbon double bond, in view of electrochemical device performance. Specific examples thereof include 4,4-difluoro-5-phenyl ethylene carbonate, 4,5-difluoro-4-phenyl ethylene carbonate, 4-fluoro-5-phenyl ethylene carbonate, 4-fluoro-5-vinyl ethylene carbonate, 4-fluoro-4-phenyl ethylene carbonate, 4,4-difluoro-4-vinyl ethylene carbonate, 4,4-difluoro-4-allyl ethylene carbonate, 4-fluoro-4-vinyl ethylene carbonate, 4-fluoro-4,5-diallyl ethylene carbonate, 4,5-difluoro-4-vinyl ethylene carbonate, 4,5-difluoro-4,5-divinyl ethylene carbonate, and 4,5-difluoro-4,5-diallyl ethylene carbonate.

One of the fluorinated cyclic carbonates may be used singly, or two or more thereof may be used in any combination at any ratio.

When the fluorinated cyclic carbonate is contained, the content of the fluorinated cyclic carbonate is preferably 5 to 90% by volume, more preferably 10 to 60% by volume, still more preferably 15 to 45% by volume with respect to the solvent, in view of electrochemical device performance.

The chain carbonate may be a non-fluorinated chain carbonate or a fluorinated chain carbonate.

Examples of the non-fluorinated chain carbonate include hydrocarbon-based chain carbonates such as $CH_3OCOOCH_3$ (dimethyl carbonate, DMC), $CH_3CH_2OCOOCH_2CH_3$ (diethyl carbonate, DEC), $CH_3CH_2OCOOCH_3$ (ethyl methyl carbonate, EMC), $CH_3OCOOCH_2CH_2CH_3$ (methyl propyl carbonate), methyl butyl carbonate, ethyl propyl carbonate, ethyl butyl carbonate, dipropyl carbonate, dibutyl carbonate, methyl isopropyl carbonate, methyl-2-phenyl phenyl carbonate, phenyl-2-phenyl phenyl carbonate, trans-2,3-pentylene carbonate, trans-2,3-butylene carbonate, and ethyl phenyl carbonate. Preferred among these is at least one selected from the group consisting of ethyl methyl carbonate, diethyl carbonate, and dimethyl carbonate.

One of the non-fluorinated chain carbonates may be used singly, or two or more thereof may be used in any combination at any ratio.

When the non-fluorinated chain carbonate is contained, the content of the non-fluorinated chain carbonate is preferably 10 to 90% by volume, more preferably 40 to 85% by volume, still more preferably 50 to 80% by volume with respect to the solvent, in view of electrochemical device performance.

The fluorinated chain carbonate is a chain carbonate having a fluorine atom. A solvent containing a fluorinated chain carbonate can be suitably used even at a high voltage.

An example of the fluorinated chain carbonate can include a compound represented by the general formula (B):

$Rf^2OCOOR^7$ (B)

(wherein $Rf^2$ is a fluorinated alkyl group having 1 to 7 carbon atoms, and $R^7$ is an alkyl group having 1 to 7 carbon atoms and optionally containing a fluorine atom.)

$Rf^2$ is a fluorinated alkyl group having 1 to 7 carbon atoms, and $R^7$ is an alkyl group having 1 to 7 carbon atoms and optionally containing a fluorine atom.

The fluorinated alkyl group is a group obtainable by replacing at least one hydrogen atom of an alkyl group by a fluorine atom. When $R^7$ is an alkyl group containing a fluorine atom, the group is a fluorinated alkyl group.

$Rf^2$ and $R^7$ preferably have 1 to 7 carbon atoms, more preferably 1 to 2 carbon atoms, in view of giving a low viscosity, the solubility of an electrolyte salt, etc.

Examples of the fluorinated alkyl group having one carbon atom include $CFH_2$-, $CF_2H$-, and $CF_3$-. In respect of hightemperature storage characteristics, particularly preferred is $CFH_2$- or $CF_3$-.

In respect of favorable solubility of an electrolyte salt, a preferred example of the fluorinated alkyl group having 2 or more carbon atoms includes a fluorinated alkyl group represented by the following general formula (d-1):

$R^2$-$R^2$- (d-1)

(wherein $R^1$ is an alkyl group having one or more carbon atoms and optionally having a fluorine atom; $R^2$ is an alkylene group having 1 to 3 carbon atoms and optionally having a fluorine atom;
provided that at least one of $R^1$ and $R^2$ has a fluorine atom.)

$R^1$ and $R^2$ each may further have an atom other than carbon, hydrogen, and fluorine atoms.

$R^1$ is an alkyl group having one or more carbon atoms and optionally having a fluorine atom. $R^1$ is preferably a linear or branched chain alkyl group having 1 to 6 carbon atoms. $R^1$ has more preferably 1 to 3 carbon atoms.

Specific examples of linear or branched chain alkyl groups for $R^1$ include $CH_3$-, $CF_3$-, $CH_3CH_2$-, $CH_3CH_2CH_2$-, $CH_3CH_2CH_2CH_2$-,

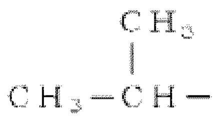 、 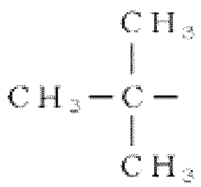

and the like.

When $R^1$ is a linear alkyl group having a fluorine atom, examples of $R^1$ include $CF_3$-, $CF_3CH_2$-, $CF_3CF_2$-, $CF_3CH_2CH_2$-, $CF_3CF_2CH_2$-, $CF_3CF_2CF_2$-, $CF_3CH_2CF_2$-, $CF_3CH_2CH_2CH_2$-, $CF_3CF_2CH_2CH_2$-, $CF_3CH_2CF_2CH_2$-, $CF_3CF_2CF_2CH_2$-, $CF_3CF_2CF_2CF_2$-, $CF_3CF_2CH_2CF_2$-, $CF_3CH_2CH_2CH_2CH_2$-, $CF_3CF_2CH_2CH_2CH_2$-, $CF_3CH_2CF_2CH_2CH_2$-, $CF_3CF_2CF_2CH_2CH_2$-, $CF_3CF_2CF_2CF_2CH_2$-, $CF_3CF_2CH_2CF_2CH_2$-, $CF_3CF_2CH_2CH_2CH_2CH_2$-, $CF_3CF_2CF_2CF_2CH_2CH_2$-, $CF_3CF_2CH_2CF_2CH_2CH_2$-, $HCF_2$-, $HCF_2CH_2$-, $HCF_2CF_2$-, $HCF_2CH_2CH_2$-, $HCF_2CF_2CH_2$-, $HCF_2CH_2CF_2$-, $HCF_2CF_2CH_2CH_2$-, $HCF_2CH_2CF_2CH_2$-, $HCF_2CF_2CF_2CF_2$-, $HCF_2CF_2CH_2CH_2CH_2$-, $HCF_2CH_2CF_2CH_2CH_2$-, $HCF_2CF_2CF_2CF_2CH_2$-, $HCF_2CF_2CF_2CF_2CH_2CH_2$-, $FCH_2$-, $FCH_2CH_2$-, $FCH_2CF_2$-, $FCH_2CF_2CH_2$-, $FCH_2CF_2CF_2$-, $CH_3CF_2CH_2$-, $CH_3CF_2CF_2$-, $CH_3CF_2CH_2CF_2$-, $CH_3CF_2CF_2CF_2$-, $CH_3CH_2CF_2CF_2$-, $CH_3CF_2CH_2CF_2CH_2$-, $CH_3CF_2CF_2CF_2CH_2$-, $CH_3CF_2CF_2CH_2CH_2$-, $CH_3CH_2CF_2CF_2CH_2$-, $CH_3CF_2CH_2CF_2CH_2CH_2$-, $CH_3CF_2CH_2CF_2CH_2CH_2$-, $HCFClCF_2CH_2$-, $HCF_2CFClCH_2$-, $HCF_2CFClCF_2CFClCH_2$-, and $HCFClCF_2CFClCF_2CH_2$- .

When $R^1$ is a branched chain alkyl group having a fluorine atom, preferred examples of $R^1$ include

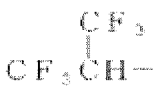 、 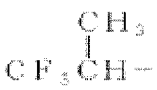 、 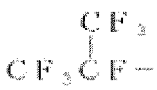 、 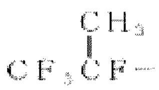 、

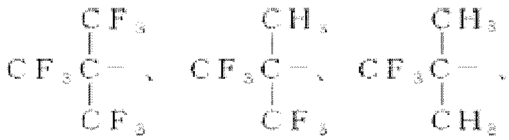

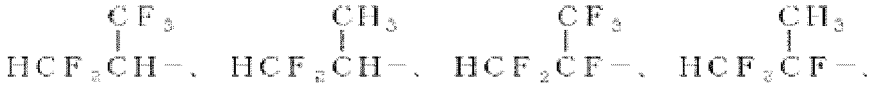

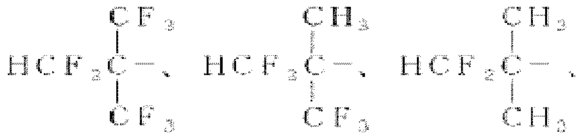

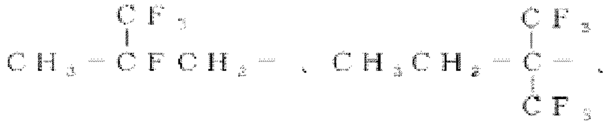

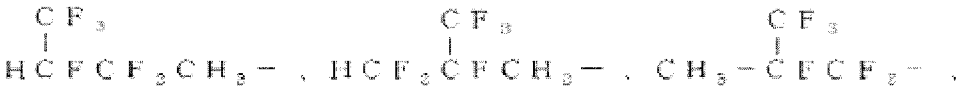

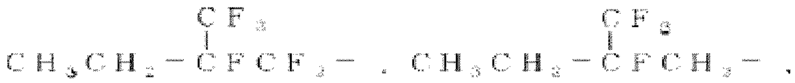

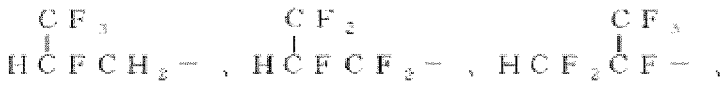

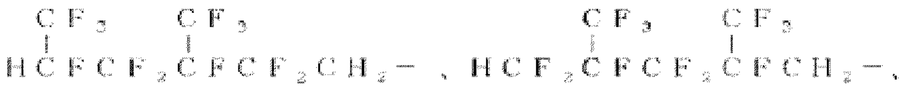

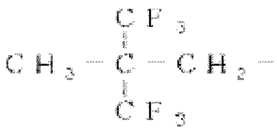

However, if a branch such as $CH_3$- or $CF_3$- is contained, the viscosity is likely to increase. Thus, the number of such branches is more preferably small (one) or zero.

$R^2$ is an alkylene group having 1 to 3 carbon atoms and optionally having a fluorine atom. $R^2$ may be linear or branched chain. Examples of a minimum structural unit constituting such a linear or branched chain alkylene group are shown below. $R^2$ is constituted by one or a combination of these units.

(i) Linear Minimum Structural Units:

$-CH_2-$, $-CHF-$, $-CF_2-$, $-CHCl-$, $-CFCl-$, $-CCl2-$

(ii) Branched Chain Minimum Structural Units:

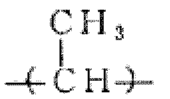, 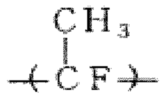, 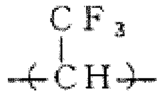, 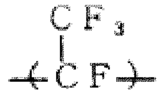,

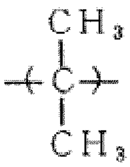, 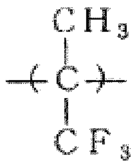, 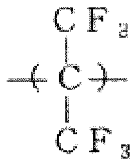,

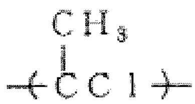, 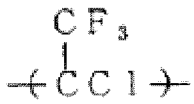

$R^2$ is preferably constituted by Cl-free structural units among these examples, because such units may not be dehydrochlorinated by a base and thus may be more stable.

When being linear, $R^2$ is composed only of any of the above linear minimum structural units, and is preferably $-CH_2-$, $-CH_2CH_2-$, or $CF_2-$ among these. Since the solubility of an electrolyte salt can be further improved, $-CH_2-$ or $-CH_2CH_2-$ is more preferred.

When being branched chain, $R^2$ includes at least one of the above branched chain minimum structural units. A preferred example thereof is a group represented by the general formula: $-(CX^aX^b)-$, wherein $X^a$ is H, F, $CH_3$, or $CF_3$; $X^b$ is $CH_3$ or $CF_3$; provided that, when $X^b$ is $CF_3$, $X^a$ is H or $CH_3$. Such groups can much further particularly improve the solubility of an electrolyte salt.

Preferred specific examples of the fluorinated alkyl group include $CF_3CF_2-$, $HCF_2CF_2-$, $H_2CFCF_2-$, $CH_3CF_2-$, $CF_3CH_2-$, $CF_3CF_2CF_2-$, $HCF_2CF_2CF_2-$, $H_2CFCF_2CF_2-$, $CH_3CF_2CF_2-$,

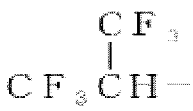, 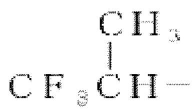, 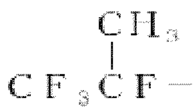,

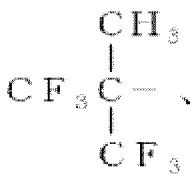, 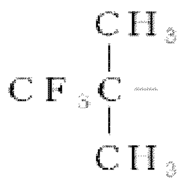,

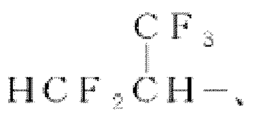, 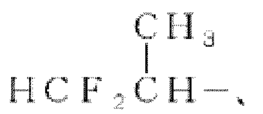, 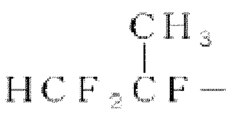,

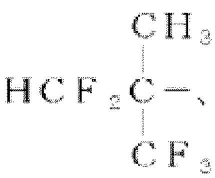, 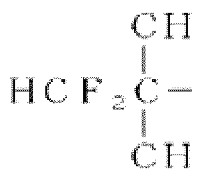,

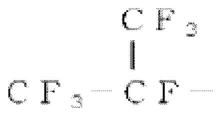,

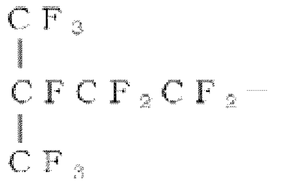,

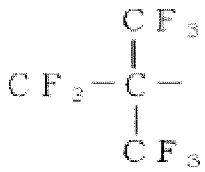,

and the like.

Among these, the fluorinated alkyl group for $Rf^2$ and $R^7$ is preferably $CF_3$-, $CF_3CF_2$-, $(CF_3)_2CH$-, $CF_3CH_2$-, $C_2F_5CH_2$-, $CF_3CF_2CH_2$-, $HCF_2CF_2CH_2$-, $CF_3CFHCF_2CH_2$-, $CFH_2$-, or $CF_2H$-, more preferably $CF_3CH_2$-, $CF_3CF_2CH_2$-, $HCF_2CF_2CH_2$-, $CFH_2$-, or $CF_2H$-, in view of high flame retardancy and favorable rate characteristics and oxidation resistance.

When $R^7$ is an alkyl group containing no fluorine atom, the group is an alkyl group having 1 to 7 carbon atoms. $R^7$ has preferably 1 to 4 carbon atoms, more preferably 1 to 3 carbon atoms, in view of giving a low viscosity.

Examples of the alkyl group containing no fluorine atom include $CH_3$-, $CH_3CH_2$-, $(CH_3)_2CH$-, and $C_3H_7$-. Among these, $CH_3$- and $CH_3CH_2$- are preferred, in view of giving a low viscosity and favorable rate characteristics.

The fluorinated chain carbonate preferably has a fluorine content of 15 to 70% by mass. The fluorinated chain carbonate, when having a fluorine content in the range described above, can maintain the solubility with a solvent and the solubility of a salt. The fluorine content is more preferably 20% by mass or more, still more preferably 30% by mass or more, even more preferably 35% by mass or more, while particularly preferably 60% by mass or less, even more preferably 50% by mass or less.

In the present disclosure, the fluorine content is a value calculated based on the structural formula of the fluorinated chain carbonate by:

$$[(\text{Number of fluorine atoms} \times 19)/(\text{Molecular weight of fluorinated chain carbonate})] \times 100 (\% \text{ by mass}).$$

The fluorinated chain carbonate is preferably any of the following compounds, in view of giving a low viscosity.

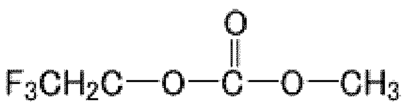 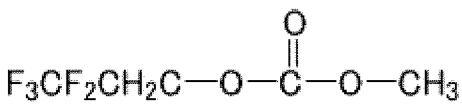

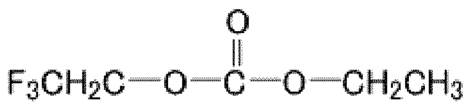 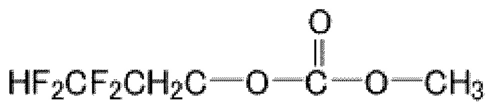

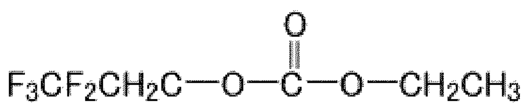 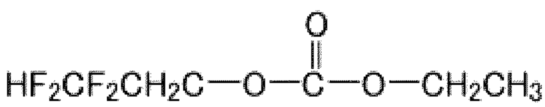

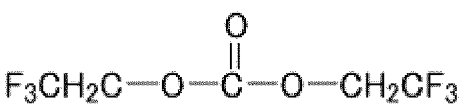 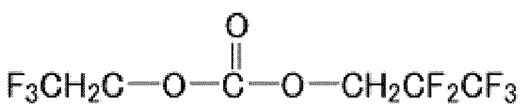

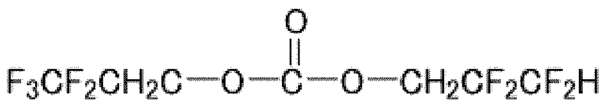

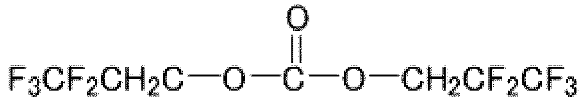

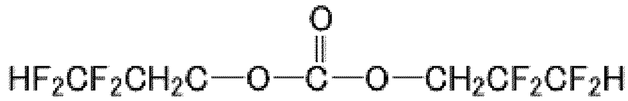

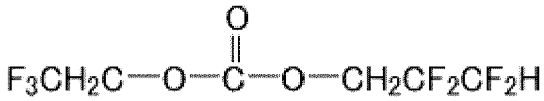

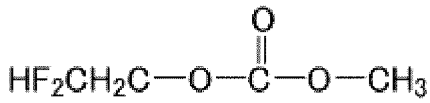

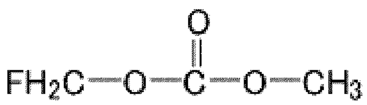

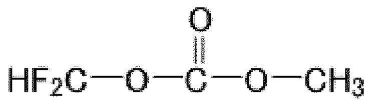

The fluorinated chain carbonate is particularly preferably methyl 2,2,2-trifluoroethyl carbonate ($F_3CH_2COC(=O)OCH_3$).

One of the fluorinated chain carbonates may be used singly, or two or more thereof may be used in any combination at any ratio.

When the fluorinated chain carbonate is contained, the content of the fluorinated chain carbonate is preferably 10 to 90% by volume, more preferably 40 to 85% by volume, further preferably 50 to 80% by volume with respect to the solvent, in view of electrochemical device performance.

The carboxylic acid ester may be a cyclic carboxylic acid ester or a chain carboxylic acid ester.

The cyclic carboxylic acid ester may be a non-fluorinated cyclic carboxylic acid ester or a fluorinated cyclic carboxylic acid ester.

An example of the non-fluorinated cyclic carboxylic acid ester includes a non-fluorinated saturated cyclic carboxylic acid ester, in view of electrochemical device performance. Preferred is a non-fluorinated saturated cyclic carboxylic acid ester having an alkylene group having 2 to 4 carbon atoms.

Specific examples of the non-fluorinated saturated cyclic carboxylic acid ester having an alkylene group having 2 to 4 carbon atoms include β-propiolactone, γ-butyrolactone, ε-caprolactone, δ-valerolactone, and α-methyl-γ-butyrolactone. Among these, γ-butyrolactone and δ-valerolactone are particularly preferred, in view of improvement of the degree of dissociation of lithium ions and improvement of the load characteristics.

One of the non-fluorinated saturated cyclic carboxylic acid esters may be used singly, or two or more thereof may be used in any combination at any ratio.

When the non-fluorinated saturated cyclic carboxylic acid ester is contained, the content of the non-fluorinated saturated cyclic carboxylic acid ester is preferably 0 to 90% by volume, more preferably 0.001 to 90% by volume, further preferably 1 to 60% by volume, particularly preferably 5 to 40% by volume with respect to the solvent, in view of electrochemical device performance.

The chain carboxylic acid ester may be a non-fluorinated chain carboxylic acid ester or a fluorinated chain carboxylic acid ester. When containing the chain carboxylic acid ester, the solvent enables the electrolyte solution to have a further suppressed increase in resistance after high-temperature storage.

Examples of the non-fluorinated chain carboxylic acid ester include methyl acetate, ethyl acetate, propyl acetate, butyl acetate, methyl propionate, ethyl propionate, propyl propionate, butyl propionate, tert-butyl propionate, tert-butyl

butyrate, sec-butyl propionate, sec-butyl butyrate, n-butyl butyrate, methyl pyrophosphate, ethyl pyrophosphate, tert-butyl formate, tert-butyl acetate, sec-butyl formate, sec-butyl acetate, n-hexyl pivalate, n-propyl formate, n-propyl acetate, n-butyl formate, n-butyl pivalate, n-octyl pivalate, ethyl 2-(dimethoxyphosphoryl)acetate, ethyl 2-(dimethylphosphoryl)acetate, ethyl 2-(diethoxyphosphoryl)acetate, ethyl 2-(diethylphosphoryl)acetate, isopropyl propionate, isopropyl acetate, ethyl formate, ethyl 2-propynyl oxalate, isopropyl formate, isopropyl butyrate, isobutyl formate, isobutyl propionate, isobutyl butyrate, and isobutyl acetate.

Among these, butyl acetate, methyl propionate, ethyl propionate, propyl propionate, and butyl propionate are preferred, and ethyl propionate and propyl propionate are particularly preferred, in view of electrochemical device performance.

One of the non-fluorinated chain carboxylic acid esters may be used singly, or two or more thereof may be used in any combination at any ratio.

When the non-fluorinated chain carboxylic acid ester is contained, the content of the non-fluorinated chain carboxylic acid ester is preferably 0 to 90% by volume, more preferably 0.001 to 90% by volume, further preferably 1 to 60% by volume, particularly preferably 5 to 40% by volume with respect to the solvent, in view of electrochemical device performance.

The fluorinated chain carboxylic acid ester is a chain carboxylic acid ester having a fluorine atom. A solvent containing a fluorinated chain carboxylic acid ester can be suitably used even at a high voltage.

In view of favorable solubility with other solvents and favorable oxidation resistance, the fluorinated chain carboxylic acid ester is preferably a fluorinated chain carboxylic acid ester represented by the following general formula:

$$R^{31}COOR^{32}$$

(wherein $R^{31}$ and $R^{32}$ are each independently an alkyl group having 1 to 4 carbon atoms and optionally having a fluorine atom, and at least one of $R^{31}$ and $R^{32}$ contains a fluorine atom.)

Examples of $R^{31}$ and $R^{32}$ include non-fluorinated alkyl groups such as a methyl group ($-CH_3$), an ethyl group ($-CH_2CH_3$), a propyl group ($-CH_2CH_2CH_3$), an isopropyl group ($-CH(CH_3)_2$), an n-butyl group ($-CH_2CH_2CH_2CH_3$), and a tertiary butyl group ($-C(CH_3)_3$); and fluorinated alkyl groups such as $-CF_3$, $-CF_2H$, $-CFH_2$, $-CF_2CF_3$, $-CF_2CF_2H$, $-CF_2CFH_2$, $-CH_2CF_3$, $-CH_2CF_2H$, $-CH_2CFH_2$, $-CF_2CF_2CF_3$, $-CF_2CF_2CF_2H$, $-CF_2CF_2CFH_2$, $-CH_2CF_2CF_3$, $-CH_2CF_2CF_2H$, $-CH_2CF_2CFH_2$, $-CH_2CH_2CF_3$, $-CH_2CH_2CF_2H$, $-CH_2CH_2CFH_2$, $-CF(CF_3)_2$, $-CF(CF_2H)_2$, $-CF(CFH_2)_2$, $-CH(CF_3)_2$, $-CH(CF_2H)_2$, $-CH(CFH_2)_2$, $-CF(OCH_3)CF_3$, $-CF_2CF_2CF_2CF_3$, $-CF_2CF_2CF_2CF_2H$, $-CF_2CF_2CF_2CFH_2$, $-CH_2CF_2CF_2CF_3$, $-CH_2CF_2CF_2CF_2H$, $-CH_2CF_2CF_2CFH_2$, $-CH_2CH_2CF_2CF_3$, $-CH_2CH_2CF_2CF_2H$, $-CH_2CH_2CF_2CFH_2$, $-CH_2CH_2CH_2CF_3$, $-CH_2CH_2CH_2CF_2H$, $-CH_2CH_2CH_2CFH_2$, $-CF(CF_3)CF_2CF_3$, $-CF(CF_2H)CF_2CF_3$, $-CF(CFH_2)CF_2CF_3$, $-CF(CF_3)CF_2CF_2H$, $-CF(CF_3)CF_2CFH_2$, $-CF(CF_3)CH_2CF_3$, $-CF(CF_3)CH_2CF_2H$, $-CF(CF_3)CH_2CFH_2$, $-CH(CF_3)CF_2CF_3$, $-CH(CF_2H)CF_2CF_3$, $-CH(CFH_2)CF_2CF_3$, $-CH(CF_3)CF_2CF_2H$, $-CH(CF_3)CF_2CFH_2$, $-CH(CF_3)CH_2CF_3$, $-CH(CF_3)CH_2CF_2H$, $-CH(CF_3)CH_2CFH_2$, $-CF_2CF(CF_3)CF_3$, $-CF_2CF(CF_2H)CF_3$, $-CF_2CF(CFH_2)CF_3$, $-CF_2CF(CF_3)CF_2H$, $-CF_2CF(CF_3)CFH_2$, $-CH_2CF(CF_3)CF_3$, $-CH_2CF(CF_2H)CF_3$, $-CH_2CF(CFH_2)CF_3$, $-CH_2CF(CF_3)CF_2H$, $-CH_2CF(CF_3)CFH_2$, $-CH_2CH(CF_3)CF_3$, $-CH_2CH(CF_2H)CF_3$, $-CH_2CH(CFH_2)CF_3$, $-CH_2CH(CF_3)CF_2H$, $-CH_2CH(CF_3)CFH_2$, $-CF_2CH(CF_3)CF_3$, $-CF_2CH(CF_2H)CF_3$, $-CF_2CH(CFH_2)CF_3$, $-CF_2CH(CF_3)CF_2H$, $-CF_2CH(CF_3)CFH_2$, $-C(CF_3)_3$, $-C(CF_2H)_3$, and $-C(CFH_2)_3$. Particularly preferred among these are a methyl group, an ethyl group, $-CF_3$, $-CF_2H$, $-CF_2CF_3$, $-CH_2CF_3$, $-CH_2CF_2H$, $-CH_2CFH_2$, $-CH_2CH_2CF_3$, $-CH_2CF_2CF_3$, $-CH_2CF_2CF_2H$, and $-CH_2CF_2CFH_2$, in view of favorable compatibility with other solvents, viscosities, and oxidation resistance.

Specific examples of the fluorinated chain carboxylic acid ester include one or two or more of $CF_3CH_2C(=O)OCH_3$ (methyl 3,3,3-trifluoropropionate), $HCF_2C(=O)OCH_3$ (methyl difluoroacetate), $HCF_2C(=O)OC_2H_5$ (ethyl difluoroacetate), $CF_3C(=O) OCH_2CH_2CF_3$, $CF_3C(=O) OCH_2C_2F_5$, $CF_3C(=O) OCH_2CF_2CF_2H$ (2,2,3,3-tetrafluoropropyl trifluoroacetate), $CF_3C(=O) OCH_2CF_3$, $CF_3C(=O) OCH(CF_3)_2$, ethyl pentafluorobutyrate, methyl pentafluoropropionate, ethyl pentafluoropropionate, methyl heptafluoroisobutyrate, isopropyl trifluorobutyrate, ethyl trifluoroacetate, tert-butyl trifluoroacetate, n-butyl trifluoroacetate, methyl tetrafluoro-2-(methoxy)propionate, 2,2-difluoroethyl acetate, 2,2,3,3-tetrafluoropropyl acetate, $CH_3C(=O)OCH_2CF_3$ (2,2,2-trifluoroethyl acetate), 1H,1H-heptafluorobutyl acetate, methyl 4,4,4-trifluorobutyrate, ethyl 4,4,4-trifluorobutyrate, ethyl 3,3,3-trifluoropropionate, 3,3,3-trifluoropropyl 3,3,3-trifluoropropionate, ethyl 3-(trifluoromethyl)butyrate, methyl 2,3,3,3-tetrafluoropropionate, butyl 2,2-difluoroacetate, methyl 2,2,3,3-tetrafluoropropionate, methyl 2-(trifluoromethyl)-3,3,3-trifluoropropionate, and methyl heptafluorobutyrate.

Among these, in view of electrochemical device performance, preferred are $CF_3CH_2C(=O)OCH_3$, $HCF_2C(=O)OCH_3$, $HCF_2C(=O)OC_2H_5$, $CF_3C(=O) OCH_2C_2F_5$, $CF_3C(=O) OCH_2CF_2CF_2H$, $CF_3C(=O) OCH_2CF_3$, $CF_3C(=O)OCH(CF_3)_2$, ethyl pentafluorobutyrate, methyl pentafluoropropionate, ethyl pentafluoropropionate, methyl heptafluoroisobutyrate, isopropyl trifluorobutyrate, ethyl trifluoroacetate, tert-butyl trifluoroacetate, n-butyl trifluoroacetate, methyl tetrafluoro-2-(methoxy)propionate, 2,2-difluoroethyl acetate, 2,2,3,3-tetrafluoropropyl acetate, $CH_3C(=O) OCH_2CF_3$, 1H, 1H-heptafluorobutyl acetate, methyl 4,4,4-trifluorobutyrate, ethyl 4,4,4-trifluorobutyrate, ethyl 3,3,3-trifluoropropionate, 3,3,3-trifluoropropyl 3,3,3-trifluoropropionate, ethyl 3-(trifluoromethyl)butyrate, methyl 2,3,3,3-tetrafluoropropionate, butyl 2,2-difluoroacetate, methyl 2,2,3,3,-tetrafluoropropionate, methyl 2-(trifluoromethyl)-3,3,3-trifluoropropionate, and methyl heptafluorobutyrate, in view of favorable compatibility with other solvents and rate characteristics, more preferred are $CF_3CH_2C(=O)\ OCH_3$, $HCF_2C(=O)\ OCH_3$, $HCF_2C(=O)OC_2H_5$, and $CH_3C(=O)OCH_2CF_3$, and particularly preferred are $HCF_2C(=O)OCH_3$, $HCF_2C(=O)\ OC_2H_5$, and $CH_3C(=O)OCH_2CF_3$.

One of the fluorinated chain carboxylic acid esters may be used singly, or two or more thereof may be used in any combination at any ratio.

When the fluorinated chain carboxylic acid ester is contained, the content of the fluorinated chain carboxylic acid ester is preferably 10 to 90% by volume, more preferably 40 to 85% by volume, further preferably 50 to 80% by volume with respect to the solvent.

In view of electrochemical device performance, the solvent preferably contains at least one member selected from the group consisting of the cyclic carbonate, the chain carbonate, and the chain carboxylic acid ester, and more preferably contains the cyclic carbonate and at least one member selected from the group consisting of the chain carbonate and the chain carboxylic acid ester. The cyclic carbonate is preferably a saturated cyclic carbonate.

The electrolyte solution containing a solvent of the composition described above can further improve high-temperature storage characteristics and cycle characteristics of electrochemical devices.

When the solvent contains the cyclic carbonate and at least one member selected from the group consisting of the chain carbonate and the chain carboxylic acid ester, the solvent preferably contains the cyclic carbonate and at least one member selected from the group consisting of the chain carbonate and the chain carboxylic acid ester in a total amount of preferably 10 to 100% by volume, more preferably 30 to 100% by volume, still more preferably 50 to 100% by volume, in view of electrochemical device performance.

When the solvent contains the cyclic carbonate and at least one member selected from the group consisting of the chain carbonate and the chain carboxylic acid ester, the volume ratio of the cyclic carbonate to the at least one member selected from the group consisting of the chain carbonate and the chain carboxylic acid ester is preferably 5/95 to 95/5, more preferably 10/90 or more, still more preferably 15/85 or more, particularly preferably 20/80 or more, while more preferably 90/10 or less, still more preferably 60/40 or less, particularly preferably 50/50 or less.

The solvent also preferably contains at least one member selected from the group consisting of the non-fluorinated saturated cyclic carbonate, the non-fluorinated chain carbonate, and the non-fluorinated chain carboxylic acid ester. In view of electrochemical device performance, the solvent more preferably contains the non-fluorinated saturated cyclic carbonate and at least one member selected from the group consisting of the non-fluorinated chain carbonate and the non-fluorinated chain carboxylic acid ester. The electrolyte solution containing a solvent of the composition described above can be suitably used for electrochemical devices used at a relatively low voltage.

When the solvent contains the fluorinated saturated cyclic carbonate and at least one member selected from the group consisting of the non-fluorinated chain carbonate and the non-fluorinated chain carboxylic acid ester, the solvent preferably contains the non-fluorinated saturated cyclic carbonate and the at least one member selected from the group consisting of the non-fluorinated chain carbonate and the non-fluorinated chain carboxylic ester in a total amount of preferably 5 to 100% by volume, more preferably 20 to 100% by volume, still more preferably 30 to 100% by volume, in view of electrochemical device performance.

When the electrolyte solution contains the non-fluorinated saturated cyclic carbonate and at least one member selected from the group consisting of the non-fluorinated chain carbonate and the non-fluorinated chain carboxylic acid ester, the volume ratio of the non-fluorinated saturated cyclic carbonate to the at least one member selected from the group consisting of the non-fluorinated chain carbonate and the non-fluorinated chain carboxylic acid ester is preferably 5/95 to 95/5, more preferably 10/90 or more, still more preferably 15/85 or more, particularly preferably 20/80 or more, while more preferably 90/10 or less, still more preferably 60/40 or less, particularly preferably 50/50 or less, in view of electrochemical device performance.

In view of electrochemical device performance, the solvent also preferably contains at least one member selected from the group consisting of the fluorinated saturated cyclic carbonate, the fluorinated chain carbonate, and the fluorinated chain carboxylic acid ester, and more preferably contains the fluorinated saturated cyclic carbonate and at least one member selected from the group consisting of the fluorinated chain carbonate and the fluorinated chain carboxylic acid ester. The electrolyte solution containing a solvent of the composition described above can be suitably used not only for electrochemical devices used at a relatively low voltage, but also for electrochemical devices used at a relatively high voltage.

When the solvent contains the fluorinated saturated cyclic carbonate and at least one member selected from the group consisting of the fluorinated chain carbonate and the fluorinated chain carboxylic acid ester, the solvent preferably contains the fluorinated saturated cyclic carbonate and the at least one member selected from the group consisting of the fluorinated chain carbonate and the fluorinated chain carboxylic acid ester in a total amount of preferably 5 to 100% by volume, more preferably 10 to 100% by volume, still more preferably 30 to 100% by volume, in view of electrochemical device performance.

When the solvent contains the fluorinated saturated cyclic carbonate and at least one member selected from the group consisting of the fluorinated chain carbonate and the fluorinated chain carboxylic acid ester, the volume ratio of the fluorinated saturated cyclic carbonate to the at least one member selected from the group consisting of the fluorinated chain carbonate and the fluorinated chain carboxylic acid ester is preferably 5/95 to 95/5, more preferably 10/90 or more, still more preferably 15/85 or more, particularly preferably 20/80 or more, while more preferably 90/10 or less, still more preferably 60/40 or less, particularly preferably 50/50 or less.

As the solvent, an ionic liquid can also be used. The "ionic liquid" is a liquid composed of ions containing an organic cation and an anion in combination.

Examples of the organic cation include, but are not limited to, imidazolium ions such as dialkyl imidazolium cations and trialkyl imidazolium cations; tetraalkyl ammonium ions; alkyl pyridinium ions; dialkyl pyrrolidinium ions; and dialkyl piperidinium ions.

Examples of the anion that serves as a counter-ion of any of these organic cations include, but are not limited to, a $PF_6$ anion, a $PF_3(C_2F_5)_3$ anion, a $PF_3(CF_3)_3$ anion, a $BF_4$ anion, a $BF_2(CF_3)_2$ anion, a $BF_3(CF_3)$ anion, a bisoxalatoborate anion, a $P(C_2O_4)F_2$ anion, a Tf (trifluoromethanesulfonyl) anion, an Nf (nonafluorobutanesulfonyl) anion, a bis(fluorosulfonyl)imide anion, a bis(trifluoromethanesulfonyl)imide anion, a bis(pentafluoroethanesulfonyl) imide anion, a dicyanoamine anion, and halide anions.

In view of electrochemical device performance, the solvent is preferably a non-aqueous solvent, and the electrolyte solution of the present disclosure is preferably a non-aqueous electrolyte solution.

In view of electrochemical device performance, the content of the solvent in the electrolyte solution is preferably 70 to 99.999% by mass, more preferably 80% by mass or more, while still more preferably 92% by mass or less.

The electrolyte solution of the present disclosure may further contain a compound (5) represented by general formula (5) .

General formula (5):

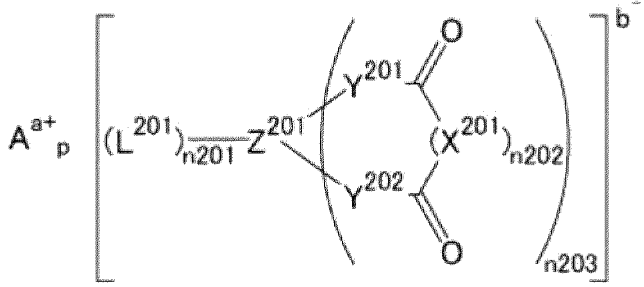

(wherein

$A^{a+}$ is a metal ion, a hydrogen ion, or an onium ion;

a is an integer of 1 to 3, b is an integer of 1 to 3, p is b/a, n203 is an integer of 1 to 4, n201 is an integer of 0 to 8, n202 is 0 or 1, $Z^{201}$ is a transition metal or an element in group III, group IV, or group V of the Periodic Table;

$X^{201}$ is O, S, an alkylene group having 1 to 10 carbon atoms, a halogenated alkylene group having 1 to 10 carbon atoms, an arylene group having 6 to 20 carbon atoms, or a halogenated arylene group having 6 to 20 carbon atoms (wherein the alkylene group, the halogenated alkylene group, the arylene group, and the halogenated arylene group each optionally have a substituent and/or a hetero atom in the structure thereof; and when n202 is 1 and n203 is 2 to 4, n203 $X^{201}$s optionally bind to each other);

$L^{201}$ is a halogen atom, a cyano group, an alkyl group having 1 to 10 carbon atoms, a halogenated alkyl group having 1 to 10 carbon atoms, an aryl group having 6 to 20 carbon atoms, a halogenated aryl group having 6 to 20 carbon atoms (wherein the alkylene group, the halogenated alkylene group, the arylene group, and the halogenated arylene group each optionally have a substituent and/or a hetero atom in the structure thereof; and when n201 is 2 to 8, n201 $L^{201}$s optionally bind to each other to form a ring), or $-Z^{203}Y^{203}$;

$Y^{201}$, $Y^{202}$ and $Z^{203}$ are each independently O, S, $NY^{204}$, a hydrocarbon group, or a fluorinated hydrocarbon group; and

$Y^{203}$ and $Y^{204}$ are each independently H, F, an alkyl group having 1 to 10 carbon atoms, a halogenated alkyl group having 1 to 10 carbon atoms, an aryl group having 6 to 20 carbon atoms, or a halogenated aryl group having 6 to 20 carbon atoms (wherein the alkyl group, the halogenated alkyl group, the aryl group, and the halogenated aryl

group each optionally have a substituent and/or a hetero atom in the structure thereof, and when multiple $Y^{203}$s or $Y^{204}$s are present, the multiple $Y^{203}$s or $Y^{204}$s optionally bind to each other to form a ring)).

Examples of $A^{a+}$ include a lithium ion, a sodium ion, a potassium ion, a magnesium ion, a calcium ion, a barium ion, a cesium ion, a silver ion, a zinc ion, a copper ion, a cobalt ion, an iron ion, a nickel ion, a manganese ion, a titanium ion, a lead ion, a chromium ion, a vanadium ion, a ruthenium ion, an yttrium ion, lanthanoid ions, actinoid ions, a tetrabutyl ammonium ion, a tetraethyl ammonium ion, a tetramethyl ammonium ion, a triethyl methyl ammonium ion, a triethyl ammonium ion, a pyridinium ion, an imidazolium ion, a hydrogen ion, a tetraethyl phosphonium ion, a tetramethyl phosphonium ion, a tetraphenyl phosphonium ion, a triphenyl sulfonium ion, and a triethyl sulfonium ion.

In applications such as electrochemical devices, $A^{a+}$ is preferably a lithium ion, a sodium ion, a magnesium ion, a tetraalkyl ammonium ion, or a hydrogen ion, particularly preferably a lithium ion. The valence a of the cation $A^{a+}$ is an integer of 1 to 3 in view of ease of dissolution in the solvent by adjustment of crystal lattice energy. When the solubility is important, the valence a is particularly preferably 1. Similarly, the valence b of the anion is also an integer of 1 to 3, and particularly preferably 1. The constant p, which represents the ratio of anion to cation, is necessarily determined by the ratio b/a of their valences.

Next, ligands in general formula (5) are described. In the present specification, an organic or inorganic moiety binding to $Z^{201}$ in general formula (5) is referred to as a ligand.

In view of electrochemical device performance, $Z^{201}$ is preferably Al, B, V, Ti, Si, Zr, Ge, Sn, Cu, Y, Zn, Ga, Nb, Ta, Bi, P, As, Sc, Hf, or Sb, more preferably Al, B, or P.

$X^{201}$ represents O, S, an alkylene group having 1 to 10 carbon atoms, a halogenated alkylene group having 1 to 10 carbon atoms, an arylene group having 6 to 20 carbon atoms, or a halogenated arylene group having 6 to 20 carbon atoms. These alkylene groups and arylene groups each may have a substituent and/or a hetero atom in the structure thereof. Specifically, instead of a hydrogen atom in the alkylene group or the arylene group, the structure may have a halogen atom, a chain or cyclic alkyl group, an aryl group, an alkenyl group, an alkoxy group, an aryloxy group, a sulfonyl group, an amino group, a cyano group, a carbonyl group, an acyl group, an amide group, or a hydroxyl group as a substituent. Alternatively, instead of a carbon atom in the alkylene group or the arylene group, the structure may have nitrogen, sulfur, or oxygen introduced thereinto. When n202 is 1 and n203 is 2 to 4, n203 $X^{201}$s may bind to each other. Examples of such structures include ligands, such as ethylenediaminetetraacetate.

$L^{201}$ represents a halogen atom, a cyano group, an alkyl group having 1 to 10 carbon atoms, a halogenated alkyl group having 1 to 10 carbon atoms, an aryl group having 6 to 20 carbon atoms, a halogenated aryl group having 6 to 20 carbon atoms, or $-z^{203}Y^{203}$ (wherein $Z^{203}$ and $Y^{203}$ are described below) . Similar to $X^{201}$, the alkyl group and the aryl group herein each may have a substituent and/or a hetero atom in the structure thereof. When n201 is 2 to 8, n201 $L^{201}$s may bind to each other to form a ring. $L^{201}$ is preferably a fluorine atom or a cyano group. This is because, in the case of a fluorine atom, the solubility and the degree of dissociation of a salt of an anion compound are enhanced, which tend to improve the ion conductivity. Further, the oxidation resistance is enhanced to thereby easily suppress the occurrence of side reactions.

$Y^{201}$, $Y^{202}$, and $Z^{203}$ each independently represent O, S, $NY^{204}$, a hydrocarbon group, or a fluorinated hydrocarbon group. In view of electrochemical device performance, $Y^{201}$ and $Y^{202}$ are each preferably O, S, or $NY^{204}$, more preferably O. Since the compound (5) characteristically has a bond between $Y^{201}$ and $Z^{201}$ and a bond between $Y^{202}$ and $Z^{201}$ in the same ligand, this ligand form a chelate structure with $Z^{201}$. The effect of this chelate tends to improve the heat resistance, the chemical stability, and the hydrolysis resistance of this compound. The constant n202 of the ligand is 0 or 1. In particular, when n202 is 0, this chelate ring becomes a five-membered ring, thus leading to the most strongly exerted chelate effect and enhanced stability.

In the present specification, the fluorinated hydrocarbon group means a group wherein at least one hydrogen atom of a hydrocarbon group is replaced with a fluorine atom.

$Y^{203}$ and $Y^{204}$ are each independently H, F, an alkyl group having 1 to 10 carbon atoms, a halogenated alkyl group having 1 to 10 carbon atoms, an aryl group having 6 to 20 carbon atoms, or a halogenated aryl group having 6 to 20 carbon atoms. These alkyl groups and aryl groups each may contain a substituent or a hetero atom in the structure thereof. When multiple $Y^{203}$s or $Y^{204}$s are present, the multiple $Y^{203}$s or $Y^{204}$s may bind to each other to form a ring.

The constant n203 relating to the number of the ligands is an integer of 1 to 4. In view of electrochemical device performance, the constant n203 is preferably 1 or 2, more preferably 2. The constant n201 relating to the number of the aforementioned ligands is an integer of 0 to 8. In view of electrochemical device performance, the constant n201 is preferably an integer of 0 to 4, more preferably 0, 2, or 4. Further, when n203 is 1, n201 is preferably 2. When n203 is 2, n201 is preferably 0.

In general formula (5), the alkyl group, the halogenated alkyl group, the aryl group, and the halogenated aryl group include those having other functional groups, such as branches, hydroxy groups, and ether bonds.

The compound (5) is preferably a compound represented by general formula:

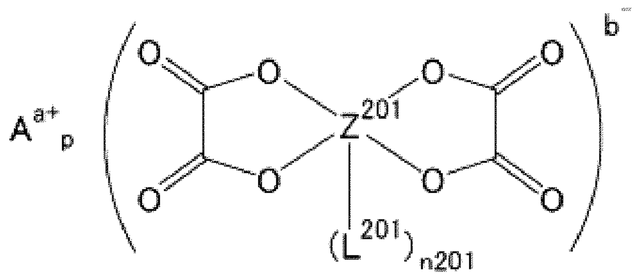

(wherein $A^{a+}$, a, b, p, n201, $Z^{201}$, and $L^{201}$ are as defined above), or
a compound represented by general formula:

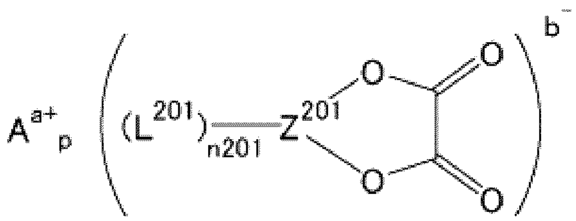

(wherein $A^{a+}$, a, b, p, n201, $Z^{201}$, and $L^{201}$ are as defined above) .

The compound (5) may be a lithium oxalatoborate salt. Examples include lithium bis(oxalato)borate (LIBOB) represented by the following formula:

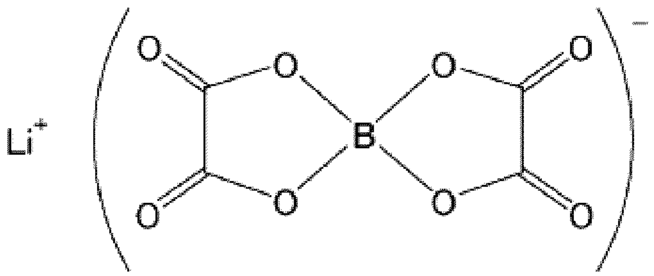

,

lithium difluorooxalatoborate (LIDFOB) represented by the following formula:

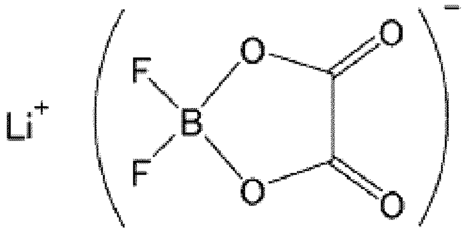

,

and the like.

Examples of the compound (5) also include lithium difluorooxalatophosphanite (LIDFOP) represented by the following formula:

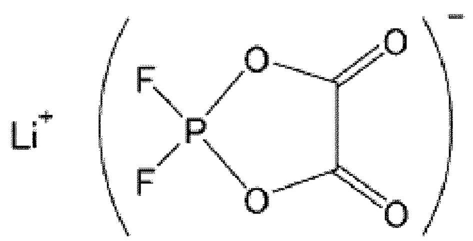

,

lithium tetrafluorooxalatophosphanite (LITFOP) represented by the following formula:

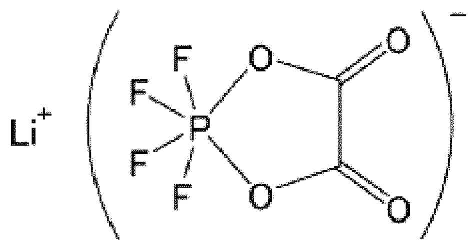

,

lithium bis(oxalato)difluorophosphanite represented by the following formula:

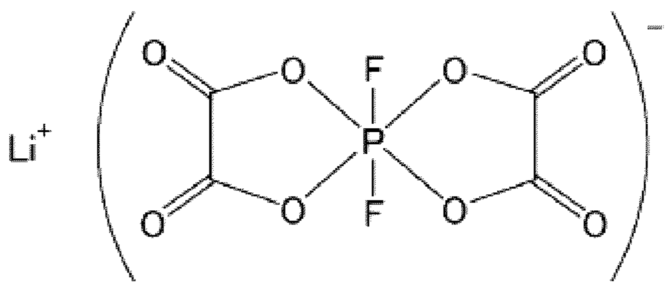

,

and the like.

In addition, specific examples of dicarboxylic acid complex salts containing boron as a complex center element include lithium bis(malonato)borate, lithium difluoro(malonato)borate, lithium bis(methylmalonato)borate, lithium difluoro(methylmalonato)borate, lithium bis(dimethylmalonato)borate, and lithium difluoro(dimethylmalonato)borate.

Specific examples of dicarboxylic acid complex salts containing phosphorus as a complex center element include lithium tris(oxalato)phosphate, lithium tris(malonato)phosphate, lithium difluorobis(malonato)phosphate, lithium tetrafluoro(malonato)phosphate, lithium tris(methylmalonato)phosphate, lithium difluorobis(methylmalonato)phosphate, lithium tetrafluoro(methylmalonato)phosphate, lithium tris(dimethylmalonato)phosphate, lithium difluorobis(dimethylmalonato)phosphate, and lithium tetrafluoro(dimethylmalonato)phosphate.

Specific examples of dicarboxylic acid complex salts containing aluminum as a complex center element include $LiAl(C_2O_4)_2$ and $LiAlF_2(C_2O_4)$.

Among these, lithium bis(oxalato)borate, lithium difluoro(oxalato)borate, lithium tris(oxalato)phosphate, lithium difluorobis(oxalato)phosphate, and lithium tetrafluoro(oxalato)phosphate are more preferably used in view of easy availability and ability to contribute to the formation of a stable film-like structure.

The compound (5) is particularly preferably lithium bis(oxalato)borate.

In view of obtaining even more excellent cycle characteristics, the content of the compound (5) is preferably 0.001% by mass or more, more preferably 0.01% by mass or more, while preferably 10% by mass or less, more preferably 3% by mass or less, with respect to the solvent.

In view of electrochemical device performance, the electrolyte solution of the present disclosure preferably further contains an electrolyte salt (excluding the compounds (1) and (5)). Examples of the electrolyte salt include not only lithium salts, ammonium salts, and metal salts, but also any salt that can be used for an electrolyte solution, such as liquid salts (ionic liquids), inorganic polymer salts, and organic polymer salts.

The electrolyte salt for electrolyte solutions for lithium ion secondary batteries is preferably a lithium salt.

The lithium salt can be any lithium salt. Specific examples include inorganic lithium salts such as $LiPF_6$, $LiBF_4$, $LiClO_4$, $LiAlF_4$, $LiSbF_6$, $LiTaF_6$, $LiWF_7$, $LiAsF_6$, $LiAlCl_4$, LiI, LiBr, LiCl, $LiB_{10}Cl_{10}$, $Li_2SiF_6$, $Li_2PFO_3$, and $LiPO_2F_2$;

lithium tungstates such as $LiWOF_5$;
lithium carboxylates such as $HCO_2Li$, $CH_3CO_2Li$, $CH_2FCO_2Li$, $CHF_2CO_2Li$, $CF_3CO_2Li$, $CF_3CH_2CO_2Li$, $CF_3CF_2CO_2Li$, $CF_3CF_2CF_2CO_2Li$, and
$CF_3CF_2CF_2CF_2CO_2Li$;
lithium salts having a S=O group such as $FSO_3Li$, $CH_3SO_3Li$, $CH_2FSO_3Li$, $CHF_2SO_3Li$, $CF_3SO_3Li$, $CF_3CF_2SO_3Li$, $CF_3CF_2CF_2SO_3Li$, $CF_3CF_2CF_2CF_2SO_3Li$, lithium methylsulfate, lithium ethylsulfate ($C_2H_5OSO_3Li$), and lithium 2,2,2-trifluoroethylsulfate;
lithium imide salts such as $LiN(FCO)_2$, $LiN(FCO)(FSO_2)$, $LiN(FSO_2)_2$, $LiN(FSO_2)(CF_3SO_2)$, $LiN(CF_3SO_2)_2$, $LiN(C_2F_5SO_2)_2$, lithium bisperfluoroethanesulfonyl imide, lithium cyclic 1,2-perfluoroethanedisulfonyl imide, lithium cyclic 1,3-perfluoropropanedisulfonyl imide, lithium cyclic 1,2-ethanedisulfonyl imide, lithium cyclic 1,3-propanedisulfonyl imide, lithium cyclic 1,4-perfluorobutanedisulfonyl imide, $LiN(CF_3SO_2)(FSO_2)$, $LiN(CF_3SO_2)(C_3F_7SO_2)$, $LiN(CF_3SO_2)(C_4F_9SO_2)$, and $LiN(POF_2)_2$;
lithium methide salts such as $LiC(FSO_2)_3$, $LiC(CF_3SO_2)_3$, and $LiC(C_2F_5SO_2)_3$; and
fluorinated alkyl organic lithium salts such as salts represented by the formula: $LiPF_a(C_nF_{2n+1})_{6-a}$ (wherein a is an integer of 0 to 5; and n is an integer of 1 to 6) such as $LiPF_3(C_2F_5)_3$, $LiPF_3(CF_3)_3$, $LiPF_3(iso\text{-}C_3F_7)_3$, $LiPF_5(iso\text{-}C_3F_7)$, $LiPF_4(CF_3)_2$, $LiPF_4(C_2F_5)_2)$, $LiPF_4(CF_3SO_2)_2$, $LiPF_4(C_2F_5SO_2)_2$, $LiBF_3CF_3$, $LiBF_3C_2F_5$, $LiBF_3C_3F_7$, $LiBF_2(CF_3)_2$, $LiBF_2(C_2F_5)_2$, $LiBF_2(CF_3SO_2)_2$, and $LiBF_2(C_2F_5SO_2)_2)$, LiSCN, $LiB(CN)_4$, $LiB(C_6H_5)_4$, $Li_2(C_2O_4)$, $LiP(C_2O_4)_3$, and $Li_2B_{12}F_bH_{12-b}$ (wherein b is an integer of 0 to 3).

Among these, for example, $LiPF_6$, $LiBF_4$, $LiSbF_6$, $LiTaF_6$, $LiPO_2F_2$, $FSO_3Li$, $CF_3SO_3Li$, $LiN(FSO_2)_2$, $LiN(FSO_2)(CF_3SO_2)$, $LiN(CF_3SO_2)_2$, $LiN(C_2F_5SO_2)_2$, lithium cyclic 1,2-perfluoroethanedisulfonyl imide, lithium cyclic 1,3-perfluoropropanedisulfonyl imide, $LiC(FSO_2)_3$, $LiC(CF_3SO_2)_3$, $LiC(C_2F_5SO_2)_3$, $LiBF_3CF_3$, $LiBF_3C_2F_5$, $LiPF_3(CF_3)_3$, and $LiPF_3(C_2F_5)_3$ are particularly preferred in view of the effects of enhancing output characteristics, high-rate charge and discharge characteristics, high-temperature storage characteristics, and cycle characteristics, and at least one lithium salt selected from the group consisting of $LiPF_6$, $LiN(FSO_2)_2$, and $LiBF_4$ are most preferred.

These electrolyte salts can be used singly or in a combination of two or more. Preferred examples in combination use of two or more electrolyte salts include a combination of $LiPF_6$ and $LiBF_4$ and a combination of $LiPF_6$ and $LiPO_2F_2$, a combination of $LiPF_6$ and $C_2H_5OSO_3Li$, and a combination of $LiPF_6$ and $FSO_3Li$. These combinations have the effect of improving the high-temperature storage characteristics, load characteristics, and cycle characteristics.

In this case, the amount of $LiBF_4$, $LiPO_2F_2$, $C_2H_5OSO_3Li$ or $FSO_3Li$ to be blended based on 100% by mass of the total electrolyte solution is not limited and may be any amount as long as the effects of the present disclosure are not significantly impaired. The amount thereof is usually 0.01% by mass or more, preferably 0.1% by mass or more, while usually 30% by mass or less, preferably 20% by mass or less, more preferably 10% by mass or less, even more preferably 5% by mass or less, with respect to the electrolyte solution of the present disclosure.

In another example, an inorganic lithium salt and an organic lithium salt are used in combination. Such a combination has an effect of suppressing deterioration due to high-temperature storage. Preferable examples of the organic lithium salt include $CF_3SO_3Li$, $LiN(FSO_2)_2$, $LiN(FSO_2)(CF_3SO_2)$, $LiN(CF_3SO_2)_2$, $LiN(C_2F_5SO_2)_2$, lithium cyclic 1,2-perfluoroethanedisulfonyl imide, lithium cyclic 1,3-perfluoropropanedisulfonyl imide, $LiC(FSO_2)_3$, $LiC(CF_3SO_2)_3$, $LiC(C_2F_5SO_2)_3$, $LiBF_3CF_3$, $LiBF_3C_2F_5$, $LiPF_3(CF_3)_3$, and $LiPF_3(C_2F_5)_3$. In this case, in view of electrochemical device performance, the proportion of the organic lithium salt is preferably 0.1% by mass or more, particularly preferably 0.5% by mass or more, while preferably 30% by mass or less, particularly preferably 20% by mass or less, based on 100% by mass of the total of the electrolyte solution.

The concentration of the electrolyte salt in the electrolyte solution is not limited as long as the effects of the present disclosure are not impaired. In order to make the electric conductivity of the electrolyte solution within a favorable range and ensure favorable battery performance, the electrolyte solution preferably contains lithium at a total molar concentration of 0.3 mol/L or more, more preferably 0.4 mol/L or more, even more preferably 0.5 mol/L or more, while preferably 3 mol/L or less, more preferably 2.5 mol/L or less, even more preferably 2.0 mol/L or less.

The electrolyte salt in the electrolyte solution for electric double layer capacitors is preferably an ammonium salt.

Examples of the ammonium salt include the following salts (IIa) to (IIe).

(IIa) Tetraalkyl quaternary ammonium salts

Preferable examples include tetraalkyl quaternary ammonium salts represented by general formula (IIa):

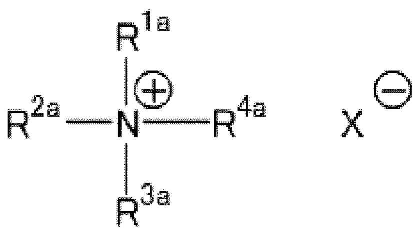

(IIa)

(wherein $R^{1a}$, $R^{2a}$, $R^{3a}$ and $R^{4a}$ are the same as or different from each other, and are each a $C_1$-$C_6$ alkyl group optionally containing an ether bond; and X- is an anion). Tetraalkyl quaternary ammonium salts wherein any or all of the hydrogen atoms in the ammonium salt are replaced with a fluorine atom and/or a $C_1$-$C_4$ fluorinated alkyl group are also preferable in view of enhanced oxidation resistance.

Specific examples of tetraalkyl quaternary ammonium salts include tetraalkyl quaternary ammonium salts represented by the following general formula (IIa-1):

$$(R^{1a})_x(R^{2a})_yN^{\oplus} \quad X^{\ominus} \qquad (IIa-1)$$

(wherein $R^{1a}$, $R^{2a}$ and X- are as defined above; x and y are the same as or different from each other, and are each an integer of 0 to 4, and x + y = 4), and alkyl ether group-containing trialkyl ammonium salts represented by the following general formula (IIa-2) :

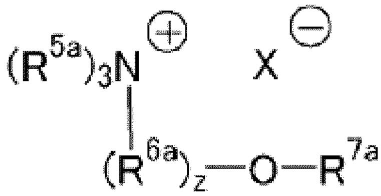

(IIa - 2)

(wherein $R^{5a}$ is a $C_1$-$C_6$ alkyl group; $R^{6a}$ is a $C_1$-$C_6$ divalent hydrocarbon group; $R^{7a}$ is a $C_1$-$C_4$ alkyl group; z is 1 or 2; and $X^-$ is an anion). The introduction of an alkyl ether group can reduce the viscosity.

The anion $X^-$ can be either an inorganic anion or an organic anion. Examples of the inorganic anion include $AlCl_4^-$, $BF_4^-$, $PF_6^-$, $AsF_6^-$, $TaF_6^-$, I-, and $SbF_6^-$. Examples of the organic anion include a bisoxalatoborate anion, a difulorooxalatoborate anion, a tetrafluorooxalatophosphate anion, a difluorobisoxalatophosphate anion, $CF_3COO^-$, $CF_3SO_3^-$, $(CF_3SO_2)_2N^-$, and $(C_2F_5SO_2)_2N^-$.

In view of high oxidation resistance and ionic dissociation, $BF_4^-$, $PF_6^-$, $AsF_6^-$, and $SbF_6^-$ are preferred.

Specific examples of preferred tetraalkyl quaternary ammonium salts include $Et_4NBF_4$, $Et_4NClO_4$, $Et_4NPF_6$, $Et_4NAsF_6$, $Et_4NSbF_6$, $Et_4NCF_3SO_3$, $Et_4N(CF_3SO_2)_2N$, $Et_4NC_4F_9SO_3$, $Et_3MeNBF_4$, $Et_3MeNClO_4$, $Et_3MeNPF_6$, Et3MeNAs$F_6$, $Et_3MeNSbF_6$, $Et_3MeNCF_3SO_3$, $Et_3MeN(CF_3SO_2)_2N$, and $Et_3MeNC_4F_9SO_3$, particularly preferably $Et_4NBF_4$, $Et_4NPF_6$, $Et_4NSbF_6$, $Et_4NAsF_6$, $Et_3MeNBF_4$, and an N,N-diethyl-N-methyl-N-(2-methoxyethyl)ammonium salt (wherein Et represents ethyl and Me represents methyl).

(IIb) Spirocyclic bipyrrolidinium salts

Preferred examples include spirocyclic bipyrrolidinium salts represented by the following general formula (IIb-1):

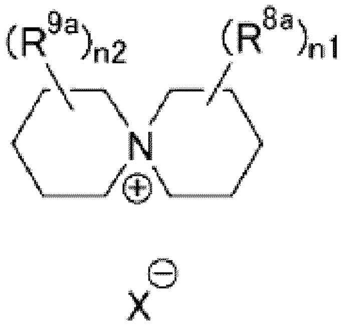

(IIb-1)

(wherein $R^{8a}$ and $R^{9a}$ are the same as or different from each other, and are each a $C_1$-$C_4$ alkyl group; X- is an anion; n1 is an integer of 0 to 5; and n2 is an integer of 0 to 5);
spirocyclic bipyrrolidinium salts represented by the following general formula (IIb-2):

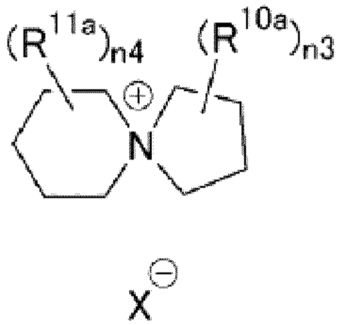

(IIb-2)

(wherein $R^{10a}$ and $R^{11a}$ are the same as or different from each other, and are each a $C_1$-$C_4$ alkyl group; X- is an anion; n3 is an integer of 0 to 5; and n4 is an integer of 0 to 5); and
spirocyclic bipyrrolidinium salts represented by the following general formula (IIb-3):

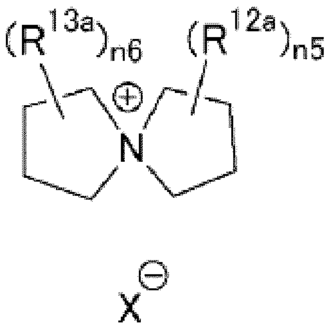

(IIb-3)

(wherein $R^{12a}$ and $R^{13a}$ are the same as or different from each other, and are each a $C_1$-$C_4$ alkyl group; X- is an anion; n5 is an integer of 0 to 5; and n6 is an integer of 0 to 5). Spirocyclic bipyrrolidinium salts wherein any or all of the hydrogen atoms are replaced with a fluorine atom and/or a $C_1$-$C_4$ fluorinated alkyl group are also preferable in view of enhanced oxidation resistance.

Specific examples of preferred anion $X^-$ are the same as those mentioned for the salts (IIa). The anion $X^-$ is preferably $BF_4$-, $PF_6$-, $(CF_3SO_2)_2N$- or $(C_2F_5SO_2)_2N$- in view of high dissociation and low internal resistance under high voltage.

Specific examples of preferred spirocyclic bipyrrolidinium salts include

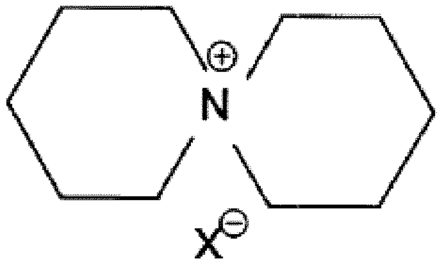
,

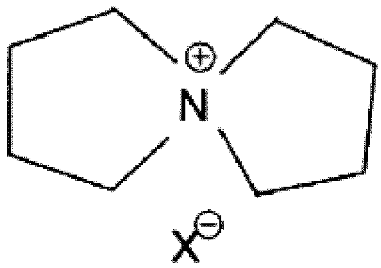
,

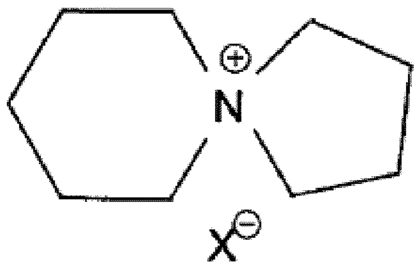

and the like.

These spirocyclic bipyrrolidinium salts are excellent in view of solubility in a solvent, oxidation resistance, and ion conductivity.

(IIc) Imidazolium salts

Preferred examples include imidazolium salts represented by the following general formula (IIc):

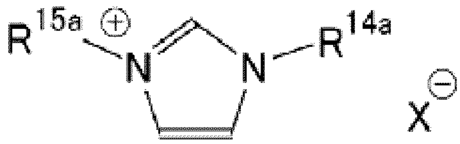
(IIc)

(wherein $R^{14a}$ and $R^{15a}$ are the same as or different from each other, and are each a $C_1$-$C_6$ alkyl group; and X- is an anion) . Such imidazolium salts wherein any or all of the hydrogen atoms are replaced with a fluorine atom and/or a $C_1$-$C_4$ fluorinated alkyl group are also preferred in view of enhanced oxidation resistance.

Specific examples of preferred anion $X^-$ are the same as those mentioned for the salts (IIa).

Specific examples of preferred anion X- include

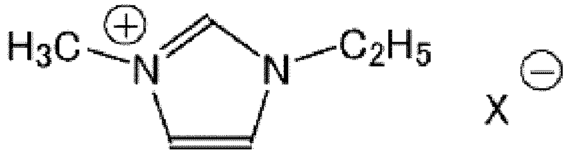

and the like.

This imidazolium salt is excellent in view of low viscosity and good solubility.

(IId) N-alkylpyridinium salts

Preferred examples include N-alkylpyridinium salts represented by the following general formula (IId):

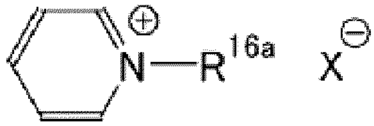

(IId)

(wherein $R^{16a}$ is a $C_1$-$C_6$ alkyl group; and $X^-$ is an anion) . Such N-alkylpyridinium salts wherein any or all of the hydrogen atoms in the N-alkylpyridinium salt are replaced with a fluorine atom and/or a $C_1$-$C_4$ fluorinated alkyl group are also preferred in view of enhanced oxidation resistance.

Specific examples of preferred anion $X^-$ are the same as those mentioned for the salts (IIa).

Specific examples of preferred N-alkylpyridinium salts include those represented by the following general formulas:

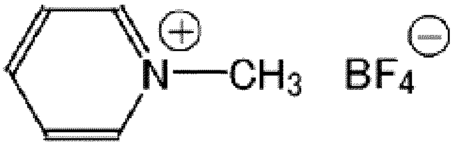

,

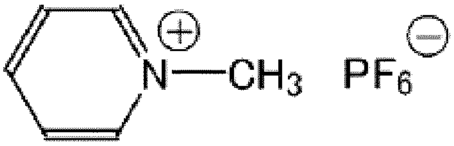

,

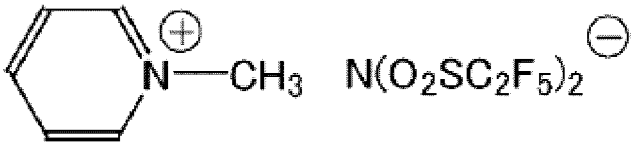

,

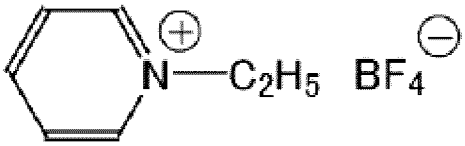

,

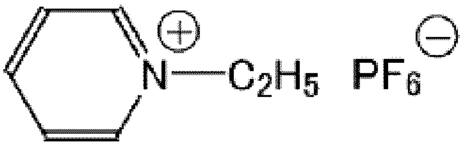

,

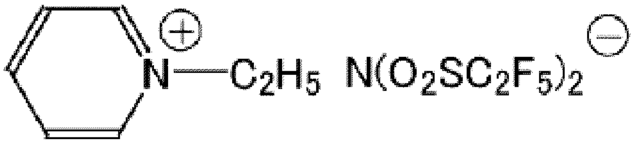

,

and the like.

Such N-alkylpyridinium salts are excellent in view of low viscosity and good solubility.

(IIe) N,N-dialkylpyrrolidinium salts

Preferred examples include N,N-dialkylpyrrolidinium salts represented by the following general formula (IIe):

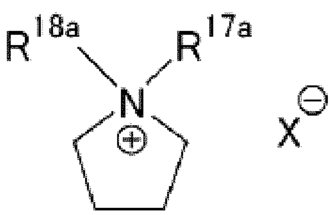

(IIe)

(wherein $R^{17a}$ and $R^{18a}$ are the same as or different from each other, and are each a $C_1$-$C_6$ alkyl group; and X- is an anion) . Such N,N-dialkylpyrrolidinium salts wherein any or all of the hydrogen atoms in the N,N-dialkylpyrrolidinium salt

are replaced with a fluorine atom and/or a $C_1$-$C_4$ fluorinated alkyl group are also preferred in view of enhanced oxidation resistance.

Specific examples of preferred anion $X^-$ are the same as those mentioned for the salts (IIa).

Specific examples of preferred N,N-dialkylpyrrolidinium salts include those represented by the following general formulas:

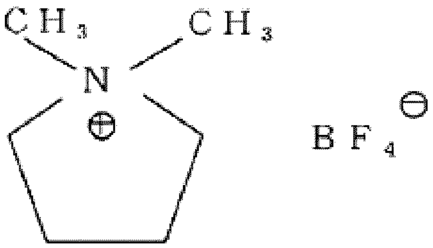
,
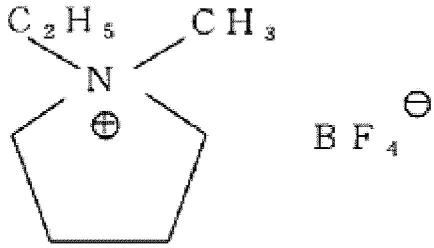
,
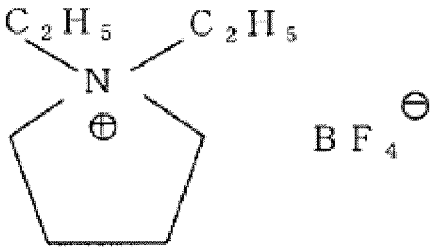
,
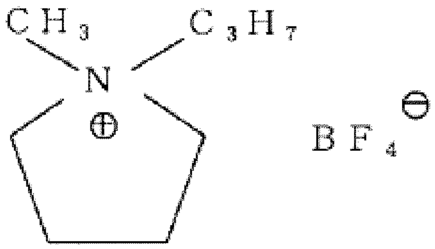
,
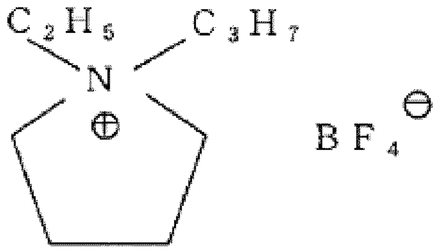
,
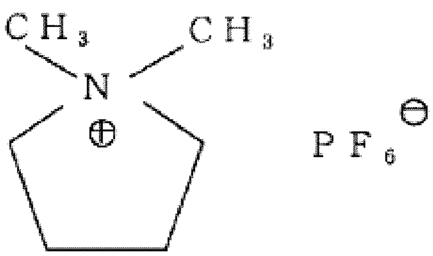
,
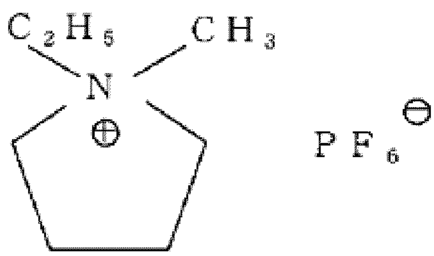
,
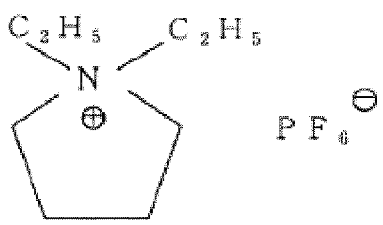
,
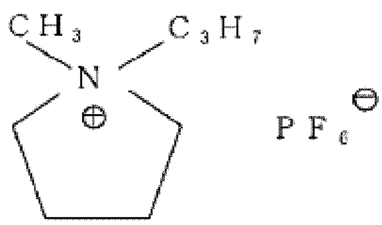
,

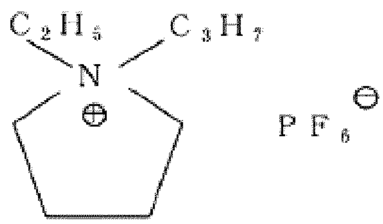
,

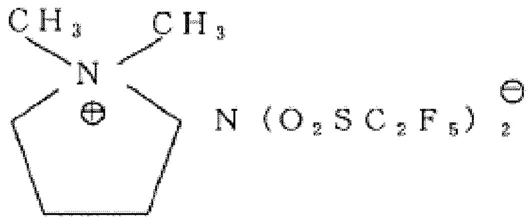
,

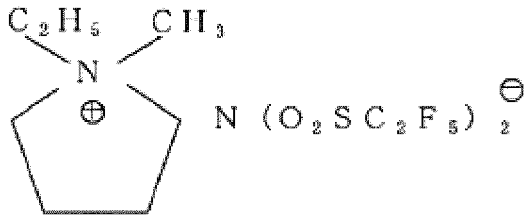
,

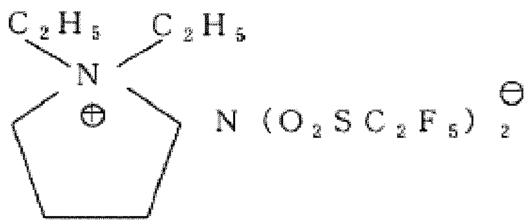
,

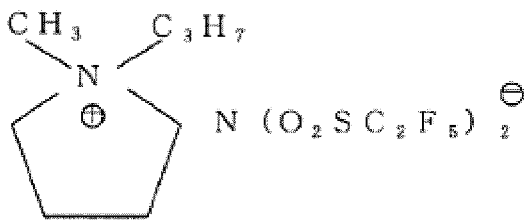
,

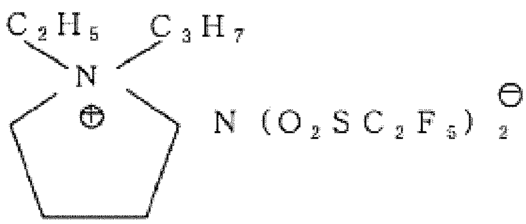

and the like.

These N,N-dialkylpyrrolidinium salts are excellent in view of low viscosity and good solubility.

In view of good solubility, oxidation resistance, and ion conductivity, ammonium salts represented by general formulas (IIa), (IIb), and (IIc) are preferred among these ammonium salts. More preferred are those represented by the following general formulas:

$(Me)_x(Et)_yN^{\oplus}X^{\ominus}$,

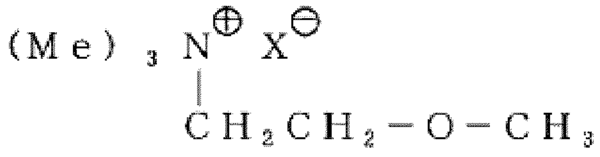

,

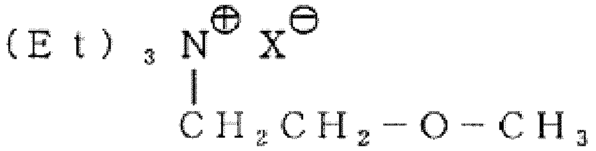

,

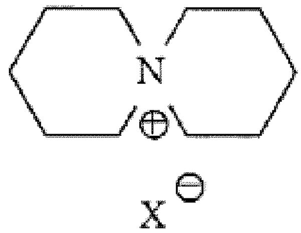

,

and

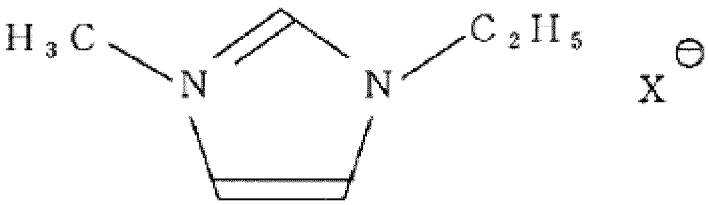

(wherein Me is a methyl group; Et is an ethyl group; and $X^-$, x, and y are as defined in formula (IIa-1)).

As an electrolyte salt for electric double layer capacitors, a lithium salt may be used. Preferred examples of the lithium salt include $LiPF_6$, $LiBF_4$, $LiN(FSO_2)_2$, $LiAsF_6$, $LiSbF_6$, and $LiN(SO_2C_2H_5)_2$.

A magnesium salt may be used in order to further increase the capacity. Preferred examples of the magnesium salt include $Mg(ClO_4)_2$ and $Mg(OOC_2H_5)_2$.

When the electrolyte salt is an ammonium salt, the concentration of the salt is preferably 0.7 mol/L or higher, more preferably 0.9 mol/L or higher, in view of, for example, low-temperature characteristics and high initial internal resistance.

The upper limit of the concentration is preferably 2.0 mol/L or lower, more preferably 1.5 mol/L or lower, in view of low-temperature characteristics.

When the ammonium salt is triethyl methyl ammonium tetrafluoroborate ($TEMABF_4$), the concentration is preferably 0.7 to 1.5 mol/L in view of excellent low-temperature characteristics.

When the ammonium salt is spirobipyrrolidinium tetrafluoroborate ($SBPBF_4$), the concentration is preferably 0.7 to 2.0 mol/L in view of electrochemical device performance.

The electrolyte solution of the present disclosure preferably further contains a compound (2) represented by the following general formula (2):

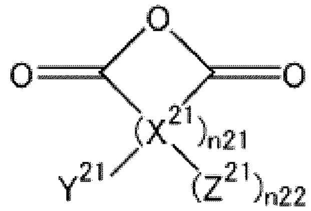

(wherein $X^{21}$ is a group containing at least H or C; n21 is an integer of 1 to 3; $Y^{21}$ and $Z^{21}$ are the same as or different from each other, and are each a group containing at least H, C, O, or F; n22 is 0 or 1; and $Y^{21}$ and $Z^{21}$ optionally bind to each other to form a ring). When the electrolyte solution contains the compound (2), the capacity retention is less likely to decrease and the amount of gas generated is less likely to increase, even when stored at high temperature.

When n21 is 2 or 3, the two or three $X^{21}$s may be the same as or different from each other.

When multiple $Y^{21}$s and $Z^{21}$s are present, the $Y^{21}$s may be the same as or different from each other, and the $Z^{21}$s may be the same as or different from each other.

In view of electrochemical device performance, $X^{21}$ is preferably a group represented by $-CY^{21}Z^{21}-$ (wherein $Y^{21}$ and $Z^{21}$ are as defined above) or $-CY^{21}=CZ^{21}-$ (wherein $Y^{21}$ and $Z^{21}$ are as defined above).

In view of electrochemical device performance, $Y^{21}$ is preferably at least one member selected from the group consisting of H-, F-, $CH_3$-, $CH_3CH_2$-, $CH_3CH_2CH_2$-, $CF_3$-, $CF_3CF_2$-, $CH_2FCH_2$-, and $CF_3CF_2CF_2$-.

In view of electrochemical device performance, $Z^{21}$ is preferably at least one member selected from the group consisting of H-, F-, $CH_3$-, $CH_3CH_2$-, $CH_3CH_2CH_2$-, $CF_3$-, $CF_3CF_2$-, $CH_2FCH_2$-, and $CF_3CF_2CF_2$-.

Alternatively, $Y^{21}$ and $Z^{21}$ may bind to each other to form a carbon ring or a heterocyclic ring that may contain an unsaturated bond and that may have aromaticity. The ring preferably contains 3 to 20 carbon atoms.

Next, specific examples of the compound (2) are described. In the following examples, the term "analog" refers to an acid anhydride obtainable by replacing part of the structure of an acid anhydride mentioned as an example with another structure within the scope of the present disclosure. Examples include dimers, trimers, and tetramers, each composed of a plurality of acid anhydrides, those having substituents that are isostructural isomers having the same number of carbon atoms but having different branch structures, and those having the same substituent(s) but at different sites in an acid anhydride.

Specific examples of acid anhydrides having a 5-membered cyclic structure include succinic anhydride, methylsuccinic anhydride (4-methylsuccinic anhydride), dimethylsuccinic anhydride (e.g., 4,4-dimethylsuccinic anhydride, 4,5-dimethylsuccinic anhydride), 4,4,5-trimethylsuccinic anhydride, 4,4,5,5-tetramethylsuccinic anhydride, 4-vinylsuccinic anhydride, 4,5-divinylsuccinic anhydride, phenylsuccinic anhydride (4-phenylsuccinic anhydride), 4,5-diphenylsuccinic anhydride, 4,4-diphenylsuccinic anhydride, citraconic anhydride, maleic anhydride, methylmaleic anhydride (4-methylmaleic anhydride), 4,5-dimethylmaleic anhydride, phenylmaleic anhydride (4-phenylmaleic anhydride), 4,5-diphenylmaleic anhydride, itaconic anhydride, 5-methylitaconic anhydride, 5,5-dimethylitaconic anhydride, phthalic anhydride, and 3,4,5,6-tetrahydrophthalic anhydride, and analogs thereof.

Specific examples of acid anhydrides having a 6-membered cyclic structure include cyclohexanedicarboxylic anhydride (e.g., cyclohexane-1,2-dicarboxylic anhydride), 4-cyclohexene-1,2-dicarboxylic anhydride, glutaric anhydride, glutaconic anhydride, and 2-phenylglutaric anhydride, and analogs thereof.

Specific examples of acid anhydrides having a different cyclic structure include 5-norbornene-2,3-dicarboxylic anhydride, cyclopentanetetracarboxylic dianhydride, pyromellitic anhydride, and diglycolic anhydride, and analogs thereof.

Specific examples of acid anhydrides having a cyclic structure and substituted with one or more halogen atoms include monofluorosuccinic anhydride (e.g., 4-fluorosuccinic anhydride), 4,4-difluorosuccinic anhydride, 4,5-difluorosuccinic anhydride, 4,4,5-trifluorosuccinic anhydride, trifluoromethylsuccinic anhydride, tetrafluorosuccinic anhydride (4,4,5,5-tetrafluorosuccinic anhydride), 4-fluoromaleic anhydride, 4,5-difluoromaleic anhydride, trifluoromethylmaleic anhydride, 5-fluoroitaconic anhydride, and 5,5-difluoroitaconic anhydride, and analogs thereof.

In view of electrochemical device performance, preferred examples of the compound (2) among these are glutaric anhydride, citraconic anhydride, glutaconic anhydride, itaconic anhydride, diglycolic anhydride, cyclohexanedicarboxylic anhydride, cyclopentanetetracarboxylic dianhydride, 4-cyclohexene-1,2-dicarboxylic anhydride, 3,4,5,6-tetrahydrophthalic anhydride, 5-norbornene-2,3-dicarboxylic anhydride, phenylsuccinic anhydride, 2-phenylglutaric anhydride, maleic anhydride, methylmaleic anhydride, trifluoromethylmaleic anhydride, phenylmaleic anhydride, succinic anhydride, methylsuccinic anhydride, dimethylsuccinic anhydride, trifluoromethylsuccinic anhydride, monofluorosuccinic anhydride, and tetrafluorosuccinic anhydride; more preferably maleic anhydride, methylmaleic anhydride, trifluoromethylmaleic anhydride, succinic anhydride, methylsuccinic anhydride, trifluoromethylsuccinic anhydride, and tetrafluorosuccinic anhydride; and still more preferably maleic anhydride and succinic anhydride.

In view of electrochemical device performance, the compound (2) is preferably at least one member selected

from the group consisting of: a compound (3) represented by the following general formula (3):

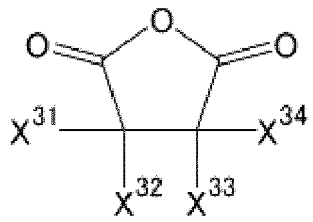

(wherein $X^{31}$ to $X^{34}$ are the same as or different from each other, and are each a group containing at least H, C, O, or F); and a compound (4) represented by the following general formula (4):

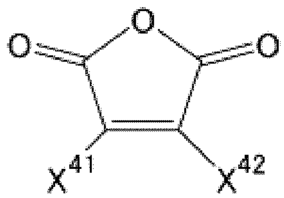

(wherein $X^{41}$ and $X^{42}$ are the same as or different from each other, and are each a group containing at least H, C, O, or F).

$X^{31}$ to $X^{34}$ are the same as or different from each other, and are preferably at least one member selected from the group consisting of alkyl groups, fluorinated alkyl groups, alkenyl groups, and fluorinated alkenyl groups. $X^{31}$ to $X^{34}$ each preferably have 1 to 10 carbon atoms, more preferably 1 to 3 carbon atoms.

$X^{31}$ to $X^{34}$ are the same as or different from each other, and are more preferably at least one member selected from the group consisting of H-, F-, $CH_3$-, $CH_3CH_2$-, $CH_3CH_2CH_2$-, $CF_3$-, $CF_3CF_2$-, $CH_2FCH_2$-, and $CF_3CF_2CF_2$-.

$X^{41}$ and $X^{42}$ are the same as or different from each other, and are preferably at least one member selected from the group consisting of alkyl groups, fluorinated alkyl groups, alkenyl groups, and fluorinated alkenyl groups. $X^{41}$ and $X^{42}$ each preferably have 1 to 10 carbon atoms, more preferably 1 to 3 carbon atoms.

$X^{41}$ and $X^{42}$ are the same as or different from each other, and are preferably at least one member selected from the group consisting of H-, F-, $CH_3$-, $CH_3CH_2$-, $CH_3CH_2CH_2$-, $CF_3$-, $CF_3CF_2$-, $CH_2FCH_2$-, and $CF_3CF_2CF_2$-.

The compound (3) is preferably any of the following compounds.

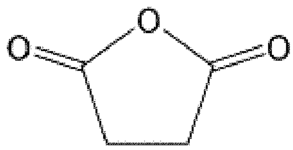

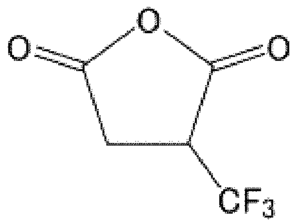

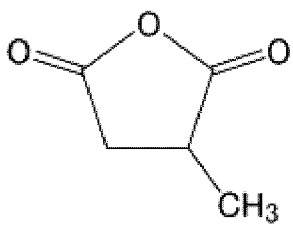

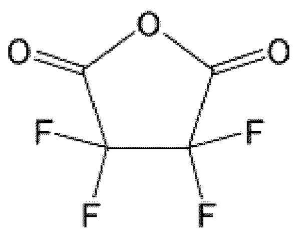

The compound (4) is preferably any of the following compounds.

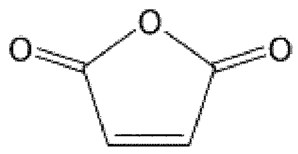

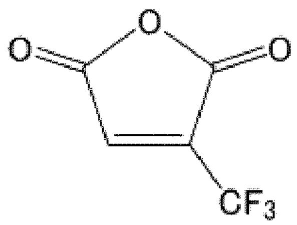

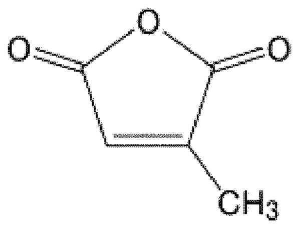

In order for the capacity retention to be less likely to decrease and for the amount of gas generated to be less likely to increase, even when stored at high temperature, the electrolyte solution preferably contains 0.0001 to 15% by mass of the compound (2) relative to the electrolyte solution. The amount of the compound (2) is more preferably 0.01 to 10% by mass, still more preferably 0.1 to 3% by mass, particularly preferably 0.1 to 1.0% by mass.

In order for the capacity retention to be less likely to decrease and for the amount of gas generated to be less likely to increase, even when stored at high temperature, the electrolyte solution, when containing both the compounds (3) and (4), preferably contains 0.08 to 2.50% by mass of the compound (3) and 0.02 to 1.50% by mass of the compound (4), more preferably 0.80 to 2.50% by mass of the compound (3) and 0.08 to 1.50% by mass of the compound (4), relative to the electrolyte solution.

The electrolyte solution of the present disclosure may contain at least one member selected from the group consisting of nitrile compounds represented by the following general formulas (1a), (1b), and (1c):

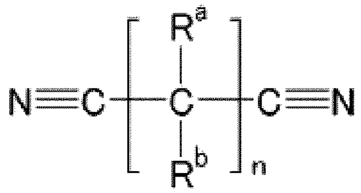 (1a)

(wherein $R^a$ and $R^b$ are each independently a hydrogen atom, a cyano group (CN), a halogen atom, an alkyl group, or a group obtainable by replacing at least one hydrogen atom of an alkyl group with a halogen atom; and n is an integer of 1 to 10);

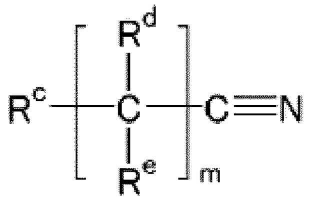 (1b)

(wherein $R^c$ is a hydrogen atom, a halogen atom, an alkyl group, a group obtainable by replacing at least one hydrogen atom of an alkyl group with a halogen atom, or a group represented by $NC-R^{c1}-X^{c1}-$ (wherein $R^{c1}$ is an alkylene group, $X^{c1}$ is an oxygen atom or a sulfur atom); $R^d$ and $R^e$ are each independently a hydrogen atom, a halogen atom, an alkyl group, or a group obtainable by replacing at least one hydrogen atom of an alkyl group with a halogen atom; and m is an integer of 1 to 10);

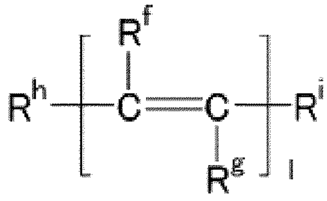

(1c)

(wherein $R^f$, $R^g$, $R^h$, and $R^i$ are each independently a group containing a cyano group (CN), a hydrogen atom (H), a halogen atom, an alkyl group, or a group obtainable by replacing at least one hydrogen atom of an alkyl group with a halogen atom; provided that at least one member selected from the group consisting of $R^f$, $R^g$, $R^h$, and $R^i$ is a group containing a cyano group; and 1 is an integer of 1 to 3). This can improve the high-temperature storage characteristics of an electrochemical device. Such nitrile compounds may be used alone, or two or more thereof may be used in any combination at any ratio.

In general formula (1a), $R^a$ and $R^b$ are each independently a hydrogen atom, a cyano group (CN), a halogen atom, an alkyl group, or a group obtainable by replacing at least one hydrogen atom of an alkyl group with a halogen atom.

Examples of the halogen atom include a fluorine atom, a chlorine atom, a bromine atom, and an iodine atom. Preferred among these is a fluorine atom.

The alkyl group is preferably a $C_1$-$C_5$ alkyl group. Specific examples of the alkyl group include a methyl group, an ethyl group, a propyl group, an isopropyl group, a butyl group, an isobutyl group, and a tert-butyl group.

An example of the group obtainable by replacing at least one hydrogen atom of an alkyl group with a halogen atom is a group obtainable by replacing at least one hydrogen atom of the aforementioned alkyl group with at least one of the halogen atoms described above.

When $R^a$ and $R^b$ are alkyl groups or groups obtainable by replacing at least one hydrogen atom of an alkyl group with a halogen atom, $R^a$ and $R^b$ may bind to each other to form a cyclic structure (e.g., a cyclohexane ring).

$R^a$ and $R^b$ are each preferably a hydrogen atom or an alkyl group.

In general formula (1a), n is an integer of 1 to 10. When n is 2 or greater, all of n $R^a$s may be the same, or at least one of them may be different from the others. The same applies to $R^b$. In the general formula, n is preferably an integer of 1 to 7, more preferably an integer of 2 to 5.

Preferred as the nitrile compound represented by general formula (1a) are dinitriles and tricarbonitriles.

Specific examples of dinitriles include malononitrile, succinonitrile, glutaronitrile, adiponitrile, pimelonitrile, suberonitrile, azelanitrile, sebaconitrile, undecanedinitrile, dodecanedinitrile, methylmalononitrile, ethylmalononitrile, isopropylmalononitrile, tert-butylmalononitrile, methylsuccinonitrile, 2,2-dimethylsuccinonitrile, 2,3-dimethylsuccinonitrile, 2,3,3-trimethylsuccinonitrile, 2,2,3,3-tetramethylsuccinonitrile, 2,3-diethyl-2,3-dimethylsuccinonitrile, 2,2-diethyl-3,3-dimethylsuccinonitrile, bicyclohexyl-1,1-dicarbonitrile, bicyclohexyl-2,2-dicarbonitrile, bicyclohexyl-3,3-dicarbonitrile, 2,5-dimethyl-2,5-hexanedicarbonitrile, 2,3-diisobutyl-2,3-dimethylsuccinonitrile, 2,2-diisobutyl-3,3-dimethylsuccinonitrile, 2-methylglutaronitrile, 2,3-dimethylglutaronitrile, 2,4-dimethylglutaronitrile, 2,2,3,3-tetramethylglutaronitrile, 2,2,4,4-tetramethylglutaronitrile, 2,2,3,4-tetramethylglutaronitrile, 2,3,3,4-tetramethylglutaronitrile, 1,4-dicyanopentane, 2,6-dicyanoheptane, 2,7-dicyanooctane, 2,8-dicyanononane, 1,6-dicyanodecane, 1,2-dicyanobenzene, 1,3-dicyanobenzene, 1,4-dicyanobenzene, 3,3'-(ethylenedioxy)dipropionitrile, 3,3'-(ethylenedithio)dipropionitrile, 3,9-bis(2-cyanoethyl)-2,4,8,10-tetraoxaspiro[5,5]undecane, butanenitrile, and phthalonitrile. Particularly preferred among these are succinonitrile, glutaronitrile, and adiponitrile.

Specific examples of the tricarbonitriles include pentanetricarbonitrile, propanetricarbonitrile, 1,3,5-hexanetricarbonitrile, 1,3,6-hexanetricarbonitrile, heptanetricarbonitrile, 1,2,3-propanetricarbonitrile, 1,3,5-pentanetricarbonitrile, cyclohexanetricarbonitrile, triscyanoethylamine, triscyanoethoxypropane, tricyanoethylene, and tris(2-cyanoethyl)amine. Particularly preferred are 1,3,6-hexanetricarbonitrile and cyclohexanetricarbonitrile. Most preferred is cyclohexanetricarbonitrile.

In general formula (1b), $R^c$ is a hydrogen atom, a halogen atom, an alkyl group, a group obtainable by replacing at least one hydrogen atom of an alkyl group with halogen atom, or a group represented by NC-$R^{c1}$-$X^{c1}$- (wherein $R^{c1}$ is an alkylene group; and $X^{c1}$ is an oxygen atom or a sulfur atom) ; and $R^d$ and $R^e$ are each independently a hydrogen atom, a halogen atom, an alkyl group, or a group obtainable by replacing at least one hydrogen atom of an alkyl group with a halogen atom.

Examples of the halogen atom, the alkyl group, and the group obtainable by replacing at least one hydrogen atom of an alkyl group with a halogen atom include those mentioned as examples thereof for general formula (1a).

$R^{c1}$ in NC-$R^{c1}$-$X^{c1}$- is an alkylene group. The alkylene group is preferably a $C_1$-$C_3$ alkylene group.

$R^c$, $R^d$, and $R^e$ are each preferably independently a hydrogen atom, a halogen atom, an alkyl group, or a group obtainable by replacing at least one hydrogen atom of an alkyl group with a halogen atom.

At least one selected from $R^c$, $R^d$, and $R^e$ is preferably a halogen atom or a group obtainable by replacing at least one hydrogen atom of an alkyl group with a halogen atom, and more preferably a fluorine atom or a group obtainable by replacing at least one hydrogen atom of an alkyl group with a fluorine atom.

When $R^d$ and $R^e$ are each an alkyl group or a group obtainable by replacing at least one hydrogen atom of an alkyl group with a halogen atom, $R^d$ and $R^e$ may bind to each other to form a cyclic structure (e.g., a cyclohexane ring).

In general formula (1b), m is an integer of 1 to 10. When m is 2 or greater, all of m $R^d$s may be the same as each other, or at least some of them may be different from the others. The same applies to $R^e$. In the general formula, m is preferably an integer of 2 to 7, and more preferably an integer of 2 to 5.

Examples of the nitrile compound represented by general formula (1b) include acetonitrile, propionitrile, butyronitrile, isobutyronitrile, valeronitrile, isovaleronitrile, lauronitrile, 3-methoxypropionitrile, 2-methylbutyronitrile, trimethylacetonitrile, hexanenitrile, cyclopentanecarbonitrile, cyclohexanecarbonitrile, fluoroacetonitrile, difluoroacetonitrile, trifluoroacetonitrile, 2-fluoropropionitrile, 3-fluoropropionitrile, 2,2-difluoropropionitrile, 2,3-difluoropropionitrile, 3,3-difluoropropionitrile, 2,2,3-trifluoropropionitrile, 3,3,3-trifluoropropionitrile, 3,3'-oxydipropionitrile, 3,3'-thiodipropionitrile, pentafluoropropionitrile, methoxyacetonitrile, and benzonitrile. Particularly preferred among these is 3,3,3-trifluoropropionitrile.

In general formula (1c), $R^f$, $R^g$, $R^h$, and $R^i$ are each independently a group containing a cyano group (CN), a hydrogen atom, a halogen atom, an alkyl group, or a group obtainable by replacing at least one hydrogen atom of an alkyl group with a halogen atom.

Examples of the halogen atom, the alkyl group, and the group obtainable by replacing at least one hydrogen atom of an alkyl group with a halogen atom include those mentioned as examples thereof for general formula (1a).

Examples of the group containing a cyano group include a cyano group and a group obtainable by replacing at least one hydrogen atom of an alkyl group with a cyano group. Examples of the alkyl group in this case include those mentioned as examples for general formula (1a).

At least one selected from $R^f$, $R^g$, $R^h$, and $R^i$ is a group containing a cyano group. Preferably, at least two selected from $R^f$, $R^g$, $R^h$, and $R^i$ are each a group containing a cyano group. More preferably, $R^h$ and $R^i$ are each a group containing a cyano group. When $R^h$ and $R^i$ are each a group containing a cyano group, $R^f$ and $R^g$ are each preferably a hydrogen atom.

In general formula (1c), 1 is an integer of 1 to 3. When l is 2 or greater, all of l $R^f$s may be the same or at least one of them may be different from the others. The same applies to $R^g$. In the general formula, 1 is preferably an integer of 1 or 2.

Examples of the nitrile compound represented by general formula (1c) include 3-hexenedinitrile, mucononitrile, maleonitrile, fumaronitrile, acrylonitrile, methacrylonitrile, crotononitrile, 3-methylcrotononitrile, 2-methyl-2-butenenitrile, 2-pentenenitrile, 2-methyl-2-pentenenitrile, 3-methyl-2-pentenenitrile, and 2-hexenenitrile. Preferred are 3-hexenedinitrile and mucononitrile. Particularly preferred is 3-hexenedinitrile.

The nitrile compound content is preferably 0.2 to 7% by mass relative to the electrolyte solution. This can further improve the high-temperature storage characteristics and safety of an electrochemical device at high voltage. The lower limit of the total amount of the nitrile compounds is more preferably 0.3% by mass, still more preferably 0.5% by mass. The upper limit thereof is more preferably 5% by mass, still more preferably 2% by mass, particularly preferably 0.5% by mass.

The electrolyte solution of the present disclosure may contain a compound having an isocyanate group (which may be simply referred to below as "isocyanate"). The isocyanate may be any isocyanate. Examples of the isocyanate include monoisocyanates, diisocyanates, and triisocyanates.

Specific examples of the monoisocyanate include isocyanatomethane, isocyanatoethane, 1-isocyanatopropane, 1-isocyanatobutane, 1-isocyanatopentane, 1-isocyanatohexane, 1-isocyanatoheptane, 1-isocyanatooctane, 1-isocyanatononane, 1-isocyanatodecane, isocyanatocyclohexane, methoxycarbonyl isocyanate, ethoxycarbonyl isocyanate, propoxycarbonyl isocyanate, butoxycarbonyl isocyanate, methoxysulfonyl isocyanate, ethoxysulfonyl isocyanate, propoxysulfonyl isocyanate, butoxysulfonyl isocyanate, fluorosulfonyl isocyanate, methyl isocyanate, butyl isocyanate, phenyl isocyanate, 2-isocyanatoethyl acrylate, 2-isocyanatoethyl methacrylate, and ethyl isocyanate.

Specific examples of the diisocyanates include 1,4-diisocyanatobutane, 1,5-diisocyanatopentane, 1,6-diisocyanatohexane, 1,7-diisocyanatoheptane, 1,8-diisocyanatooctane, 1,9-diisocyanatononane, 1,10-diisocyanatodecane, 1,3-diisocyanatopropene, 1,4-diisocyanato-2-butene, 1,4-diisocyanato-2-fluorobutane, 1,4-diisocyanato-2,3-difluorobutane, 1,5-diisocyanato-2-pentene, 1,5-diisocyanato-2-methylpentane, 1,6-diisocyanato-2-hexene, 1,6-diisocyanato-3-hexene, 1,6-diisocyanato-3-fluorohexane, 1,6-diisocyanato-3,4-difluorohexane, toluene diisocyanate, xylene diisocyanate, tolylene diisocyanate, 1,2-bis(isocyanatomethyl)cyclohexane, 1,3-bis(isocyanatomethyl)cyclohexane, 1,4-bis(isocyanatomethyl)cyclohexane, 1,2-diisocyanatocyclohexane, 1,3-diisocyanatocyclohexane, 1,4-diisocyanatocyclohexane, dicyclohexylmethane-1,1'-diisocyanate, dicyclohexylmethane-2,2'-diisocyanate, dicyclohexylmethane-3,3'-diisocyanate, dicyclohexylmethane-4,4'-diisocyanate, isophorone diisocyanate, bicyclo[2.2.1]heptane-2,5-diylbis(methyl=isocyanate), bicyclo[2.2.1]heptane-2,6-diylbis(methyl=isocyanate), 2,4,4-trimethylhexamethylene diisocyanate,

2,2,4-trimethylhexamethylene diisocyanate, hexamethylene diisocyanate, 1,4-phenylene diisocyanate, octamethylene diisocyanate, and tetramethylene diisocyanate.

Specific examples of the triisocyanates include 1,6,11-triisocyanatoundecane, 4-isocyanatomethyl-1,8-octamethylene diisocyanate, 1,3,5-triisocyanatomethylbenzene, 1,3,5-tris(6-isocyanatohex-1-yl)-1,3,5-triazine-2,4,6(1H,3H,5H)-trione, and 4-(isocyanatomethyl)octamethylene=diisocyanate.

Among these, 1,6-diisocyanatohexane, 1,3-bis(isocyanatomethyl)cyclohexane, 1,3,5-tris(6-isocyanatohex-1-yl)-1,3,5-triazine-2,4,6(1H,3H,5H)-trione, 2,4,4-trimethylhexamethylene diisocyanate, and 2,2,4-trimethylhexamethylene diisocyanate are preferred in view of ease of availability on an industrial scale and cost reduction in the production of the electrolyte solution. From a technical standpoint as well, these compounds, which can contribute to the formation of a stable film-shaped structure, are more preferably used.

The isocyanate content is not limited as long as the effects of the present disclosure are not significantly impaired. The amount is preferably 0.001% by mass or more and 1.0% by mass or less, relative to the electrolyte solution. When the isocyanate content is equal to or higher than this lower limit, a sufficient effect of improving the cycle characteristics can be provided to a non-aqueous electrolyte secondary battery. When the isocyanate content is equal to or lower than this upper limit, an initial increase in the resistance of a non-aqueous electrolyte secondary battery can be avoided. The isocyanate content is more preferably 0.01% by mass or more, still more preferably 0.1% by mass or more, particularly preferably 0.2% by mass or more, while more preferably 0.8% by mass or less, still more preferably 0.7% by mass or less, particularly preferably 0.6% by mass or less.

The electrolyte solution of the present disclosure may contain a cyclic sulfonic acid ester. The cyclic sulfonic acid ester may be any cyclic sulfonic acid ester. Examples of the cyclic sulfonic acid ester include a saturated cyclic sulfonic acid ester, an unsaturated cyclic sulfonic acid ester, a saturated cyclic disulfonic acid ester, and an unsaturated cyclic disulfonic acid ester.

Specific examples of saturated cyclic sulfonic acid esters include 1,3-propanesultone, 1-fluoro-1,3-propanesultone, 2-fluoro-1,3-propanesultone, 3-fluoro-1,3-propanesultone, 1-methyl-1,3-propanesultone, 2-methyl-1,3-propanesultone, 3-methyl-1,3-propanesultone, 1,3-butanesultone, 1,4-butanesultone, 1-fluoro-1,4-butanesultone, 2-fluoro-1,4-butanesultone, 3-fluoro-1,4-butanesultone, 4-fluoro-1,4-butanesultone, 1-methyl-1,4-butanesultone, 2-methyl-1,4-butanesultone, 3-methyl-1,4-butanesultone, 4-methyl-1,4-butanesultone, and 2,4-butanesultone.

Specific examples of unsaturated cyclic sulfonic acid esters include 1-propene-1,3-sultone, 2-propene-1,3-sultone, 1-fluoro-1-propene-1,3-sultone, 2-fluoro-1-propene-1,3-sultone, 3-fluoro-1-propene-1,3-sultone, 1-fluoro-2-propene-1,3-sultone, 2-fluoro-2-propene-1,3-sultone, 3-fluoro-2-propene-1,3-sultone, 1-methyl-1-propene-1,3-sultone, 2-methyl-1-propene-1,3-sultone, 3-methyl-1-propene-1,3-sultone, 1-methyl-2-propene-1,3-sultone, 2-methyl-2-propene-1,3-sultone, 3-methyl-2-propene-1,3-sultone, 1-butene-1,4-sultone, 2-butene-1,4-sultone, 3-butene-1,4-sultone, 1-fluoro-1-butene-1,4-sultone, 2-fluoro-1-butene-1,4-sultone, 3-fluoro-1-butene-1,4-sultone, 4-fluoro-1-butene-1,4-sultone, 1-fluoro-2-butene-1,4-sultone, 2-fluoro-2-butene-1,4-sultone, 3-fluoro-2-butene-1,4-sultone, 4-fluoro-2-butene-1,4-sultone, 1,3-propenesultone, 1-fluoro-3-butene-1,4-sultone, 2-fluoro-3-butene-1,4-sultone, 3-fluoro-3-butene-1,4-sultone, 4-fluoro-3-butene-1,4-sultone, 1-methyl-1-butene-1,4-sultone, 2-methyl-1-butene-1,4-sultone, 3-methyl-1-butene-1,4-sultone, 4-methyl-1-butene-1,4-sultone, 1-methyl-2-butene-1,4-sultone, 2-methyl-2-butene-1,4-sultone, 3-methyl-2-butene-1,4-sultone, 4-methyl-2-butene-1,4-sultone, 1-methyl-3-butene-1,4-sultone, 2-methyl-3-butene-1,4-sultone, 3-methyl-3-butene-1,4-sultone, and 4-methyl-3-butene-14-sultone.

In view of easy availability and contribution to the formation of a stable film-shaped structure, more preferred among these are 1,3-propanesultone, 1-fluoro-1,3-propanesultone, 2-fluoro-1,3-propanesultone, 3-fluoro-1,3-propanesultone, and 1-propene-1,3-sultone. The cyclic sulfonic acid ester content may be any amount that does not significantly impair the effects of the present disclosure. The cyclic sulfonic acid ester content is preferably 0.001% by mass or more and 3.0% by mass or less, relative to the electrolyte solution.

When the cyclic sulfonic acid ester content is equal to or more than this lower limit, a sufficient effect of improving the cycle characteristics can be provided to a non-aqueous electrolyte secondary battery. When the cyclic sulfonic acid ester content is equal to or less than this upper limit, an increase in the cost of producing a non-aqueous electrolyte secondary battery can be avoided. The cyclic sulfonic acid ester content is more preferably 0.01% by mass or more, still more preferably 0.1% by mass or more, particularly preferably 0.2% by mass or more, while more preferably 2.5% by mass or less, still more preferably 2.0% by mass or less, particularly preferably 1.8% by mass or less.

The electrolyte solution of the present disclosure may further contain a polyethylene oxide that has a weight average molecular weight of 2000 to 4000, and that has -OH, - OCOOH, or -COOH at an end.

The presence of such a compound in the electrolyte solution can enhance the stability at the interfaces with the electrodes, thus improving the characteristics of an electrochemical device.

Examples of the polyethylene oxide include polyethylene oxide monool, polyethylene oxide carboxylic acid, polyethylene oxide diol, polyethylene oxide dicarboxylic acid, polyethylene oxide triol, and polyethylene oxide tricarboxylate. Such polyethylene oxides may be used alone or two or more thereof may be used in any combination.

In view of better characteristics of an electrochemical device, preferred are a mixture of polyethylene oxide

monool and polyethylene oxide diol and a mixture of polyethylene carboxylic acid and polyethylene dicarboxylic acid.

The polyethylene oxide having an overly low weight average molecular weight may be easily oxidatively decomposed. The weight average molecular weight is more preferably 3000 to 4000.

The weight average molecular weight can be determined by gel permeation chromatography (GPC) in terms of polystyrene.

In view of electrochemical device performance, the polyethylene oxide content is preferably $1 \times 10^{-6}$ to $1 \times 10^{-2}$ mol/kg in the electrolyte solution.

The polyethylene oxide content is more preferably $5 \times 10^{-6}$ mol/kg or more.

The electrolyte solution of the present disclosure may further contain as an additive any of other components such as a fluorinated saturated cyclic carbonate, an unsaturated cyclic carbonate, an overcharge inhibitor, and other known aids. This can reduce impairment of the characteristics of an electrochemical device.

Examples of the fluorinated saturated cyclic carbonate include compounds represented by general formula (A) described above. Preferred among these are fluoroethylene carbonate, difluoroethylene carbonate, monofluoromethyl ethylene carbonate, trifluoromethyl ethylene carbonate, 2,2,3,3,3-pentafluoropropylethylene carbonate (4-(2,2,3,3,3-pentafluoropropyl)-[1,3]dioxolan-2-one). One fluorinated saturated cyclic carbonate may be used alone, or two or more thereof may be used in any combination at any ratio.

In view of electrochemical device performance, the fluorinated saturated cyclic carbonate content is preferably 0.001 to 10% by mass, more preferably 0.01 to 5% by mass, and even more preferably 0.1 to 3% by mass, relative to the electrolyte solution.

Examples of the unsaturated cyclic carbonate include vinylene carbonates; ethylene carbonates substituted with a substituent containing an aromatic ring, a carbon-carbon double bond, or a carbon-carbon triple bond; phenyl carbonates; vinyl carbonates; allyl carbonates; and catechol carbonates.

Examples of vinylene carbonates include vinylene carbonate, methylvinylene carbonate, 4,5-dimethylvinylene carbonate, phenylvinylene carbonate, 4,5-diphenylvinylene carbonate, vinylvinylene carbonate, 4,5-divinylvinylene carbonate, allylvinylene carbonate, 4,5-diallylvinylene carbonate, 4-fluorovinylene carbonate, 4-fluoro-5-methylvinylene carbonate, 4-fluoro-5-phenylvinylene carbonate, 4-fluoro-5-vinylvinylene carbonate, 4-allyl-5-fluorovinylene carbonate, ethynylethylene carbonate, propargylethylene carbonate, methylvinylene carbonate, and dimethylvinylene carbonate.

Specific examples of ethylene carbonates substituted with a substituent containing an aromatic ring, a carbon-carbon double bond, or a carbon-carbon triple bond include vinylethylene carbonate, 4,5-divinylethylene carbonate, 4-methyl-5-vinylethylene carbonate, 4-allyl-5-vinylethylene carbonate, ethynylethylene carbonate, 4,5-diethynylethylene carbonate, 4-methyl-5-ethynylethylene carbonate, 4-vinyl-5-ethynylethylene carbonate, 4-allyl-5-ethynylethylene carbonate, phenylethylene carbonate, 4,5-diphenylethylene carbonate, 4-phenyl-5-vinylethylene carbonate, 4-allyl-5-phenylethylene carbonate, allylethylene carbonate, 4,5-diallylethylene carbonate, 4-methyl-5-allylethylene carbonate, 4-methylene-1,3-dioxolan-2-one, 4,5-di methylene-1,3-dioxolan-2-one, and 4-methyl-5-allylethylene carbonate.

Among them, the unsaturated cyclic carbonate is preferably vinylene carbonate, methylvinylene carbonate, 4,5-dimethylvinylene carbonate, vinylvinylene carbonate, 4,5-vinylvinylene carbonate, allylvinylene carbonate, 4,5-diallylvinylene carbonate, vinylethylene carbonate, 4,5-divinylethylene carbonate, 4-methyl-5-vinylethylene carbonate, allylethylene carbonate, 4,5-diallylethylene carbonate, 4-methyl-5-allylethylene carbonate, 4-allyl-5-vinylethylene carbonate, ethynylethylene carbonate, 4,5-diethynylethylene carbonate, 4-methyl-5-ethynylethylene carbonate, or 4-vinyl-5-ethynylethylene carbonate. In view of forming a more stable interface protecting film, vinylene carbonate, vinylethylene carbonate, and ethynylethylene carbonate are particularly preferred, and vinylene carbonate is most preferred.

The unsaturated cyclic carbonate may have any molecular weight that does not significantly impair the effects of the present disclosure. The molecular weight is preferably 50 or higher and 250 or lower. The unsaturated cyclic carbonate having a molecular weight within this range can easily ensure its solubility in the electrolyte solution and can easily lead to sufficient achievement of the effects of the present disclosure. The molecular weight of the unsaturated cyclic carbonate is more preferably 80 or higher and 150 or lower.

The unsaturated cyclic carbonate may be produced by any production method, and can be produced by a known method selected as appropriate.

One unsaturated cyclic carbonate may be used alone or two or more thereof may be used in any combination at any ratio.

The unsaturated cyclic carbonate content may be any amount that does not significantly impair the effects of the present disclosure. The unsaturated cyclic carbonate content is preferably 0.001% by mass or more, more preferably 0.01% by mass or more, still more preferably 0.1% by mass or more, while preferably 5% by mass or less, more preferably 4% by mass or less, still more preferably 3% by mass or less, per 100% by mass of the electrolyte solution. The unsaturated cyclic carbonate content within the above range allows an electrochemical device using the electrolyte solution to easily exhibit a sufficient effect of improving the cycle characteristics, and can easily avoid a situation with impaired high-temperature storage characteristics, generation of a large amount of gas, and a reduced discharge capacity retention.

In addition to the non-fluorinated unsaturated cyclic carbonates described above, a fluorinated unsaturated cyclic carbonate can also suitably be used as an unsaturated cyclic carbonate.

The fluorinated unsaturated cyclic carbonate is a cyclic carbonate containing an unsaturated bond and a fluorine atom. The number of fluorine atoms in the fluorinated unsaturated cyclic carbonate may be any number that is 1 or greater. The number of fluorine atoms is usually 6 or smaller, preferably 4 or smaller, most preferably 1 or 2.

Examples of the fluorinated unsaturated cyclic carbonate include fluorinated vinylene carbonate derivatives and fluorinated ethylene carbonate derivatives substituted with a substituent that contains an aromatic ring or a carbon-carbon double bond.

Examples of the fluorinated vinylene carbonate derivatives include 4-fluorovinylene carbonate, 4-fluoro-5-methylvinylene carbonate, 4-fluoro-5-phenylvinylene carbonate, 4-allyl-5-fluorovinylene carbonate, and 4-fluoro-5-vinylvinylene carbonate.

Examples of the fluorinated ethylene carbonate derivatives substituted with a substituent that contains an aromatic ring or a carbon-carbon double bond include 4-fluoro-4-vinylethylene carbonate, 4-fluoro-4-allylethylene carbonate, 4-fluoro-5-vinylethylene carbonate, 4-fluoro-5-allylethylene carbonate, 4,4-difluoro-4-vinylethylene carbonate, 4,4-difluoro-4-allylethylene carbonate, 4,5-difluoro-4-vinylethylene carbonate, 4,5-difluoro-4-allylethylene carbonate, 4-fluoro-4,5-divinylethylene carbonate, 4-fluoro-4,5-diallylethylene carbonate, 4,5-difluoro-4,5-divinylethylene carbonate, 4,5-difluoro-4,5-diallylethylene carbonate, 4-fluoro-4-phenylethylene carbonate, 4-fluoro-5-phenylethylene carbonate, 4,4-difluoro-5-phenylethylene carbonate, and 4,5-difluoro-4-phenylethylene carbonate.

Of these, in view of forming a stable interface protecting film, the following fluorinated unsaturated cyclic carbonates are more preferably used: 4-fluorovinylene carbonate, 4-fluoro-5-methylvinylene carbonate, 4-fluoro-5-vinylvinylene carbonate, 4-allyl-5-fluorovinylene carbonate, 4-fluoro-4-vinylethylene carbonate, 4-fluoro-4-allylethylene carbonate, 4-fluoro-5-vinylethylene carbonate, 4-fluoro-5-allylethylene carbonate, 4,4-difluoro-4-vinylethylene carbonate, 4,4-difluoro-4-allylethylene carbonate, 4,5-difluoro-4-vinylethylene carbonate, 4,5-difluoro-4-allylethylene carbonate, 4-fluoro-4,5-divinylethylene carbonate, 4-fluoro-4,5-diallylethylene carbonate, 4,5-difluoro-4,5-divinylethylene carbonate, and 4,5-difluoro-4,5-diallylethylene carbonate.

The fluorinated unsaturated cyclic carbonate may have any molecular weight that does not significantly impair the effects of the present disclosure. The molecular weight is preferably 50 or higher and 500 or lower. A fluorinated unsaturated cyclic carbonate having a molecular weight within this range is more likely to have solubility in the electrolyte solution.

The fluorinated unsaturated cyclic carbonate may be produced by any method, and may be produced according to any known method selected as appropriate. The molecular weight is more preferably 100 or higher and more preferably 200 or lower.

One fluorinated unsaturated cyclic carbonate may be used alone, or two or more thereof may be used in any combination at any ratio. The fluorinated unsaturated cyclic carbonate may be contained in any amount that does not significantly impair the effects of the present disclosure. The amount of the fluorinated unsaturated cyclic carbonate is usually preferably 0.001% by mass or more, more preferably 0.01% by mass or more, still more preferably 0.1% by mass or more, while preferably 5% by mass or less, more preferably 4% by mass or less, still more preferably 3% by mass or less, of 100% by mass of the electrolyte solution. A fluorinated unsaturated cyclic carbonate in an amount within this range is more likely to allow an electrochemical device containing the electrolyte solution to sufficiently exhibit an effect of improving cycle characteristics and more likely to prevent reduced high-temperature storage characteristics, generation of a large amount of gas, and a reduced discharge capacity retention.

The electrolyte solution of the present disclosure may contain a compound containing a triple bond. This compound may be of any type as long as it contains one or more triple bonds in the molecule.

Specific examples of the compound containing a triple bond include the following compounds:

hydrocarbon compounds such as 1-penthyne, 2-penthyne, 1-hexyne, 2-hexyne, 3-hexyne, 1-heptyne, 2-heptyne, 3-heptyne, 1-octyne, 2-octyne, 3-octyne, 4-octyne, 1-nonyne, 2-nonyne, 3-nonyne, 4-nonyne, 1-dodecyne, 2-dodecyne, 3-dodecyne, 4-dodecyne, 5-dodecyne, phenyl acetylene, 1-phenyl-1-propyne, 1-phenyl-2-propyne, 1-phenyl-1-butyne, 4-phenyl-1-butyne, 4-phenyl-1-butyne, 1-phenyl-1-penthyne, 5-phenyl-1-penthyne, 1-phenyl-1-hexyne, 6-phenyl-1-hexyne, diphenyl acetylene, 4-ethynyl toluene, and dicyclohexyl acetylene;

monocarbonates such as 2-propynylmethyl carbonate, 2-propynylethyl carbonate, 2-propynylpropyl carbonate, 2-propynylbutyl carbonate, 2-propynylphenyl carbonate, 2-propynylcyclohexyl carbonate, di-2-propynylcarbonate, 1-methyl-2-propynylmethyl carbonate, 1,1-dimethyl-2-propynylmethyl carbonate, 2-butynylmethyl carbonate, 3-butynylmethyl carbonate, 2-pentynylmethyl carbonate, 3-pentynylmethyl carbonate, and 4-pentynylmethyl carbonate;

dicarbonates such as 2-butyne-1,4-diol dimethyl dicarbonate, 2-butyne-1,4-diol diethyl dicarbonate, 2-butyne-1,4-diol dipropyl dicarbonate, 2-butyne-1,4-diol dibutyl dicarbonate, 2-butyne-1,4-diol diphenyl dicarbonate, and 2-butyne-1,4-diol dicyclohexyl dicarbonate;

monocarboxylic acid esters such as 2-propynyl acetate, 2-propynyl propionate, 2-propynyl butyrate, 2-propynyl

benzoate, 2-propynyl cyclohexylcarboxylate, 1,1-dimethyl-2-propynyl acetate, 1,1-dimethyl-2-propynyl propionate, 1,1-dimethyl-2-propynyl butyrate, 1,1-dimethyl-2-propynyl benzoate, 1,1-dimethyl-2-propynyl cyclohexylcarboxylate, 2-butynyl acetate, 3-butynyl acetate, 2-pentynyl acetate, 3-pentynyl acetate, 4-pentynyl acetate, methyl acrylate, ethyl acrylate, propyl acrylate, vinyl acrylate, 2-propenyl acrylate, 2-butenyl acrylate, 3-butenyl acrylate, methyl methacrylate, ethyl methacrylate, propyl methacrylate, vinyl methacrylate, 2-propenyl methacrylate, 2-butenyl methacrylate, 3-butenyl methacrylate, methyl 2-propynoate, ethyl 2-propynoate, propyl 2-propynoate, vinyl 2-propynoate, 2-propenyl 2-propynoate, 2-butenyl 2-propynoate, 3-butenyl 2-propynoate, methyl 2-butynoate, ethyl 2-butynoate, propyl 2-butynoate, vinyl 2-butynoate, 2-propenyl 2-butynoate, 2-butenyl 2-butynoate, 3-butenyl 2-butynoate, methyl 3-butynoate, ethyl 3-butynoate, propyl 3-butynoate, vinyl 3-butynoate, 2-propenyl 3-butynoate, 2-butenyl 3-butynoate, 3-butenyl 3-butynoate, methyl 2-penthynoate, ethyl 2-penthynoate, propyl 2-penthynoate, vinyl 2-penthynoate, 2-propenyl 2-penthynoate, 2-butenyl 2-penthynoate, 3-butenyl 2-penthynoate, methyl 3-penthynoate, ethyl 3-penthynoate, propyl 3-penthynoate, vinyl 3-penthynoate, 2-propenyl 3-penthynoate, 2-butenyl 3-penthynoate, 3-butenyl 3-penthynoate, methyl 4-penthynoate, ethyl 4-penthynoate, propyl 4-penthynoate, vinyl 4-penthynoate, 2-propenyl 4-penthynoate, 2-butenyl 4-penthynoate, and 3-butenyl 4-penthynoate, fumarates, methyl trimethylacetate, and ethyl trimethylacetate;

dicarboxylic acid esters such as 2-butyne-1,4-diol diacetate, 2-butyne-1,4-diol dipropionate, 2-butyne-1,4-diol dibutyrate, 2-butyne-1,4-diol dibenzoate, 2-butyne-1,4-diol dicyclohexanecarboxylate, hexahydrobenzo[1,3,2]dioxathiolane-2-oxide (1,2-cyclohexanediol, 2,2-dioxide-1,2-oxathiolan-4-yl acetate, and 2,2-dioxide-1,2-oxathiolan-4-yl acetate;

oxalic acid diesters such as methyl 2-propynyl oxalate, ethyl 2-propynyl oxalate, propyl 2-propynyl oxalate, 2-propynyl vinyl oxalate, allyl 2-propynyl oxalate, di-2-propynyl oxalate, 2-butynyl methyl oxalate, 2-butynyl ethyl oxalate, 2-butynyl propyl oxalate, 2-butynyl vinyl oxalate, allyl 2-butynyl oxalate, di-2-butynyl oxalate, 3-butynyl methyl oxalate, 3-butynyl ethyl oxalate, 3-butynyl propyl oxalate, 3-butynyl vinyl oxalate, allyl 3-butynyl oxalate, and di-3-butynyl oxalate;

phosphine oxides such as methyl(2-propynyl)(vinyl)phosphine oxide, divinyl(2-propynyl)phosphine oxide, di(2-propynyl)(vinyl)phosphine oxide, di(2-propenyl)2(-propynyl)phosphine oxide, di(2-propynyl)(2-propenyl)phosphine oxide, di(3-butenyl)(2-propynyl)phosphine oxide, and di(2-propynyl)(3-butenyl)phosphine oxide;

phosphinates such as 2-propynyl methyl(2-propenyl)phosphinate, 2-propynyl 2-butenyl(methyl)phosphinate, 2-propynyl di(2-propenyl)phosphinate, 2-propynyl di(3-butenyl)phosphinate, 1,1-dimethyl-2-propynyl methyl(2-propenyl)phosphinate, 1,1-dimethyl-2-propynyl 2-butenyl(methyl)phosphinate, 1,1-dimethyl-2-propynyl di(2-propenyl)phosphinate, 1,1-dimethyl-2-propynyl di(3-butenyl)phosphinate, 2-propenyl methyl(2-propynyl)phosphinate, 3-butenyl methyl(2-propynyl)phosphinate, 2-propenyl di(2-propynyl)phosphinate, 3-butenyl di(2-propynyl)phosphinate, 2-propenyl 2-propynyl(2-propenyl)phosphinate, and 3-butenyl 2-propynyl(2-propenyl)phosphinate;

phosphonates such as methyl 2-propynyl 2-propenylphosphonate, methyl(2-propynyl) 2-butenylphosphonate, (2-propynyl)(2-propenyl) 2-propenylphosphonate, (3-butenyl)(2-propynyl) 3-butenylphosphonate, (1,1-dimethyl-2-propynyl)(methyl) 2-propenylphosphonate, (1,1-dimethyl-2-propynyl)(methyl) 2-butenylphosphonate, (1,1-dimethyl-2-propynyl)(2-propenyl) 2-propenylphosphonate, (3-butenyl)(1,1-dimethyl-2-propynyl) 3-butenylphosphonate, (2-propynyl)(2-propenyl) methylphosphonate, (3-butenyl)(2-propynyl) methylphosphonate, (1,1-dimethyl-2-propynyl)(2-propenyl) methylphosphonate, (3-butenyl)(1,1-dimethyl-2-propynyl) methylphosphonate, (2-propynyl)(2-propenyl) ethylphosphonate, (3-butenyl)(2-propynyl) ethylphosphonate, (1,1-dimethyl-2-propynyl)(2-propenyl) ethylphosphonate, and (3-butenyl)(1,1-dimethyl-2-propynyl) ethylphosphonate; and phosphates such as (methyl)(2-propenyl)(2-propynyl) phosphate, (ethyl)(2-propenyl)(2-propynyl) phosphate, (2-butenyl)(methyl)(2-propynyl) phosphate, (2-butenyl)(ethyl)(2-propynyl) phosphate, (1,1-dimethyl-2-propynyl)(methyl)(2-propenyl) phosphate, (1,1-dimethyl-2-propynyl)(ethyl)(2-propenyl) phosphate, (2-butenyl)(1,1-dimethyl-2-propynyl)(methyl) phosphate, and (2-butenyl)(ethyl)(1,1-dimethyl-2-propynyl) phosphate.

Of these, compounds containing an alkynyloxy group are preferred in order to more stably form a negative electrode film in the electrolyte solution.

In view of improved storage characteristics, compounds such as 2-propynylmethyl carbonate, di-2-propynyl carbonate, 2-butyne-1,4-diol dimethyl dicarbonate, 2-propynyl acetate, 2-butyne-1,4-diol diacetate, methyl 2-propynyl oxalate, and di-2-propynyl oxalate are particularly preferred.

One compound containing a triple bond may be used alone, or two or more thereof may be used in any combination at any ratio. The compound containing a triple bond may be present in any amount that does not significantly impair the effects of the present disclosure relative to the entire electrolyte solution of the present disclosure. The compound can be typically contained at a concentration of 0.01% by mass or more, preferably 0.05% by mass or more, more preferably 0.1% by mass or more, while typically 5% by mass or less, preferably 3% by mass or less, more preferably 1% by mass or less, relative to the electrolyte solution of the present disclosure. A compound satisfying the above range is more likely to further improve the effects such as output characteristics, load characteristics, cycle

characteristics, and high-temperature storage characteristics.

The electrolyte solution of the present disclosure may contain an overcharge inhibitor in order to effectively reduce or prevent the burst or combustion of a battery in case of overcharge, for example, of an electrochemical device containing the electrolyte solution.

Examples of overcharge inhibitors include aromatic compounds, including unsubstituted or alkyl-substituted terphenyl derivatives such as biphenyl, o-terphenyl, m-terphenyl, and p-terphenyl, partially hydrogenated products of unsubstituted or alkyl-substituted terphenyl derivatives, cyclohexylbenzene, t-butylbenzene, t-amylbenzene, diphenyl ether, dibenzofuran, diphenyl cyclohexane, 1,1,3-trimethyl-3-phenylindan, cyclopentylbenzene, cyclohexylbenzene, cumene, 1,3-diisopropylbenzene, 1,4-diisopropylbenzene, t-butylbenzene, t-amylbenzene, t-hexylbenzene, and anisole; partially fluorinated products of the above aromatic compounds such as 2-fluorobiphenyl, 4-fluorobiphenyl, o-cyclohexylfluorobenzene, p-cyclohexylfluorobenzene, fluorotoluene, and benzotrifluoride; fluorinated anisole compounds such as 2,4-difluoroanisole, 2,5-difluoroanisole, 1,6-difluoroanisole, 2,6-difluoroanisole, and 3,5-difluoroanisole; aromatic acetates such as 3-propylphenyl acetate, 2-ethylphenyl acetate, benzylphenyl acetate, methylphenyl acetate, benzyl acetate, and phenethylphenyl acetate; aromatic carbonates such as diphenyl carbonate and methylphenyl carbonate, toluene derivatives such as toluene and xylene, and unsubstituted or alkyl-substituted biphenyl derivatives such as 2-methylbiphenyl, 3-methylbiphenyl, 4-methylbiphenyl, and o-cyclohexylbiphenyl. In particular, the following are preferred: aromatic compounds such as biphenyl, alkylbiphenyl, terphenyl, partially hydrogenated terphenyl, cyclohexylbenzene, t-butylbenzene, t-amylbenzene, diphenyl ether, and dibenzofuran, diphenyl cyclohexane, 1,1,3-trimethyl-3-phenylindan, 3-propylphenyl acetate, 2-ethylphenyl acetate, benzylphenyl acetate, methylphenyl acetate, benzyl acetate, diphenyl carbonate, and methylphenyl carbonate. One of these compounds may be used alone or two or more thereof may be used in any combination. In view of the balance between overcharge inhibiting characteristics and high-temperature storage characteristics, the combination use of two or more of these compounds is preferably a combination of cyclohexylbenzene with t-butylbenzene or t-amylbenzene, or a combination of at least one oxygen-free aromatic compound selected from biphenyl, alkylbiphenyl, terphenyl, partially hydrogenated terphenyl, cyclohexylbenzene, t-butylbenzene, t-amylbenzene, or the like with at least one oxygen-containing aromatic compound selected from diphenyl ether, dibenzofuran, or the like.

The electrolyte solution for use in the present disclosure may contain a carboxylic anhydride other than the compound (2). The carboxylic anhydride is preferably a compound represented by the following general formula (6). The carboxylic anhydride may be produced according to any method selected from known methods.

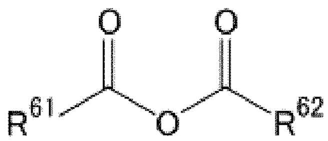

In general formula (6), $R^{61}$ and $R^{62}$ are each independently a hydrocarbon group having a carbon number of 1 or greater and 15 or smaller and optionally containing a substituent.

$R^{61}$ and $R^{62}$ each may be any monovalent hydrocarbon group. For example, $R^{61}$ and $R^{62}$ each may be either an aliphatic hydrocarbon group or an aromatic hydrocarbon group, or may be a group formed by a bond of an aliphatic hydrocarbon group with an aromatic hydrocarbon group. The aliphatic hydrocarbon group may be a saturated hydrocarbon group or may contain an unsaturated bond (carbon-carbon double bond or carbon-carbon triple bond). The aliphatic hydrocarbon group may be either chain or cyclic. In the case of a chain group, it may be either linear or branched. The group may be a bond of a chain group and a cyclic group. $R^{61}$ and $R^{62}$ may be the same as or different from each other.

When the hydrocarbon group represented by $R^{61}$ and $R^{62}$ contains a substituent, the substituent may be of any type as long as it is not beyond the scope of the present disclosure. Examples of substituents include halogen atoms such as a fluorine atom, a chlorine atom, a bromine atom, and an iodine atom, with a fluorine atom being preferred. Examples of substituents other than halogen atoms include substituents containing a functional group such as an ester group, a cyano group, a carbonyl group, or an ether group, with a cyano group and a carbonyl group being preferred. The hydrocarbon group represented by $R^{61}$ and $R^{62}$ may contain only one of these substituents, or two or more of these. When the hydrocarbon group contains two or more substituents, these substituents may be the same as or different from each other.

The hydrocarbon group represented by $R^{61}$ and $R^{62}$ has a carbon number of usually 1 or greater, while usually 15 or smaller, preferably 12 or smaller, more preferably 10 or smaller, still more preferably 9 or smaller. When $R^1$ and $R^2$ bind to each other to form a divalent hydrocarbon group, the divalent hydrocarbon group has a carbon number of usually 1 or greater, while usually 15 or smaller, preferably 13 or smaller, more preferably 10 or smaller, still more preferably 8 or smaller. When the hydrocarbon group represented by $R^{61}$ and $R^{62}$ contains a substituent that contains a carbon atom, the carbon number of the whole $R^{61}$ or $R^{62}$ including the substituent preferably satisfies the above range.

The following describes specific examples of the acid anhydride represented by general formula (6). In the examples below, the term "analog" means an acid anhydride obtainable by replacing part of the structure of an acid anhydride described as an example by another structure without departing from the spirit and principal concept of the present disclosure. Examples include dimers, trimers, and tetramers each composed of multiple acid anhydrides, structural isomers, such as those having the same carbon number but having a branched chain, and those having the same substituent but at different sites of the acid anhydrides.

First, specific examples of an acid anhydride in which $R^{61}$ and $R^{62}$ are the same as each other are described.

Specific examples of acid anhydrides in which $R^{61}$ and $R^{62}$ are chain alkyl groups include acetic anhydride, propionic anhydride, butanoic anhydride, 2-methylpropionic anhydride, 2,2-dimethylpropionic anhydride, 2-methylbutanoic anhydride, 3-methylbutanoic anhydride, 2,2-dimethylbutanoic anhydride, 2,3-dimethylbutanoic anhydride, 3,3-dimethylbutanoic anhydride, 2,2,3-trimethylbutanoic anhydride, 2,3,3-trimethylbutanoic anhydride, 2,2,3,3-tetramethylbutanoic anhydride, 2-ethylbutanoic anhydride, and analogs thereof.

Specific examples of acid anhydrides in which $R^{61}$ and $R^{62}$ are cyclic alkyl groups include cyclopropanecarboxylic anhydride, cyclopentanecarboxylic anhydride, cyclohexanecarboxylic anhydride, and analogs thereof.

Specific examples of acid anhydrides in which $R^{61}$ and $R^{62}$ are alkenyl groups include acrylic anhydride, 2-methylacrylic anhydride, 3-methylacrylic anhydride, 2,3-dimethylacrylic anhydride, 3,3-dimethylacrylic anhydride, 2,3,3-trimethylacrylic anhydride, 2-phenylacrylic anhydride, 3-phenylacrylic anhydride, 2,3-diphenylacrylic anhydride, 3,3-diphenylacrylic anhydride, 3-butenoic anhydride, 2-methyl-3-butenoic anhydride, 2,2-dimethyl-3-butenoic anhydride, 3-methyl-3-butenoic anhydride, 2-methyl-3-methyl-3-butenoic anhydride, 2,2-dimethyl-3-methyl-3-butenoic anhydride, 3-pentenoic anhydride, 4-pentenoic anhydride, 2-cyclopentenecarboxylic anhydride, 3-cyclopentenecarboxylic anhydride, 4-cyclopentenecarboxylic anhydride, and analogs thereof.

Specific examples of acid anhydrides in which $R^{61}$ and $R^{62}$ are alkynyl groups include propynoic anhydride, 3-phenylpropynoic anhydride, 2-butynoic anhydride, 2-penthynoic anhydride, 3-butynoic anhydride, 3-penthynoic anhydride, 4-penthynoic anhydride, and analogs thereof.

Specific examples of acid anhydrides in which $R^{61}$ and $R^{62}$ are aryl groups include benzoic anhydride, 4-methylbenzoic anhydride, 4-ethylbenzoic anhydride, 4-tert-butylbenzoic anhydride, 2-methylbenzoic anhydride, 2,4,6-trimethylbenzoic anhydride, 1-naphthalenecarboxylic anhydride, 2-naphthalenecarboxylic anhydride, and analogs thereof.

Examples of acid anhydrides substituted with a fluorine atom are mainly listed below as examples of acid anhydrides in which $R^{61}$ and $R^{62}$ are substituted with a halogen atom. Acid anhydrides obtainable by replacing any or all of the fluorine atoms with a chlorine atom, a bromine atom, or an iodine atom are also included in the exemplary compounds.

Examples of acid anhydrides in which $R^{61}$ and $R^{62}$ are halogen-substituted chain alkyl groups include fluoroacetic anhydride, difluoroacetic anhydride, trifluoroacetic anhydride, 2-fluoropropionic anhydride, 2,2-difluoropropionic anhydride, 2,3-difluoropropionic anhydride, 2,2,3-trifluoropropionic anhydride, 2,3,3-trifluoropropionic anhydride, 2,2,3,3-tetrapropionic anhydride, 2,3,3,3-tetrapropionic anhydride, 3-fluoropropionic anhydride, 3,3-difluoropropionic anhydride, 3,3,3-trifluoropropionic anhydride, perfluoropropionic anhydride, and analogs thereof.

Examples of acid anhydrides in which $R^{61}$ and $R^{62}$ are halogen-substituted cyclic alkyl groups include 2-fluorocyclopentanecarboxylic anhydride, 3-fluorocyclopentanecarboxylic anhydride, 4-fluorocyclopentanecarboxylic anhydride, and analogs thereof.

Examples of acid anhydrides in which $R^{61}$ and $R^{62}$ are halogen-substituted alkenyl groups include 2-fluoroacrylic anhydride, 3-fluoroacrylic anhydride, 2,3-difluoroacrylic anhydride, 3,3-difluoroacrylic anhydride, 2,3,3-trifluoroacrylic anhydride, 2-(trifluoromethyl)acrylic anhydride, 3-(trifluoromethyl)acrylic anhydride, 2,3-bis(trifluoromethyl)acrylic anhydride, 2,3,3-tris(trifluoromethyl)acrylic anhydride, 2-(4-fluorophenyl)acrylic anhydride, 3-(4-fluorophenyl)acrylic anhydride, 2,3-bis(4-fluorophenyl)acrylic anhydride, 3,3-bis(4-fluorophenyl)acrylic anhydride, 2-fluoro-3-butenoic anhydride, 2,2-difluoro-3-butenoic anhydride, 3-fluoro-2-butenoic anhydride, 4-fluoro-3-butenoic anhydride, 3,4-difluoro-3-butenoic anhydride, 3,3,4-trifluoro-3-butenoic anhydride, and analogs thereof.

Examples of acid anhydrides in which $R^{61}$ and $R^{62}$ are halogen-substituted alkynyl groups include 3-fluoro-2-propynoic anhydride, 3-(4-fluorophenyl)-2-propynoic anhydride, 3-(2,3,4,5,6-pentafluorophenyl)-2-propynoic anhydride, 4-fluoro-2-butynoic anhydride, 4,4-difluoro-2-butynoic anhydride, 4,4,4-trifluoro-2-butynoic anhydride, and analogs thereof.

Examples of acid anhydrides in which $R^{61}$ and $R^{62}$ are halogen-substituted aryl groups include 4-fluorobenzoic anhydride, 2,3,4,5,6-pentafluorobenzoic anhydride, 4-trifluoromethylbenzoic anhydride, and analogs thereof.

Examples of acid anhydrides in which $R^{61}$ and $R^{62}$ each contain a substituent containing a functional group such as an ester, a nitrile, a ketone, or an ether include methoxyformic anhydride, ethoxyformic anhydride, methyloxalic anhydride, ethyloxalic anhydride, 2-cyanoacetic anhydride, 2-oxopropionic anhydride, 3-oxobutanoic anhydride, 4-acetylbenzoic anhydride, methoxyacetic anhydride, 4-methoxybenzoic anhydride, and analogs thereof.

Then, specific examples of acid anhydrides in which $R^{61}$ and $R^{62}$ are different from each other are described

below.

$R^{61}$ and $R^{62}$ may be in any combination of those described as examples above and analogs thereof. The following gives representative examples.

Examples of combinations of chain alkyl groups include acetic propionic anhydride, acetic butanoic anhydride, butanoic propionic anhydride, and acetic 2-methylpropionic anhydride.

Examples of combinations of a chain alkyl group and a cyclic alkyl group include acetic cyclopentanoic anhydride, acetic cyclohexanoic anhydride, and cyclopentanoic propionic anhydride.

Examples of combinations of a chain alkyl group and an alkenyl group include acetic acrylic anhydride, acetic 3-methylacrylic anhydride, acetic 3-butenoic anhydride, and acrylic propionic anhydride.

Examples of combinations of a chain alkyl group and an alkynyl group include acetic propynoic anhydride, acetic 2-butynoic anhydride, acetic 3-butynoic anhydride, acetic 3-phenyl propynoic anhydride, and propionic propynoic anhydride.

Examples of combinations of a chain alkyl group and an aryl group include acetic benzoic anhydride, acetic 4-methylbenzoic anhydride, acetic 1-naphthalenecarboxylic anhydride, and benzoic propionic anhydride.

Examples of combinations of a chain alkyl group and a hydrocarbon group containing a functional group include acetic fluoroacetic anhydride, acetic trifluoroacetic anhydride, acetic 4-fluorobenzoic anhydride, fluoroacetic propionic anhydride, acetic alkyloxalic anhydride, acetic 2-cyanoacetic anhydride, acetic 2-oxopropionic anhydride, acetic methoxyacetic anhydride, and methoxyacetic propionic anhydride.

Examples of combinations of cyclic alkyl groups include cyclopentanoic cyclohexanoic anhydride.

Examples of combinations of a cyclic alkyl group and an alkenyl group include acrylic cyclopentanoic anhydride, 3-methylacrylic cyclopentanoic anhydride, 3-butenoic cyclopentanoic anhydride, and acrylic cyclohexanoic anhydride.

Examples of combinations of a cyclic alkyl group and an alkynyl group include propynoic cyclopentanoic anhydride, 2-butynoic cyclopentanoic anhydride, and propynoic cyclohexanoic anhydride.

Examples of combinations of a cyclic alkyl group and an aryl group include benzoic cyclopentanoic anhydride, 4-methylbenzoic cyclopentanoic anhydride, and benzoic cyclohexanoic anhydride.

Examples of combinations of a cyclic alkyl group and a hydrocarbon group containing a functional group include fluoroacetic cyclopentanoic anhydride, cyclopentanoic trifluoroacetic anhydride, cyclopentanoic 2-cyanoacetic anhydride, cyclopentanoic methoxyacetic anhydride, and cyclohexanoic fluoroacetic anhydride.

Examples of combinations of alkenyl groups include acrylic 2-methylacrylic anhydride, acrylic 3-methylacrylic anhydride, acrylic 3-butenoic anhydride, and 2-methylacrylic 3-methylacrylic anhydride.

Examples of combinations of an alkenyl group and an alkynyl group include acrylic propynoic anhydride, acrylic 2-butynoic anhydride, and 2-methylacrylic propynoic anhydride.

Examples of combinations of an alkenyl group and an aryl group include acrylic benzoic anhydride, acrylic 4-methylbenzoic anhydride, and 2-methylacrylic benzoic anhydride.

Examples of combinations of an alkenyl group and a hydrocarbon group containing a functional group include acrylic fluoroacetic anhydride, acrylic trifluoroacetic anhydride, acrylic 2-cyanoacetic anhydride, acrylic methoxyacetic anhydride, and 2-methylacrylic fluoroacetic anhydride.

Examples of combinations of alkynyl groups include propynoic 2-butynoic anhydride, propynoic 3-butynoic anhydride, and 2-butynoic 3-butynoic anhydride.

Examples of combinations of an alkynyl group and an aryl group include benzoic propynoic anhydride, 4-methylbenzoic propynoic anhydride, and benzoic 2-butynoic anhydride.

Examples of combinations of an alkynyl group and a hydrocarbon group containing a functional group include propynoic fluoroacetic anhydride, propynoic trifluoroacetic anhydride, propynoic 2-cyanoacetic anhydride, propynoic methoxyacetic anhydride, and 2-butynoic fluoroacetic anhydride.

Examples of combinations of aryl groups include benzoic 4-methylbenzoic anhydride, benzoic 1-naphthalenecarboxylic anhydride, and 4-methylbenzoic 1-naphthalenecarboxylic anhydride.

Examples of combinations of an aryl group and a hydrocarbon group containing a functional group include benzoic fluoroacetic anhydride, benzoic trifluoroacetic anhydride, benzoic 2-cyanoacetic anhydride, benzoic methoxyacetic anhydride, and 4-methylbenzoic fluoroacetic anhydride.

Examples of combinations of hydrocarbon groups each containing a functional group include fluoroacetic trifluoroacetic anhydride, fluoroacetic 2-cyanoacetic anhydride, fluoroacetic methoxyacetic anhydride, and trifluoroacetic 2-cyanoacetic anhydride.

Of the acid anhydrides having a chain structure, the following are preferred: acetic anhydride, propionic anhydride, 2-methylpropionic anhydride, cyclopentanecarboxylic anhydride, cyclohexanecarboxylic anhydride, acrylic anhydride, 2-methylacrylic anhydride, 3-methylacrylic anhydride, 2,3-dimethylacrylic anhydride, 3,3-dimethylacrylic anhydride, 3-butenoic anhydride, 2-methyl-3-butenoic anhydride, propynoic anhydride, 2-butynoic anhydride, benzoic anhydride, 2-methylbenzoic anhydride, 4-methylbenzoic anhydride, 4-tert-butylbenzoic anhydride, trifluoroacetic anhydride, 3,3,3-trifluoropropionic anhydride, 2-(trifluoromethyl)acrylic anhydride, 2-(4-fluorophenyl)acrylic anhydride, 4-fluorobenzoic anhydride, 2,3,4,5,6-pentafluorobenzoic anhydride, methoxyformic anhydride, and ethoxyformic anhydride. The following are more preferred: acrylic anhydride, 2-methylacrylic anhydride, 3-methylacrylic anhydride, benzoic anhydride, 2-methylbenzoic anhydride, 4-methylbenzoic anhydride, 4-tert-butylbenzoic anhydride, 4-fluorobenzoic anhydride, 2,3,4,5,6-pentafluorobenzoic anhydride, methoxyformic anhydride, and ethoxyformic anhydride.

These compounds are preferred because they are likely to appropriately form a bond with lithium oxalate to provide a film having excellent durability, and thus improve, in particular, the charge and discharge rate characteristics after a durability test, input and output characteristics, and impedance characteristics.

The carboxylic anhydride may have any molecular weight that does not significantly impair the effects of the present disclosure. The molecular weight is usually 90 or higher, preferably 95 or higher, while usually 300 or lower, preferably 200 or lower. A carboxylic anhydride having a molecular weight within the above range is likely to appropriately improve durability due to its ability to limit an increase in viscosity of an electrolyte solution and to optimize film density.

The carboxylic anhydride may be produced according to any production method, which may be selected from known methods. One of the carboxylic anhydrides described above alone may be contained in the non-aqueous electrolyte solution of the present disclosure, or two or more thereof may be contained in any combination at any ratio.

The carboxylic anhydride may be contained in any amount that does not significantly impair the effects of the present disclosure relative to the electrolyte solution of the present disclosure. The carboxylic anhydride is usually contained at a concentration of 0.01% by mass or more, preferably 0.1% by mass or more, while usually 5% by mass or less, preferably 3% by mass or less, relative to the electrolyte solution of the present disclosure. A carboxylic anhydride in an amount within the above range can easily achieve an effect of improving cycle characteristics and easily improves battery characteristics due to its good reactivity.

The electrolyte solution of the present disclosure may further contain an additional known aid. Examples of such aids include hydrocarbon compounds such as pentane, heptane, octane, nonane, decane, cycloheptane, benzene, furan, naphthalene, 2-phenyl bicyclohexyl, cyclohexane, 2,4,8,10-tetraoxaspiro[5.5]undecane, and 3,9-divinyl-2,4,8,10-tetraoxaspiro[5.5]undecane;

fluorinated aromatic compounds such as fluorobenzene, difluorobenzene, hexafluorobenzene, benzotrifluoride, monofluorobenzene, 1-fluoro-2-cyclohexylbenzene, 1-fluoro-4-tert-butylbenzene, 1-fluoro-3-cyclohexylbenzene, 1-fluoro-2-cyclohexylbenzene, and biphenyl fluoride;

carbonate compounds such as erythritan carbonate, spiro-bis-dimethylene carbonate, and methoxyethyl-methyl carbonate;

ether compounds such as dioxolane, dioxane, 2,5,8,11-tetraoxadodecane, 2,5,8,11,14-pentaoxapentadecane, ethoxymethoxyethane, trimethoxymethane, glyme, and ethyl monoglyme;

ketone compounds such as dimethyl ketone, diethyl ketone, and 3-pentanone;

acid anhydrides such as 2-allylsuccinic anhydride;

ester compounds such as dimethyl oxalate, diethyl oxalate, ethylmethyl oxalate, di(2-propynyl) oxalate, methyl 2-propynyl oxalate, dimethyl succinate, di(2-propynyl) glutarate, methyl formate, ethyl formate, 2-propynyl formate, 2-butyne-1,4-diyl diformate, 2-propynyl methacrylate, and dimethyl malonate;

amide compounds such as acetamide, N-methyl formamide, N,N-dimethyl formamide, and N,N-dimethyl acetamide;

sulfur-containing compounds such as ethylene sulfate, vinylene sulfate, ethylene sulfite, methyl fluorosulfonate, ethyl fluorosulfonate, methyl methanesulfonate, ethyl methanesulfonate, busulfan, sulfolene, diphenyl sulfone, N,N-dimethylmethanesulfonamide, N,N-diethylmethanesulfonamide, methyl vinyl sulfonate, ethyl vinyl sulfonate, allyl vinyl sulfonate, propargyl vinyl sulfonate, methyl allyl sulfonate, ethyl allyl sulfonate, allyl allyl sulfonate, propargyl allyl sulfonate, 1,2-bis(vinylsulfonyloxy)ethane, propanedisulfonic anhydride, sulfobutyric anhydride, sulfobenzoic anhydride, sulfopropionic anhydride, ethanedisulfonic anhydride, methylene methanedisulfonate, 2-propynyl methanesulfonate, pentene sulfite, pentafluorophenyl methanesulfonate, propylene sulfate, propylene sulfite, propane sultone, butylene sulfite, butane-2,3-diyl dimethanesulfonate, 2-butyne-1,4-diyl dimethanesulfonate, 2-propynyl vinyl sulfonate, bis(2-vinylsulfonylethyl)ether, 5-vinyl-hexahydro-1,3,2-benzodioxathiol-2-oxide, 2-propynyl 2-(methanesulfonyloxy)propionate, 5,5-dimethyl-1,2-oxathiolan-4-one 2,2-dioxide, 3-sulfo-propionic anhydride, trimethylene methanedisulfonate, 2-methyl tetrahydrofuran, trimethylene methanedisulfonate, tetramethylene sulfoxide, dimethylene methanedisulfonate, difluoroethyl methyl sulfone, divinyl sulfone, 1,2-bis(vinylsulfonyl)ethane, methyl ethylenebissulfonate, ethyl ethylenebissulfonate, ethylene sulfate, and thiophene 1-oxide;

nitrogen-containing compounds such as 1-methyl-2-pyrrolidinone, 1-methyl-2-piperidone, 3-methyl-2-oxazolidinone, 1,3-dimethyl-2-imidazolidinone, N-methylsuccinimide, nitromethane, nitroethane, and ethylene diamine;

phosphorus-containing compounds such as trimethyl phosphite, triethyl phosphite, triphenyl phosphite, trimethyl phosphate, triethyl phosphate, triphenyl phosphate, dimethyl methyl phosphonate, diethyl ethyl phosphonate, dimethyl vinyl phosphonate, diethyl vinyl phosphonate, ethyl diethyl phosphonoacetate, methyl dimethyl phosphinate, ethyl diethyl phosphinate, trimethylphosphine oxide, triethylphosphine oxide, bis(2,2-difluoroethyl)2,2,2-trifluoroethyl phosphate, bis(2,2,3,3-tetrafluoropropyl)2,2,2-trifluoroethyl phosphate, bis(2,2,2-trifluoroethyl)methyl phosphate,

bis(2,2,2-trifluoroethyl)ethyl phosphate, bis(2,2,2-trifluoroethyl)2,2-difluoroethyl phosphate, bis(2,2,2-trifluoroethyl)2,2,3,3-tetrafluoropropyl phosphate, tributyl phosphate, tris(2,2,2-trifluoroethyl) phosphate, tris(1,1,1,3,3,3-hexafluoropropan-2-yl) phosphate, trioctyl phosphate, 2-phenylphenyldimethyl phosphate, 2-phenylphenyldiethyl phosphate, (2,2,2-trifluoroethyl)(2,2,3,3-tetrafluoropropyl)methyl phosphate, methyl 2-(dimethoxyphosphoryl) acetate, methyl 2-(dimethylphosphoryl) acetate, methyl 2-(diethoxyphosphoryl) acetate, methyl 2-(diethylphosphoryl) acetate, methyl methylenebisphosphonate, ethyl methylenebisphosphonte, methyl ethylenebisphosphonte, ethyl ethylenebisphosphonte, methyl butylenebisphosphonate, ethyl butylenebisphosphonate, 2-propynyl 2-(dimethoxyphosphoryl) acetate, 2-propynyl 2-(dimethylphosphoryl) acetate, 2-propynyl 2-(diethoxyphosphoryl) acetate, 2-propynyl 2-(diethylphosphoryl) acetate, tris(trimethylsilyl) phosphate, tris(triethylsilyl) phosphate, tris(trimethoxysilyl) phosphate, tris(trimethylsilyl) phosphite, tris(triethylsilyl) phosphite, tris(trimethoxysilyl) phosphite, and trimethylsilyl polyphosphate;

boron-containing compounds such as tris(trimethylsilyl) borate and tris(trimethoxysilyl) borate; and

silane compounds such as dimethoxyaluminoxytrimethoxysilane, diethoxyaluminoxytriethoxysilane, dipropoxyaluminoxytriethoxysilane, dibutoxyaluminoxytrimethoxysilane, dibutoxyaluminoxytriethoxysilane, titanium tetrakis(trimethylsiloxide), titanium tetrakis(triethylsiloxide), and tetramethylsilane. One of these compounds may be used alone or two or more thereof may be used in any combination. These aids make it easier to improve capacity retention characteristics and cycle characteristics after high-temperature storage.

Of these, phosphorus-containing compounds are preferred as the additional aid, and tris(trimethylsilyl) phosphate and tris(trimethylsilyl) phosphite are preferred.

The aid may be present in any amount that does not significantly impair the effects of the present disclosure. The amount of the aid is preferably 0.01% by mass or more and 5% by mass or less of 100% by mass of the electrolyte solution. An aid in an amount within this range can easily sufficiently exhibit its effects and can easily avoid impairment of battery characteristics such as high-load discharge characteristics. The amount of the aid is more preferably 0.1% by mass or more, still more preferably 0.2% by mass or more, while more preferably 3% by mass or less, still more preferably 1% by mass or less.

The electrolyte solution of the present disclosure may further contain additives such as a cyclic carboxylic acid ester, a chain carboxylic acid ester, an ether compound, a nitrogen-containing compound, a boron-containing compound, an organosilicon-containing compound, a fireproof agent (flame retardant), a surfactant, an additive for increasing permittivity, an improver for cycle characteristics and rate characteristics, and a sulfone-based compound to the extent that the effects of the present disclosure are not impaired.

Examples of the cyclic carboxylic acid ester above include those having a carbon number of 3 to 12 in total in the structural formula. Specific examples include gamma-butyrolactone, gamma-valerolactone, gamma-caprolactone, epsilon-caprolactone, and 3-methyl-γ-butyrolactone. In view of improved electrochemical device characteristics derived from enhanced degree of lithium ion dissociation, gamma-butyrolactone is particularly preferred.

In general, the cyclic carboxylic acid ester as an additive is preferably added in an amount of 0.1% by mass or more, more preferably 1% by mass or more, of 100% by mass of the solvent. A cyclic carboxylic acid ester in an amount within this range can easily improve the electric conductivity of the electrolyte solution, improving the large-current discharge characteristics of an electrochemical device. The amount of the cyclic carboxylic acid ester is also preferably 10% by mass or less, more preferably 5% by mass or less. Such an upper limit makes it easier to allow the electrolyte solution to have a viscosity within an appropriate range, avoid a decrease in the electric conductivity, limit an increase in the resistance of the negative electrode, and allow an electrochemical device to have large-current discharge characteristics within a favorable range.

The cyclic carboxylic acid ester to be suitably used may also be a fluorinated cyclic carboxylic acid ester (fluorinated lactone). Examples of fluorinated lactones include a fluorinated lactone represented by the following formula (C):

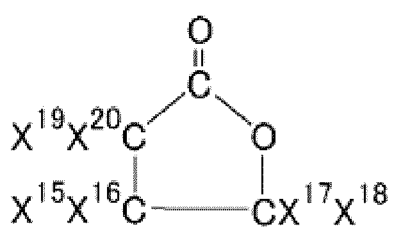

(C)

(wherein $X^{15}$ to $X^{20}$ are the same as or different from each other and are each -H, -F, -Cl, $-CH_3$, or a fluorinated alkyl group, with the proviso that at least one selected from $X^{15}$ to $X^{20}$ is a fluorinated alkyl group).

Examples of the fluorinated alkyl group represented by $X^{15}$ to $X^{20}$ include $-CFH_2$, $-CF_2H$, $-CF_3$, $-CH_2CF_3$, $-CF_2CF_3$, $-CH_2CF_2CF_3$, and $-CF(CF_3)_2$. In view of high oxidation resistance and improved safety, $-CH_2CF_3$ and $-CH_2CF_2CF_3$ are preferred.

Only one of $X^{15}$ to $X^{20}$ or a plurality thereof may be replaced by -H, -F, -Cl, $-CH_3$, or a fluorinated alkyl group as long as at least one of $X^{15}$ to $X^{20}$ is a fluorinated alkyl group. In view of good solubility of an electrolyte salt, preferably 1 to 3, more preferably 1 or 2 of $X^{15}$ to $X^{20}$ are -H, -F, -Cl, $-CH_3$, or a fluorinated alkyl group.

The substitution with the fluorinated alkyl group may be at any of the above sites. In view of a good synthesis yield, the substitution site is preferably $X^{17}$ and/or $X^{18}$. In particular, $X^{17}$ or $X^{18}$ is preferably a fluorinated alkyl group, especially - $CH_2CF_3$ or $-CH_2CF_2CF_3$. The substituent for $X^{15}$ to $X^{20}$ other than the fluorinated alkyl group is -H, -F, -Cl, or $CH_3$. In view of the good solubility of an electrolyte salt, -H is preferred.

In addition to those represented by the above formula, the fluorinated lactone may also be a fluorinated lactone represented by the following formula (D):

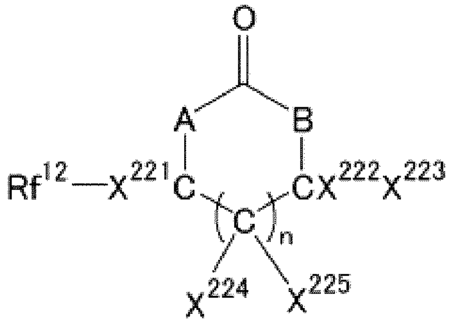
(D)

(wherein one of A or B is $CX^{226}X^{227}$ (wherein $X^{226}$ and $X^{227}$ are the same as or different from each other and are each -H, -F, -Cl, $-CF_3$, $-CH_3$, or an alkylene group having its hydrogen atom optionally replaced by a halogen atom and/or optionally containing a heteroatom in its chain) and the other is an oxygen atom; $Rf^{12}$ is a fluorinated alkyl group optionally containing an ether bond or a fluorinated alkoxy group; $X^{221}$ and $X^{222}$ are the same as or different from each other and are each -H, -F, -Cl, $-CF_3$, or CH3 ; $X^{223}$ to $X^{225}$ are the same as or different from each other and are each -H, -F, -Cl, or an alkyl group having its hydrogen atom optionally replaced by a halogen atom and/or optionally containing a heteroatom in its chain; and n = 0 or 1).

A preferred example of the fluorinated lactone represented by formula (D) is a 5-membered ring structure represented by the following formula (E):

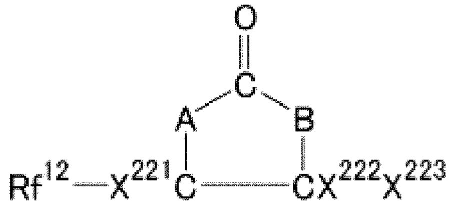
(E)

(wherein A, B, $Rf^{12}$, $X^{221}$, $X^{222}$, and $X^{223}$ are defined as in formula (D)) in view of simple synthesis and good chemical stability. Further, in regards to the combination of A and B, the following are also preferred examples of the fluorinated lactone represented by formula (D):

A fluorinated lactone represented by the following formula (F):

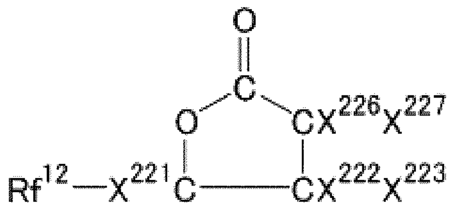
(F)

(wherein $Rf^{12}$, $X^{221}$, $X^{222}$, $X^{223}$, $X^{226}$, and $X^{227}$ are defined as in formula (D)); and
A fluorinated lactone represented by the following formula (G):

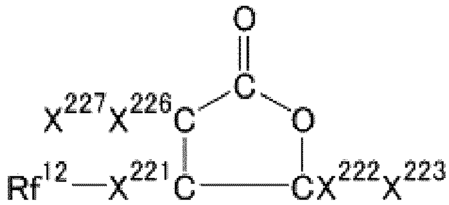

(G)

(wherein $Rf^{12}$, $X^{221}$, $X^{222}$, $X^{223}$, $X^{226}$, and $X^{227}$ are defined as in formula (D)).

Of these, in view of excellent characteristics such as high permittivity and high withstand voltage, and improved characteristics of the electrolyte solution of the present disclosure such as good solubility of an electrolyte salt and decreased internal resistance, the following are also preferred examples of the fluorinated lactone represented by formula (D):

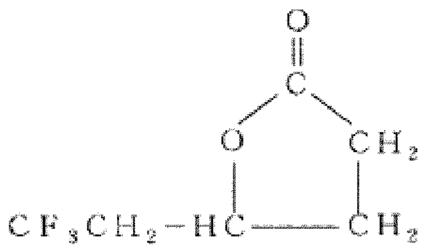 、 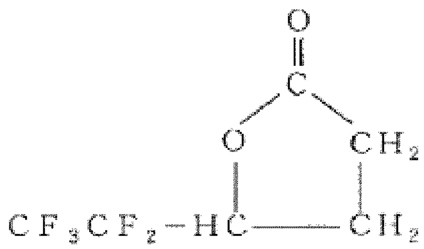 、

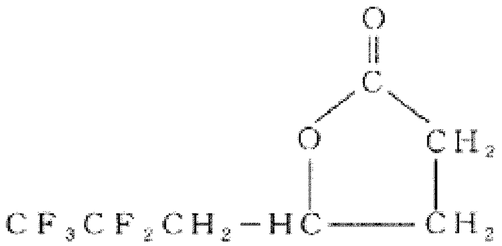 、 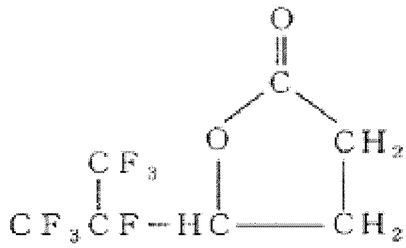

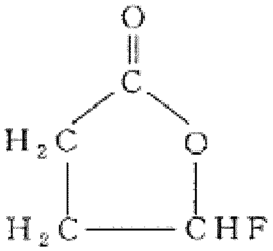 、 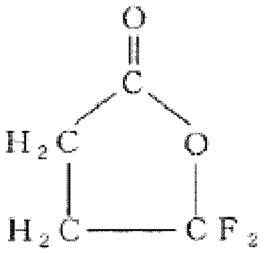 、 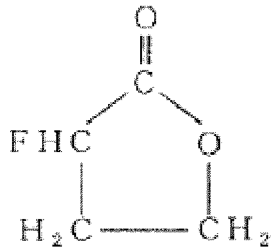 、

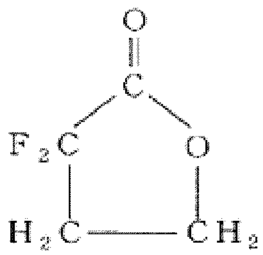 、 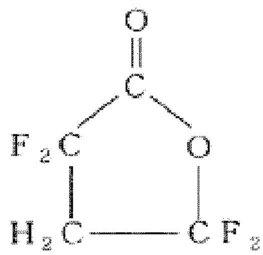

The addition of a fluorinated cyclic carboxylic acid ester makes it easier to improve, for example, ion conductivity, safety, and stability at high temperatures.

Examples of chain carboxylic acid esters include those having a carbon number of 3 to 7 in total in the structural formula thereof. Specific examples include methyl acetate, ethyl acetate, n-propyl acetate, isopropyl acetate, n-butyl acetate, isobutyl acetate, t-butyl acetate, methyl propionate, ethyl propionate, n-propyl propionate, isobutyl propionate, n-butyl propionate, methyl butyrate, isobutyl propionate, t-butyl propionate, methyl butyrate, ethyl butyrate, n-propyl butyrate, isopropyl butyrate, methyl isobutyrate, ethyl isobutyrate, n-propyl isobutyrate, and isopropyl isobutyrate.

In view of improved ion conductivity due to decreased viscosity, methyl acetate, ethyl, n-propyl acetate, n-butyl acetate, methyl propionate, ethyl propionate, n-propyl propionate, isopropyl propionate, methyl butyrate, and ethyl butyrate are preferred.

The ether compound is preferably a $C_{2-10}$ chain ether or a $C_{3-6}$ cyclic ether.

Examples of $C_{2-10}$ chain ethers include dimethyl ether, diethyl ether, di-n-butyl ether, dimethoxymethane, methoxyethoxymethane, diethoxymethane, dimethoxyethane, methoxyethoxyethane, diethoxyethane, ethylene glycol di-n-propyl ether, ethylene glycol di-n-butyl ether, diethylene glycol, diethylene glycol dimethyl ether, pentaethylene glycol, triethylene glycol dimethyl ether, triethylene glycol, tetraethylene glycol, tetraethylene glycol dimethyl ether, and diisopropyl ether.

Further, the ether compound may also suitably be a fluorinated ether.

An example of fluorinated ethers is a fluorinated ether (I) represented by the following general formula (I):

$$Rf^3\text{-O-}Rf^4 \qquad (I)$$

(wherein $Rf^3$ and $Rf^4$ are the same as or different from each other and are each a $C_{1-10}$ alkyl group or a $C_{1-10}$ fluorinated alkyl group; and at least one selected from the group consisting $Rf^3$ and $Rf^4$ is a fluorinated alkyl group). The presence of the fluorinated ether (I) makes it easier to improve incombustibility of the electrolyte solution as well as to improve stability and safety at high temperature under high voltage.

In general formula (I), at least one selected from the group consisting $Rf^3$ and $Rf^4$ is a $C_{1-10}$ fluorinated alkyl group. In view of allowing the electrolyte solution to have further improved incombustibility and further improved stability and safety at high temperature under high voltage, both $Rf^3$ and $Rf^4$ are preferably a $C_{1-10}$ fluorinated alkyl group. In this case, $Rf^3$ and $Rf^4$ may be the same as or different from each other.

In particular, due to the ease of optimizing the boiling point of the fluorinated ether and excellence in solubility of the electrolyte salt and compatibility with other solvents, it is preferred that $Rf^3$ and $Rf^4$ are the same as or different from each other, and $Rf^3$ is a $C_{3-6}$ fluorinated alkyl group and $Rf^4$ is a $C_{2-6}$ fluorinated alkyl group.

If the sum of the carbon numbers of $Rf^3$ and $Rf^4$ is overly small, the fluorinated ether may have an overly low boiling point. An overly large carbon number of $Rf^3$ and $Rf^4$ may cause low solubility of an electrolyte salt, may start to adversely affect the compatibility with other solvents, and may cause high viscosity, resulting in poor rate characteristics. A fluorinated ether (I) wherein the carbon number of $Rf^3$ is 3 or 4 and the carbon number of $Rf^4$ is 2 or 3 is advantageous in terms of its excellent boiling point and rate characteristics.

The fluorinated ether (I) preferably has a fluorine content of 40 to 75% by mass. The fluorinated ether (I) having a fluorine content within this range is likely to have particularly excellent balance between non-flammability and compatibility. The above range is also preferred from the viewpoint of good oxidation resistance and safety.

The lower limit of the fluorine content is more preferably 45% by mass, still more preferably 50% by mass, particularly preferably 55% by mass. The upper limit thereof is more preferably 70% by mass, still more preferably 66% by mass.

The fluorine content of the fluorinated ether (I) is a value calculated based on the structural formula of the fluorinated ether (I) from the following formula:

$$\{(\text{Number of fluorine atoms} \times 19)/(\text{Molecular weight of fluorinated ether (I)})\} \times 100\ (\%\ \text{by mass}).$$

Examples of $Rf^3$ include $CF_3CF_2CH_2$-, $CF_3CFHCF_2$-, $HCF_2CF_2CF_2$-, $HCF_2CF_2CH_2$-, $CF_3CF_2CH_2CH_2$-, $CF_3CFHCF_2CH_2$-, $HCF_2CF_2CF_2CF_2$-, $HCF_2CF_2CF_2CH_2$-, $HCF_2CF_2CH_2CH_2$-, and $HCF_2CF(CF_3)CH_2$-. Examples of $Rf^4$ include $-CH_2CF_2CF_3$, $-CF_2CFHCF_3$, $-CF_2CF_2CF_2H$, $-CH_2CF_2CF_2H$, $-CH_2CH_2CF_2CF_3$, $-CH_2CF_2CFHCF_3$, $-CF_2CF_2CF_2CF_2H$, $-CH_2CF_2CF_2CF_2H$, $-CH_2CH_2CF_2CF_2H$, $-CH_2CF(CF_3)CF_2H$, $-CF_2CF_2H$, $-CH_2CF_2H$, and $-CF_2CH_3$.

Specific examples of the fluorinated ether (I) include $HCF_2CF_2CH_2OCF_2CF_2H$, $CF_3CF_2CH_2OCF_2CF_2H$, $HCF_2CF_2CH_2OCF_2CFHCF_3$, $CF_3CF_2CH_2OCF_2CFHCF_3$, $C_6F_{13}OCH_3$, $C_6F_{13}OC_2H_5$, $C_8F_{17}OCH_3$, $C_8F_{17}OC_2H_5$, $CF_3CFHCF_2CH(CH_3)OCF_2CFHCF_3$, $HCF_2CF_2OCH(C_2H_5)_2$, $HCF_2CF_2OC_4H_9$, $HCF_2CF_2OCH_2CH(C_2H_5)_2$, and $HCF_2CF_2OCH_2CH(CH_3)_2$.

In particular, those having $HCF_2$- or $CF_3CFH$- at one or each end can be a fluorinated ether (I) having excellent polarizability and a high boiling point. The boiling point of the fluorinated ether (I) is preferably 67°C to 120°C, more preferably 80°C or higher, still more preferably 90°C or higher.

Such a fluorinated ether (I) may include one or two or more of, for example, $CF_3CH_2OCF_2CFHCF_3$, $CF_3CF_2CH_2OCF_2CFHCF_3$, $HCF_2CF_2CH_2OCF_2CFHCF_3$, $HCF_2CF_2CH_2OCH_2CF_2CF_2H$, $CF_3CFHCF_2CH_2OCF_2CFHCF_3$, $HCF_2CF_2CH_2OCF_2CF_2H$, and $CF_3CF_2CH_2OCF_2CF_2H$.

Due to advantages in terms of its high boiling point, good compatibility with other solvents, and good solubility of an electrolyte salt, the fluorinated ether (I) is preferably at least one selected from the group consisting of $HCF_2CF_2CH_2OCF_2CFHCF_3$ (boiling point: 106°C), $CF_3CF_2CH_2OCF_2CFHCF_3$ (boiling point: 82°C), $HCF_2CF_2CH_2OCF_2CF_2H$ (boiling point: 92°C), and $CF_3CF_2CH_2OCF_2CF_2H$ (boiling point: 68°C), more preferably at least one selected from the group consisting of $HCF_2CF_2CH_2OCF_2CFHCF_3$ (boiling point: 106°C), and $HCF_2CF_2CH_2OCF_2CF_2H$ (boiling point: 92°C).

Examples of $C_{3-6}$ cyclic ethers include 1,2-dioxane, 1,3-dioxane, 2-methyl-1,3-dioxane, 4-methyl-1,3-dioxane, 1,4-dioxane, metaformaldehyde, 2-methyl-1,3-dioxolane, 1,3-dioxolane, 4-methyl-1,3-dioxolane, 2-(trifluoroethyl)dioxolane, 2,2,-bis(trifluoromethyl)-1,3-dioxolane, and fluorinated compounds thereof. In view of their high ability to solvate with lithium ions and increased degree of ion dissociation, dimethoxymethane, diethoxymethane, ethoxymethoxymethane, ethylene glycol n-propyl ether, ethylene glycol di-n-butyl ether, diethylene glycol dimethyl ether, and crown ethers are preferred. In view of their low viscosity and giving high ion conductivity, dimethoxymethane, diethoxymethane, and ethoxymethoxymethane are particularly preferred.

Examples of nitrogen-containing compounds include nitrile, fluorinated nitrile, carboxylic acid amide, fluorinated carboxylic acid amide, sulfonic acid amide, fluorinated sulfonic acid amide, acetamide, and formamide. 1-methyl-2-pyrrolidinone, 1-methyl-2-piperidone, 3-methyl-2-oxazolidinone, 1,3-dimethyl-2-imidazolidinone, and N-methylsuccinimide may also be used. The nitrile compounds represented by general formulas (1a), (1b), and (1c) are not included in the above nitrogen-containing compounds.

Examples of boron-containing compounds include borates such as trimethyl borate and triethyl borate, boric acid ethers, and alkyl borates.

Examples of organosilicon-containing compounds include $(CH_3)_4$-Si, $(CH_3)_3$-Si-Si$(CH_3)_3$, and silicone oil.

Examples of fireproof agents (flame retardants) include phosphates and phosphazene-based compounds. Examples of phosphates include fluorinated alkyl phosphates, non-fluorinated alkyl phosphates, and aryl phosphates. In view of their flame retardant effect brought about in a small amount, fluorinated alkyl phosphates are particularly preferred.

Examples of phosphazene-based compounds include methoxypentafluorocyclotriphosphazene, phenoxypentafluorocyclotriphosphazene, dimethylaminopentafluorocyclotriphosphazene, diethylaminopentafluorocyclotriphosphazene, ethoxypentafluorocyclotriphosphazene, and ethoxyheptafluorocyclotetraphosphazene.

Specific examples of fluorinated alkyl phosphates include the fluorinated dialkyl phosphates disclosed in JPH11-233141 A, the cyclic alkyl phosphates disclosed in JPH11-283669 A, and fluorinated trialkyl phosphates.

Preferred examples of fireproof agents (flame retardants) include $(CH_3O)_3P=O$, $(CF_3CH_2O)_3P=O$, $(HCF_2CH_2O)_3P=O$, $(CF_3CF_2CH_2)_3P=O$, and $(HCF_2CF_2CH_2)_3P=O$.

The surfactant may be any of cationic surfactants, anionic surfactants, nonionic surfactants, and amphoteric surfactants. In view of good cycle characteristics and rate characteristics, the surfactant is preferably one containing a fluorine atom.

Preferred examples of such a surfactant containing a fluorine atom include a fluorinated carboxylic acid salt represented by the following formula (30):

$$Rf^5COO^-M^+ \qquad (30)$$

(wherein $Rf^5$ is a $C_{3-10}$ fluorinated alkyl group optionally containing an ether bond; $M^+$ is $Li^+$, $Na^+$, $K^+$, or $NHR'_3{}^+$, wherein R's are the same as or different from each other and are each H or a $C_{1-3}$ alkyl group), and a fluorinated sulfonic acid salt represented by the following formula (40):

$$Rf^6SO_3^-M^+ \qquad (40)$$

(wherein $Rf^6$ is a $C_{3-10}$ fluorinated alkyl group optionally containing an ether bond; $M^+$ is $Li^+$, $Na^+$, $K^+$, or $NHR'_3{}^+$, wherein R's are the same as or different from each other and are each H or a $C_{1-3}$ alkyl group).

In view of ease of decreasing the surface tension of the electrolyte solution without impairing the charge and discharge cycle characteristics, the surfactant is preferably present in an amount of 0.01 to 2% by mass of the electrolyte

solution.

Examples of additives for increasing permittivity include sulfolane, methylsulfolane, γ-butyrolactone, and γ-valerolactone.

Examples of improvers for cycle characteristics and rate characteristics include methyl acetate, ethyl acetate, tetrahydrofuran, and 1,4-dioxane.

The electrolyte solution of the present disclosure may be combined with a polymer material to form a gelatinous (plasticized), gel electrolyte solution.

Examples of such polymer materials include known polyethylene oxide, polypropylene oxide, and modified products of these (JPH08-222270A, JP 2002-100405A); polyacrylate-based polymers, polyacrylonitrile, and fluororesins such as polyvinylidene fluoride and vinylidene fluoride-hexafluoropropylene copolymers (JPH04-506726T, JPH08-507407T, JPH10-294131A); and composites of any of these fluororesins and any hydrocarbon resin (JPH11-35765A, JPH11-86630A). In particular, polyvinylidene fluoride or a vinylidene fluoride-hexafluoropropylene copolymer is preferably used as a polymer material for gel electrolytes.

The electrolyte solution of the present disclosure may also contain an ion conductive compound disclosed in Japanese Patent Application No. 2004-301934.

This ion conductive compound is an amorphous fluorinated polyether compound having a fluorinated group at a side chain and is represented by the following formula (101):

A-(D)-B (101)

wherein D is represented by the following formula (201):

$$-(D1)_n-(FAE)_m-(AE)_p-(Y)_q- \qquad (201)$$

(wherein D1 is an ether unit containing a fluorinated ether group at a side chain and is represented by the following formula (2a):

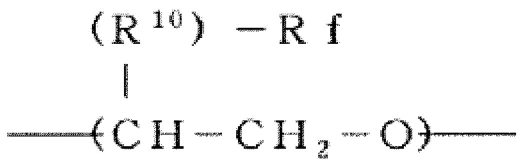

(2a)

(wherein Rf is a fluorinated ether group optionally containing a crosslinkable functional group; and $R^{10}$ is a group or a bond that links Rf and the main chain);

FAE is an ether unit containing a fluorinated alkyl group at a side chain and is represented by the following formula (2b) :

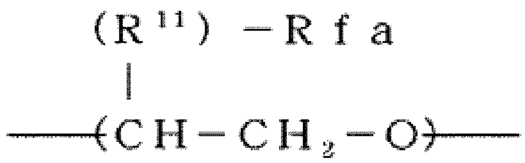

(2b)

(wherein Rfa is a hydrogen atom or a fluorinated alkyl group optionally containing a crosslinkable functional group; and $R^{11}$ is a group or a bond that links Rfa and the main chain);

AE is an ether unit represented by the following formula (2c):

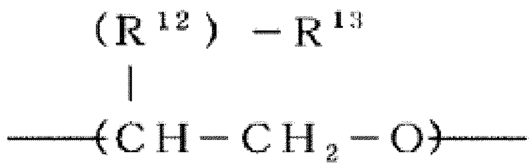

(2c)

(wherein $R^{13}$ is a hydrogen atom, an alkyl group optionally containing a crosslinkable functional group, an aliphatic cyclic hydrocarbon group optionally containing a crosslinkable functional group, or an aromatic hydrocarbon group optionally containing a crosslinkable functional group; and $R^{12}$ is a group or a bond that links $R^{13}$ and the main chain) ;

Y is a unit containing at least one selected from the following formulas (2d-1) to (2d-3):

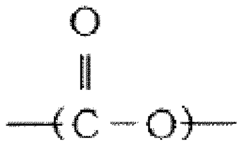

(2d-1)、

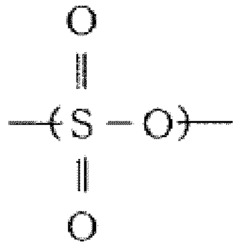

(2d-2)、

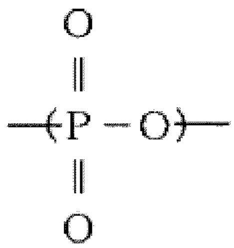

(2d-3)

(wherein n is an integer of 0 to 200; m is an integer of 0 to 200;

p is an integer of 0 to 10000; q is an integer of 1 to 100; n + m is not 0; and the bonding order of D1, FAE, AE, and Y is not specified); and

A and B are the same as or different from each other and are each a hydrogen atom, an alkyl group optionally containing a fluorine atom and/or a crosslinkable functional group, a phenyl group optionally containing a fluorine atom and/or a crosslinkable functional group, a -COOH group, -OR (wherein R is a hydrogen atom or an alkyl group optionally containing a fluorine atom and/or a crosslinkable functional group), an ester group, or a carbonate group, with the proviso that when an end of D is an oxygen atom, A and B are each none of a -COOH group, -OR, an ester group, or a carbonate group).

The electrolyte solution of the present disclosure may contain a sulfone-based compound. The sulfone-based compound is preferably a $C_{3-6}$ cyclic sulfone or a $C_{2-6}$ chain sulfone. The number of sulfonyl groups in one molecule is preferably 1 or 2.

Examples of cyclic sulfones include monosulfone compounds such as trimethylene sulfones, tetramethylene sulfones, and hexamethylene sulfones; disulfone compounds such as trimethylene disulfones, tetramethylene disulfones, and hexamethylene disulfones. In view of good permittivity and viscosity, tetramethylene sulfones, tetramethylene disulfones, hexamethylene sulfones, and hexamethylene disulfones are more preferred, and tetramethylene sulfones (sulfolanes) are particularly preferred.

The sulfolanes are preferably a sulfolane and/or a sulfolane derivative (which may be abbreviated as "sulfolanes" including a sulfolane). The sulfolane derivatives are preferably those in which one or more hydrogen atoms binding to any carbon atom constituting the sulfolane ring are replaced by a fluorine atom or an alkyl group.

In view of high ion conductivity and high input and output, the following are preferred: 2-methylsulfolane, 3-methylsulfolane, 2-fluorosulfolane, 3-fluorosulfolane, 2,2-difluorosulfolane, 2,3-difluorosulfolane, 2,4-difluorosulfolane, 2,5-difluorosulfolane, 3,4-difluorosulfolane, 2-fluoro-3-methylsulfolane, 2-fluoro-2-methylsulfolane, 3-fluoro-3-methylsulfolane, 3-fluoro-2-methylsulfolane, 4-fluoro-3-methylsulfolane, 4-fluoro-2-methylsulfolane, 5-fluoro-3-methylsulfolane, 5-fluoro-2-methylsulfolane, 2-fluoromethylsulfolane, 3-fluoromethylsulfolane, 2-difluoromethylsulfolane, 3-difluoromethylsulfolane, 2-trifluoromethylsulfolane, 3-trifluoromethylsulfolane, 2-fluoro-3-(trifluoromethyl)sulfolane, 3-fluoro-3-(trifluoromethyl)sulfolane, 4-fluoro-3-(trifluoromethyl)sulfolane, 3-sulfolene, 5-fluoro-3-(trifluoromethyl)sulfolane, and the like.

Examples of chain sulfones include dimethyl sulfone, ethyl methyl sulfone, diethyl sulfone, n-propyl methyl

sulfone, n-propyl ethyl sulfone, di-n-propyl sulfone, isopropyl methyl sulfone, isopropyl ethyl sulfone, diisopropyl sulfone, n-butyl methyl sulfone, n-butyl ethyl sulfone, t-butyl methyl sulfone, t-butyl ethyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, perfluoroethyl methyl sulfone, ethyl trifluoroethyl sulfone, ethyl pentafluoroethyl sulfone, di(trifluoroethyl)sulfone, perfluorodiethyl sulfone, fluoromethyl-n-propyl sulfone, difluoromethyl-n-propyl sulfone, trifluoromethyl-n-propyl sulfone, fluoromethyl isopropyl sulfone, difluoromethyl isopropyl sulfone, trifluoromethyl isopropyl sulfone, trifluoroethyl-n-propyl sulfone, trifluoroethyl isopropyl sulfone, pentafluoroethyl-n-propyl sulfone, pentafluoroethyl isopropyl sulfone, trifluoroethyl-n-butyl sulfone, trifluoroethyl-t-butyl sulfone, pentafluoroethyl-n-butyl sulfone, and pentafluoroethyl-t-butyl sulfone.

In view of high ion conductivity and high input and output, the following are preferred: dimethyl sulfone, ethyl methyl sulfone, diethyl sulfone, n-propyl methyl sulfone, isopropyl methyl sulfone, n-butyl methyl sulfone, t-butyl methyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, ethyl trifluoroethyl sulfone, ethyl pentafluoroethyl sulfone, trifluoromethyl-n-propyl sulfone, trifluoromethyl isopropyl sulfone, trifluoroethyl-n-butyl sulfone, trifluoroethyl-t-butyl sulfone, trifluoromethyl-n-butyl sulfone, trifluoromethyl-t-butyl sulfone, and the like.

The sulfone-based compound may be present in any amount that does not significantly impair the effects of the present disclosure. The amount is usually 0.3% by volume or more, preferably 0.5% by volume or more, more preferably 1% by volume or more, while usually 40% by volume or less, preferably 35% by volume or less, more preferably 30% by volume or less, of 100% by volume of the solvent. A sulfone-based compound in an amount within these ranges makes it easier to achieve an effect of improving cycle characteristics and durability such as storage characteristics, allows a non-aqueous electrolyte solution to have a viscosity within an appropriate range, prevents a decrease in electric conductivity, and allows a non-aqueous electrolyte secondary battery to have input and output characteristics and charge and discharge rate characteristics within appropriate ranges.

In view of improved output characteristics, the electrolyte solution of the present disclosure also preferably contains at least one compound selected from the group consisting of lithium fluorophosphates (excluding $LiPF_6$) and lithium salts containing a S=O group as an additive.

When this compound is used as an additive, the above-described electrolyte salt is preferably a compound other than this compound.

Examples of lithium fluorophosphates include lithium monofluorophosphate ($LiPO_3F$) and lithium difluorophosphate ($LiPO_2F_2$).

Examples of lithium salts containing a S=O group include lithium monofluorosulfonate ($FSO_3Li$), lithium methyl sulfate ($CH_3OSO_3Li$), lithium ethyl sulfate ($C_2H_5OSO_3Li$), and lithium 2,2,2-trifluoroethyl sulfate.

Of these, $LiPO_2F_2$, $FSO_3Li$, and $C_2H_5OSO_3Li$ are preferred as such a compound.

In view of electrochemical device performance, the compound is preferably present in an amount of 0.001 to 20% by mass, more preferably 0.01 to 15% by mass, still more preferably 0.1 to 10% by mass, particularly preferably 0.1 to 7% by mass, relative to the electrolyte solution.

The electrolyte solution of the present disclosure may further optionally contain other additives. Examples of such other additives include metal oxides and glass.

The electrolyte solution of the present disclosure preferably contains 5 to 200 ppm of hydrogen fluoride (HF). The presence of HF facilitates the formation of a film of the additives described above. An overly small amount of HF tends to impair the ability of the electrolyte solution to form a film on a negative electrode, impairing the characteristics of an electrochemical device. An overly large amount of HF tends to impair the oxidation resistance of the electrolyte solution due to the influence by HF. The electrolyte solution of the present disclosure, despite containing HF in an amount within the above range, does not decrease the capacity recovery of an electrochemical device stored at high temperature.

The amount of HF is more preferably 10 ppm or more, and still more preferably 20 ppm or more. The amount of HF is also more preferably 100 ppm or less, still more preferably 80 ppm or less, and particularly preferably 50 ppm or less.

The amount of HF can be determined according to neutralization titration.

The electrolyte solution of the present disclosure may be prepared according to any method using the components described above.

The electrolyte solution of the present disclosure can be suitably applied to electrochemical devices such as lithium ion secondary batteries, lithium ion capacitors, hybrid capacitors, and electric double layer capacitors. The following describes a non-aqueous electrolyte battery containing the electrolyte solution of the present disclosure.

The non-aqueous electrolyte battery can be of a known structure, typically including positive and negative electrodes that can occlude and release ions (e.g., lithium ions) and the electrolyte solution of the present disclosure. Such an electrochemical device containing the electrolyte solution of the present disclosure is also one aspect of the

present disclosure.

Examples of the electrochemical devices include lithium ion secondary batteries, lithium ion capacitors, capacitors (hybrid capacitors and electric double layer capacitors), radical batteries, solar cells (in particular, dye-sensitized solar cells), lithium ion primary batteries, fuel cells, various electrochemical sensors, electrochromic elements, electrochemical switching elements, aluminum electrolytic capacitors, and tantalum electrolytic capacitors. Preferred are lithium ion secondary batteries, lithium ion capacitors, and electric double layer capacitors.

A module including the electrochemical device is also one aspect of the present disclosure.

The present disclosure also relates to a lithium ion secondary battery comprising the electrolyte solution of the present disclosure.

The lithium ion secondary battery preferably comprises a positive electrode, a negative electrode, and the electrolyte solution.

Positive Electrode

The positive electrode can include a positive electrode active material layer containing a positive electrode active material, and a current collector.

The positive electrode active material may be any material that can electrochemically occlude and release lithium ions. Examples thereof include lithium-containing transition metal composite oxides, lithium-containing transition metal phosphoric acid compounds, sulfides (sulfur-based materials), and conductive polymers. Preferred among these as the positive electrode active material are lithium-containing transition metal composite oxides and lithium-containing transition metal phosphoric acid compounds. Particularly preferred is a lithium-containing transition metal composite oxide that generates high voltage.

The transition metal of the lithium-containing transition metal composite oxide is preferably V, Ti, Cr, Mn, Fe, Co, Ni, Cu, or the like. Specific examples thereof include lithium-cobalt composite oxides such as $LiCoO_2$, lithium-nickel composite oxides such as $LiNiO_2$, lithium-manganese composite oxides such as $LiMnO_2$, $LiMn_2O_4$, and $Li_2MnO_4$, and those obtained by substituting some of transition metal atoms as main components of these lithium transition metal composite oxides with another element such as Na, K, B, F, Al, Ti, V, Cr, Mn, Fe, Co, Li, Ni, Cu, Zn, Mg, Ga, Zr, Si, Nb, Mo, Sn, or W. Specific examples of those obtained by substitution include $LiNi_{0.5}Mn_{0.5}O_2$, $LiNi_{0.85}Co_{0.10}Al_{0.05}O_2$, $LiNi_{0.5}Co_{0.2}Mn_{0.3}O_2$, $LiNi_{0.6}Co_{0.2}Mn_{0.2}O_2$, $LiNi_{0.33}Co_{0.33}Mn_{0.33}O_2$, $LiNi_{0.45}Co_{0.10}Al_{0.45}O_2$, $LiMn_{1.8}Al_{0.2}O_4$, and $LiMn_{1.5}Ni_{0.5}O_4$.

The lithium-containing transition metal composite oxide is preferably any of $LiMn_{1.5}Ni_{0.5}O_4$, $LiNi_{0.5}Co_{0.2}Mn_{0.3}O_2$, and $LiNi_{0.6}Co_{0.2}Mn_{0.2}O_2$, each of which has a high energy density even at a high voltage. In particular, $LiMn_{1.5}Ni_{0.5}O_4$ is preferred at a high voltage of 4.4 V or higher.

The transition metal of the lithium-containing transition metal phosphoric acid compound is preferably V, Ti, Cr, Mn, Fe, Co, Ni, Cu, or the like. Specific examples thereof include iron phosphates such as $LiFePO_4$, $Li_3Fe_2(PO_4)_3$, and $LiFeP_2O_7$, cobalt phosphates such as $LiCoPO_4$, and those obtained by substituting some of transition metal atoms as main components of these lithium transition metal phosphoric acid compounds with another element such as Al, Ti, V, Cr, Mn, Fe, Co, Li, Ni, Cu, Zn, Mg, Ga, Zr, Nb, or Si.

Examples of the lithium-containing transition metal composite oxide include lithium-manganese spinel composite oxides represented by the formula: $Li_aMn_{2-b}M^1_bO_4$ (wherein $0.9 \le a$; $0 \le b \le 1.5$; and $M^1$ is at least one metal selected from the group consisting of Fe, Co, Ni, Cu, Zn, Al, Sn, Cr, V, Ti, Mg, Ca, Sr, B, Ga, In, Si, and Ge),

> lithium-nickel composite oxides represented by the formula: $LiNi_{1-c}M^2_cO_2$ (wherein $0 \le c \le 0.5$; and $M^2$ is at least one metal selected from the group consisting of Fe, Co, Mn, Cu, Zn, Al, Sn, Cr, V, Ti, Mg, Ca, Sr, B, Ga, In, Si, and Ge), and
> lithium-cobalt composite oxides represented by the formula: $LiCo_{1-d}M^3_dO_2$ (wherein $0 \le d \le 0.5$; and $M^3$ is at least one metal selected from the group consisting of Fe, Ni, Mn, Cu, Zn, Al, Sn, Cr, V, Ti, Mg, Ca, Sr, B, Ga, In, Si, and Ge).

In view of providing a high-power lithium ion secondary battery having a high energy density, preferred is $LiCoO_2$, $LiMnO_2$, $LiNiO_2$, $LiMn_2O_4$, $LiNi_{0.8}Co_{0.15}Al_{0.05}O_2$, or $LiNi_{1/3}Co_{1/3}Mn_{1/3}O_2$.

Other examples of the positive electrode active material include $LiFePO_4$, $LiNi_{0.8}Co_{0.2}O_2$, $Li_{1.2}Fe_{0.4}Mn_{0.4}O_2$, $LiNi_{0.5}Mn_{0.5}O_2$, $LiV_3O_6$, and $Li_2MnO_3$.

An example of the sulfur-based material is a sulfur-containing material. The sulfur-based material is preferably at least one member selected from the group consisting of an elemental sulfur, a metal sulfide, and an organic sulfur compound. The sulfur-based material is more preferably an elemental sulfur. The metal sulfide may be a metal polysulfide. The organic sulfur compound may be an organic polysulfide.

Examples of the metal sulfides include compounds represented by $LiS_x$ ($0<x\le 8$), compounds represented by $Li_2S_x$ ($0<x\le 8$), compounds having a two-dimensional lamellar structure such as $TiS_2$ and $MoS_2$, and Chevrel compounds having a rigid three-dimensional skeletal structure such as those represented by the general formula: $Me_xMo_6S_8$ (wherein

Me is a transition metal such as Pb, Ag, or Cu).

An example of the organic sulfur compound is a carbon sulfide compound.

The organic sulfur compound may be carried on a porous material such as carbon, and used as a carbon composite material. In view of further excellent cycle performance and further reduced overvoltage, the carbon composite material preferably contains sulfur in an amount of 10 to 99% by mass, more preferably 20% by mass or more, still more preferably 30% by mass or more, particularly preferably 40% by mass or more, and preferably 85% by mass or less relative to the carbon composite material.

When the positive electrode active material is an elemental sulfur, the amount of sulfur in the positive electrode active material equals to the amount of the elemental sulfur.

Examples of conductive polymers include p-doped conductive polymers and n-doped conductive polymers. Examples of conductive polymers include polyacetylene-based polymers, polyphenylene-based polymers, heterocyclic polymers, ionic polymers, ladder-shaped polymers, and network polymers.

In view of improving the continuous charge characteristics, the positive electrode active material preferably contains lithium phosphate. Lithium phosphate may be used in any manner, and is preferably used in admixture with the positive electrode active material. The lower limit of the amount of lithium phosphate for use is preferably 0.1% by mass or more, more preferably 0.3% by mass or more, still more preferably 0.5% by mass or more, relative to the sum of the amounts of the positive electrode active material and lithium phosphate. The upper limit thereof is preferably 10% by mass or less, more preferably 8% by mass or less, still more preferably 5% by mass or less.

To a surface of the positive electrode active material may be attached a substance having a formulation different from the positive electrode active material. Examples of the substance attached to the surface include oxides such as aluminum oxide, silicon oxide, titanium oxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; carbonates such as lithium carbonate, calcium carbonate, and magnesium carbonate; and carbon.

Such a substance may be attached to a surface of the positive electrode active material by, for example, a method of dissolving or suspending the substance in a solvent, impregnating the solution or suspension into the positive electrode active material, and drying the impregnated material; a method of dissolving or suspending a precursor of the substance in a solvent, impregnating the solution or suspension into the positive electrode active material, and heating the material and the precursor to cause a reaction therebetween; or a method of adding the substance to a precursor of the positive electrode active material and simultaneously sintering the materials. In the case of attaching carbon, for example, a carbonaceous material in the form of activated carbon may be mechanically attached to the surface afterward.

The lower limit of the amount of the substance attached to the surface in terms of the mass relative to the amount of the positive electrode active material is preferably 0.1 ppm or more, more preferably 1 ppm or more, still more preferably 10 ppm or more, while the upper limit thereof is preferably 20% or less, more preferably 10% or less, still more preferably 5% or less. The substance attached to the surface can reduce oxidation of the electrolyte solution on the surface of the positive electrode active material, improving the battery life. An overly small amount of the substance may fail to sufficiently provide this effect. An overly large amount thereof may hinder the entrance and exit of lithium ions, increasing the resistance.

Particles of the positive electrode active material may have any shape conventionally used, such as a bulky shape, a polyhedral shape, a spherical shape, an ellipsoidal shape, a plate shape, a needle shape, or a pillar shape. The primary particles may agglomerate to form secondary particles.

The positive electrode active material has a tap density of preferably 0.5 g/cm$^3$ or higher, more preferably 0.8 g/cm$^3$ or higher, still more preferably 1.0 g/cm$^3$ or higher. When the positive electrode active material has a tap density below the lower limit, the amount of a dispersion medium, as well as the amounts of a conductive material and a binder, required in the formation of the positive electrode active material layer may increase, which limits the packing fraction of the positive electrode active material in the positive electrode active material layer, resulting in a limitation on the battery capacity. A composite oxide powder having a high tap density enables formation of a positive electrode active material layer with a high density. Typically, a higher tap density is more preferable, and the upper limit is not limited. An excessively high tap density may cause diffusion of lithium ions through an electrolyte solution as a medium in a positive electrode active material layer, which is likely to result in a deterioration of load characteristics. The upper limit is thus preferably 4.0 g g/cm$^3$ or less, more preferably 3.7 g/cm$^3$ or less, further preferably 3.5 g/cm$^3$ or less.

In the present disclosure, the tap density is determined as a powder packing density (tap density) (g/cm$^3$) by placing 5 to 10 g of the positive electrode active material powder in a 10-ml glass graduated cylinder, and tapping the cylinder 200 times with a stroke of about 20 mm.

The particles of the positive electrode active material have a median size d50 (or a secondary particle size when the primary particles agglomerate to form secondary particles) of preferably 0.3 μm or greater, more preferably 0.5 pm or greater, still more preferably 0.8 um or greater, most preferably 1.0 pm or greater, while preferably 30 pm or smaller, more preferably 27 pm or smaller, still more preferably 25 pm or smaller, most preferably 22 pm or smaller. The

particles having a median size below the lower limit may fail to provide a product with a high tap density. The particles having a median size d50 greater than the upper limit may cause prolonged diffusion of lithium in the particles. For example, this can impair the battery performance or form streaks when the positive electrode for a battery is formed, i.e., when the active material and components such as a conductive material and a binder are formed into a slurry with a solvent, and the slurry is applied to form a thin film. Mixing two or more positive electrode active materials having different median sizes d50 can further improve packing properties in the formation of the positive electrode.

In the present disclosure, the median size d50 is determined using a known laser diffraction/scattering particle size distribution analyzer. In the case of using LA-920 (Horiba, Ltd.) as the particle size distribution analyzer, the dispersion medium used in the measurement is a 0.1% by mass sodium hexametaphosphate aqueous solution, and the measurement refractive index is set to 1.24 after 5-minute ultrasonic dispersion.

When the primary particles agglomerate to form secondary particles, the average primary particle size of the positive electrode active material is preferably 0.05 μm or greater, more preferably 0.1 pm or greater, still more preferably 0.2 pm or greater. The upper limit thereof is preferably 5 pm or smaller, more preferably 4 pm or smaller, still more preferably 3 pm or smaller, most preferably 2 pm or smaller. The primary particles having an average primary particle size greater than the upper limit may have difficulty in forming spherical secondary particles, adversely affecting the powder packing properties. Further, such primary particles may have a greatly reduced specific surface area, highly possibly impairing the battery performance, such as output characteristics. In contrast, the primary particles having an average primary particle size below the lower limit may usually be insufficiently grown crystals, causing poor charge and discharge reversibility, for example.

In the present disclosure, the primary particle size is measured by scanning electron microscopic (SEM) observation. Specifically, the primary particle size is determined as follows. A photograph at a magnification of 10000x is first taken. Any 50 primary particles are selected, and the maximum length of a section between the left and right boundary lines of each primary particle along the horizontal line is measured. Then, the average value of the maximum lengths is calculated, which is defined as the primary particle size.

The positive electrode active material has a BET specific surface area of preferably 0.1 $m^2/g$ or larger, more preferably 0.2 $m^2/g$ or larger, still more preferably 0.3 $m^2/g$ or larger. The upper limit thereof is preferably 50 $m^2/g$ or smaller, more preferably 40 $m^2/g$ or smaller, still more preferably 30 $m^2/g$ or smaller. The positive electrode active material having a BET specific surface area smaller than the above range may easily impair the battery performance. The positive electrode active material having a BET specific surface area larger than the above range may less easily have an increased tap density, easily causing difficulty in applying the material in the formation of the positive electrode active material layer.

In the present disclosure, the BET specific surface area is defined by a value determined by single-point BET nitrogen adsorption based on a gas flow method using a surface area analyzer (e.g., a fully automatic surface area measurement device, Ohkura Riken Co., Ltd.), a sample pre-dried in nitrogen stream at 150°C for 30 minutes, and a nitrogen-helium gas mixture with the nitrogen pressure relative to the atmospheric pressure being accurately adjusted to 0.3.

When the lithium ion secondary battery of the present disclosure is used as a large lithium ion secondary battery for hybrid vehicles or for distributed generation, high output is required. Thus, the particles of the positive electrode active material are preferably mainly composed of secondary particles.

The particles of the positive electrode active material preferably include 0.5 to 7.0% by volume of fine particles having an average secondary particle size of 40 um or smaller and having an average primary particle size of 1 pm or smaller. The presence of fine particles having an average primary particle size of 1 um or smaller enlarges the contact area with the electrolyte solution and enables more rapid diffusion of lithium ions between the electrode and the electrolyte solution, improving the output performance of the battery.

The positive electrode active material may be produced by any typical method of producing an inorganic compound. In particular, a spherical or ellipsoidal active material can be produced by various methods. For example, a material substance of transition metal is dissolved, or crushed and dispersed, in a solvent, such as water, and the pH of the solution or dispersion is adjusted under stirring to form a spherical precursor. The precursor is collected and, if necessary, dried. Then, a Li source, such as LiOH, $Li_2CO_3$, or $LiNO_3$, is added thereto, and the mixture is sintered at a high temperature, thereby providing an active material.

In the production of the positive electrode, one of the positive electrode active materials described above may be used alone or two or more thereof having different formulations may be used in any combination at any ratio. Preferred examples of the combination in this case include a combination of $LiCoO_2$ with $LiMn_2O_4$ in which part of Mn is optionally replaced by a different transition metal (e.g., $LiNi_{0.33}Co_{0.33}Mn_{0.33}O_2$), and a combination with $LiCoO_2$ in which part of Co is optionally replaced by a different transition metal.

In view of high battery capacity, the amount of the positive electrode active material is preferably 50 to 99.5% by mass, more preferably 80 to 99% by mass, of the positive electrode mixture. The amount of the positive electrode active material in the positive electrode active material layer is preferably 80% by mass or more, more preferably 82%

by mass or more, particularly preferably 84% by mass or more. The upper limit thereof is preferably 99% by mass or less, more preferably 98% by mass or less. An overly small amount of the positive electrode active material in the positive electrode active material layer may cause an insufficient electric capacity. In contrast, an overly large amount thereof may cause insufficient strength of the positive electrode.

The positive electrode mixture preferably further contains a binder, a thickening agent, and a conductive material.

The binder may be any material that is safe against the solvent and the electrolyte solution used in the production of the electrode. Examples thereof include resin polymers such as polyethylene, polypropylene, polyethylene terephthalate, polymethyl methacrylate, aromatic polyamide, chitosan, alginic acid, polyacrylic acid, polyimide, cellulose, and nitro cellulose; rubbery polymers such as SBR (styrene-butadiene rubber), isoprene rubber, butadiene rubber, fluororubber, NBR (acrylonitrile-butadiene rubber), and ethylene-propylene rubber; styrene-butadiene-styrene block copolymers and hydrogenated products thereof; thermoplastic elastomer polymers such as EPDM (ethylene-propylene-diene terpolymers), styrene-ethylene-butadiene-styrene copolymers, and styrene-isoprene-styrene block copolymers and hydrogenated products thereof; soft resin polymers such as syndiotactic-1,2-polybutadiene, polyvinyl acetate, ethylene-vinyl acetate copolymers, and propylene-$\alpha$-olefin copolymers; fluoropolymers such as polyvinylidene fluoride, polytetrafluoroethylene, vinylidene fluoride copolymer, and tetrafluoroethylene-ethylene copolymers; and polymer compositions having ion conductivity of alkali metal ions (especially, lithium ions). One of these may be used alone or two or more thereof may be used in any combination at any ratio.

The amount of the binder, which is expressed as the proportion of the binder in the positive electrode active material layer, is usually 0.1% by mass or more, preferably 1% by mass or more, more preferably 1.5% by mass or more. The proportion is also usually 80% by mass or less, preferably 60% by mass or less, still more preferably 40% by mass or less, most preferably 10% by mass or less. An overly low proportion of the binder may fail to sufficiently hold the positive electrode active material and cause insufficient mechanical strength of the positive electrode, impairing the battery performance such as cycle characteristics. In contrast, an overly high proportion thereof may cause reduction in battery capacity and conductivity.

Examples of the thickening agent include carboxymethyl cellulose, methyl cellulose, hydroxymethyl cellulose, ethyl cellulose, polyvinyl alcohol, oxidized starch, monostarch phosphate, casein, polyvinylpyrrolidone, and salts thereof. One of these agents may be used alone or two or more thereof may be used in any combination at any ratio.

The proportion of the thickening agent relative to the active material is usually 0.1% by mass or higher, preferably 0.2% by mass or higher, more preferably 0.3% by mass or higher, while usually 5% by mass or lower, preferably 3% by mass or lower, more preferably 2% by mass or lower. The thickening agent at a proportion lower than the above range may result in significantly deteriorated application properties. The thickening agent at a proportion higher than the above range may cause a low proportion of the active material in the positive electrode active material layer, resulting in a low capacity of the battery and high resistance between the positive electrode active materials.

The conductive material may be any known conductive material. Specific examples thereof include metal materials such as copper and nickel, and carbon materials such as graphite, including natural graphite and artificial graphite, carbon black, including acetylene black, Ketjenblack, channel black, furnace black, lamp black, and thermal black, and amorphous carbon, including needle coke, carbon nanotube, fullerene, and VGCF. One of these materials may be used alone or two or more thereof may be used in any combination at any ratio. The conductive material is used in an amount of usually 0.01% by mass or more, preferably 0.1% by mass or more, more preferably 1% by mass or more, while usually 50% by mass or less, preferably 30% by mass or less, more preferably 15% by mass or less, in the positive electrode active material layer. The conductive material in an amount less than the above range may cause insufficient conductivity. In contrast, the conductive material in an amount more than the above range may cause a low battery capacity.

The solvent for forming slurry may be any solvent that can dissolve or disperse therein the positive electrode active material, the conductive material, and the binder, as well as the thickening agent used as appropriate. The solvent may be either an aqueous solvent or an organic solvent. Examples of the aqueous solvent include water and solvent mixtures of an alcohol and water. Examples of the organic solvent include aliphatic hydrocarbons such as hexane; aromatic hydrocarbons such as benzene, toluene, xylene, and methyl naphthalene; heterocyclic compounds such as quinoline and pyridine; ketones such as acetone, methyl ethyl ketone, and cyclohexanone; esters such as methyl acetate and methyl acrylate; amines such as diethylene triamine and N,N-dimethylaminopropylamine; ethers such as diethyl ether, propylene oxide, and tetrahydrofuran (THF); amides such as N-methylpyrrolidone (NMP), dimethyl formamide, and dimethyl acetamide; and aprotic polar solvents such as hexamethylphosphoramide and dimethyl sulfoxide.

Examples of the material of the current collector for a positive electrode include metal materials such as aluminum, titanium, tantalum, stainless steel, and nickel, and alloys thereof; and carbon materials such as carbon cloth and carbon paper. Preferred is a metal material, in particular, aluminum or an alloy thereof.

In the case of a metal material, the current collector may be in the form of metal foil, metal cylinder, metal coil, metal plate, metal thin film, expanded metal, punched metal, metal foam, or the like. In the case of a carbon material, the current collector may be in the form of carbon plate, carbon thin film, carbon cylinder, or the like. Preferred among

these is a metal thin film. The thin film may be in the form of mesh, as appropriate. The thin film may have any thickness, and the thickness is usually 1 pm or greater, preferably 3 μm or greater, more preferably 5 μm or greater, while usually 1 mm or smaller, preferably 100 μm or smaller, more preferably 50 μm or smaller. The thin film having a thickness smaller than the above range may have insufficient strength as a current collector. In contrast, the thin film having a thickness greater than the above range may have poor handleability.

In view of reducing the electric contact resistance between the current collector and the positive electrode active material layer, the current collector also preferably has a conductive aid applied on the surface thereof. Examples of the conductive aid include carbon and noble metals such as gold, platinum, and silver.

The ratio between the thicknesses of the current collector and the positive electrode active material layer may be any value, and the ratio {(thickness of positive electrode active material layer on one side immediately before injection of electrolyte solution)/(thickness of current collector)} is preferably 20 or lower, more preferably 15 or lower, most preferably 10 or lower. The ratio is also preferably 0.5 or higher, more preferably 0.8 or higher, most preferably 1 or higher. The current collector and the positive electrode active material layer showing a ratio higher than the above range may cause the current collector to generate heat due to Joule heating during high-current-density charge and discharge. The current collector and the positive electrode active material layer showing a ratio lower than the above range may cause an increased ratio by volume of the current collector to the positive electrode active material, reducing the battery capacity.

The positive electrode may be produced by a usual method. An example of the production method is a method in which the positive electrode active material is mixed with the aforementioned binder, thickening agent, conductive material, solvent, and other components to form a positive electrode mixture in the form of slurry, and this mixture is then applied to a current collector, dried, and pressed so as to be densified.

The densification may be achieved using a manual press or a roll press, for example. The density of the positive electrode active material layer is preferably 1.5 $g/cm^3$ or higher, more preferably 2 $g/cm^3$ or higher, still more preferably 2.2 $g/cm^3$ or higher, while preferably 5 $g/cm^3$ or lower, more preferably 4.5 $g/cm^3$ or lower, still more preferably 4 $g/cm^3$ or lower. The positive electrode active material layer having a density higher than the above range may cause low permeability of the electrolyte solution toward the vicinity of the interface between the current collector and the active material, and poor charge and discharge characteristics particularly at a high current density, failing to provide high output. The positive electrode active material layer having a density lower than the above range may cause poor conductivity between the active materials and increase the battery resistance, failing to provide high output.

In view of improving the stability at high output and high temperature in the case of using the electrolyte solution of the present disclosure, the area of the positive electrode active material layer is preferably large relative to the outer surface area of an external case of the battery. Specifically, the total area of the positive electrode is preferably 15 times or more, more preferably 40 times or more, greater than the surface area of the external case of the secondary battery. For closed, square-shaped cases, the outer surface area of an external case of the battery herein means the total area calculated from the dimensions of length, width, and thickness of the case portion into which a power-generating element is packed except for a protruding portion of a terminal. For closed, cylinder-like cases, the outer surface area of an external case of the battery herein means the geometric surface area of an approximated cylinder of the case portion into which a power-generating element is packed except for a protruding portion of a terminal. The total area of the positive electrode herein means the geometric surface area of the positive electrode mixture layer facing a mixture layer containing the negative electrode active material. For structures including a current collector foil and positive electrode mixture layers on both sides of the current collector, the total area of the positive electrode is the sum of the areas calculated on the respective sides.

The positive electrode plate may have any thickness. In view of high capacity and high output, the lower limit of the thickness of the mixture layer on one side of the current collector excluding the thickness of the base metal foil is preferably 10 pm or greater, more preferably 20 pm or greater, while preferably 500 pm or smaller, more preferably 450 um or smaller.

To a surface of the positive electrode plate may be attached a substance having a formulation different from the positive electrode plate. Examples of the substance attached to the surface include oxides such as aluminum oxide, silicon oxide, titanium oxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; carbonates such as lithium carbonate, calcium carbonate, and magnesium carbonate; and carbon.

Negative Electrode

The negative electrode includes a negative electrode active material layer containing a negative electrode active material, and a current collector.

The negative electrode material may be any material that can electrochemically occlude and release lithium ions. Specific examples thereof include carbon materials, alloyed materials, lithium-containing metal composite oxide

materials, and conductive polymers. One of these may be used alone or two or more thereof may be used in any combination.

Examples of the negative electrode active material include carbonaceous materials that can occlude and release lithium such as pyrolysates of organic matter under various pyrolysis conditions, artificial graphite, and natural graphite; metal oxide materials that can occlude and release lithium such as tin oxide and silicon oxide; lithium metals; various lithium alloys; and lithium-containing metal composite oxide materials. Two or more of these negative electrode active materials may be used in combination.

The carbonaceous material that can occlude and release lithium is preferably artificial graphite produced by high-temperature treatment of easily graphitizable pitch from various materials, purified natural graphite, or a material obtained by surface treatment on such graphite with pitch or other organic matter, followed by carbonization. In view of excellent balance between the initial irreversible capacity and the high-current-density charge and discharge characteristics, the carbonaceous material is more preferably selected from carbonaceous materials obtained by heat-treating natural graphite, artificial graphite, artificial carbonaceous substances, or artificial graphite substances at 400°C to 3200°C once or more; carbonaceous materials that allow the negative electrode active material layer to include at least two or more carbonaceous matters having different crystallinities and/or have an interface between the carbonaceous matters having the different crystallinities; and carbonaceous materials that allow the negative electrode active material layer to have an interface between at least two or more carbonaceous matters having different orientations. One of these carbonaceous materials may be used alone or two or more thereof may be used in any combination at any ratio.

Examples of the carbonaceous materials obtained by heat-treating artificial carbonaceous substances or artificial graphite substances at 400°C to 3200°C once or more include coal-based coke, petroleum-based coke, coal-based pitch, petroleum-based pitch, and those prepared by oxidizing these pitches; needle coke, pitch coke, and carbon materials prepared by partially graphitizing these cokes; pyrolysates of organic matter such as furnace black, acetylene black, and pitch-based carbon fibers; carbonizable organic matter and carbides thereof; and solutions prepared by dissolving carbonizable organic matter in a low-molecular-weight organic solvent such as benzene, toluene, xylene, quinoline, or n-hexane, and carbides thereof.

The metal material (excluding lithium-titanium composite oxides) for use as the negative electrode active material may be any compound that can occlude and release lithium. Examples thereof include elemental lithium, elemental metals and alloys that constitute lithium alloys, and oxides, carbides, nitrides, silicides, sulfides, and phosphides thereof. The elemental metals and alloys that constitute lithium alloys are preferably materials containing any of metal and semimetal elements in Groups 13 and 14, more preferably elemental metals of aluminum, silicon, and tin (hereinafter, referred to as "specific metal elements"), and alloys and compounds containing any of these atoms. One of these materials may be used alone or two or more thereof may be used in any combination at any ratio.

Examples of negative electrode active materials containing at least one atom selected from the specific metal elements include elemental metals of any one specific metal element, alloys of two or more specific metal elements, alloys of one or two or more specific metal elements and one or two or more other metal elements, compounds containing one or two or more specific metal elements, and composite compounds such as oxides, carbides, nitrides, silicides, sulfides, and phosphides of the compounds. Such an elemental metal, alloy, or metal compound used as the negative electrode active material can lead to a high-capacity battery.

Examples thereof further include compounds in which any of the above composite compounds are complexly bonded with several elements such as elemental metals, alloys, and nonmetal elements. Specifically, in the case of silicon or tin, for example, an alloy of this element and a metal that does not serve as a negative electrode may be used. In the case of tin, for example, a composite compound containing a combination of 5 or 6 elements, including tin, a metal (excluding silicon) that serves as a negative electrode, a metal that does not serve as a negative electrode, and a nonmetal element, may be used.

Specific examples thereof include elemental Si, $SiB_4$, $SiB_6$, $Mg_2Si$, $Ni_2Si$, $TiSi_2$, $MoSi_2$, $CoSi_2$, $NiSi_2$, $CaSi_2$, $CrSi_2$, $Cu_6Si$, $FeSi_2$, $MnSi_2$, $NbSi_2$, $TaSi_2$, $VSi_2$, $WSi_2$, $ZnSi_2$, SiC, $Si_3N_4$, $Si_2N_2O$, $SiO_v$ ($0<v\leq2$), LiSiO, elemental tin, $SnSiO_3$, LiSnO, $Mg_2Sn$, and $SnO_w$ ($0<w\leq2$).

Examples thereof further include composite materials of Si or Sn used as a first constitutional element, and second and third constitutional elements. The second constitutional element is at least one member selected from the group consisting of cobalt, iron, magnesium, titanium, vanadium, chromium, manganese, nickel, copper, zinc, gallium, and zirconium, for example. The third constitutional element is at least one member selected from the group consisting of boron, carbon, aluminum, and phosphorus, for example.

In view of high battery capacity and excellent battery characteristics, the metal material is preferably elemental silicon or tin (which may contain trace impurities), $SiO_v$ ($0<v\leq2$), $SnO_w$ ($0\leq w\leq2$), a Si-Co-C composite material, a Si-Ni-C composite material, a Sn-Co-C composite material, or a Sn-Ni-C composite material.

The lithium-containing metal composite oxide material for use as the negative electrode active material may be any material that can occlude and release lithium. In view of good high-current-density charge and discharge characteristics, materials containing titanium and lithium are preferred, lithium-containing metal composite oxide materials

containing titanium are more preferred, and composite oxides of lithium and titanium (hereinafter, abbreviated as "lithium-titanium composite oxide") are still more preferred. In other words, use of a spinel-structured lithium-titanium composite oxide in the negative electrode active material for an electrolyte battery is particularly preferred because this can markedly reduce the output resistance.

Preferred examples of the lithium titanium composite oxides include compounds represented by the following general formula:

$$Li_xTi_yM_zO_4$$

(wherein M is at least one element selected from the group consisting of Na, K, Co, Al, Fe, Ti, Mg, Cr, Ga, Cu, Zn, and Nb.)

In view of excellent balance of the battery performance, particularly preferred among the formulations above are those satisfying any of the following:

(i) $1.2 \leq x \leq 1.4$, $1.5 \leq y \leq 1.7$, $z=0$
(ii) $0.9 \leq x \leq 1.1$, $1.9 \leq y \leq 2.1$, $z=0$
(iii) $0.7 \leq x \leq 0.9$, $2.1 \leq y \leq 2.3$, $z=0$.

Particularly preferred representative formulations of the compounds are $Li_{4/3}Ti_{5/3}O_4$ in terms of (i), $Li_1Ti_2O_4$ in terms of (ii), and $Li_{4/5}Ti_{11/5}O_4$ in terms of (iii). Preferred examples of the structure satisfying $Z \neq 0$ include $Li_{4/3}Ti_{4/3}Al_{1/3}O_4$.

The negative electrode mixture preferably further contains a binder, a thickening agent, and a conductive material.

Examples of the binder include the same binders as those mentioned for the positive electrode. The proportion of the binder in the negative electrode active material is preferably 0.1% by mass or more, more preferably 0.5% by mass or more, particularly preferably 0.6% by mass or more, while preferably 20% by mass or less, more preferably 15% by mass or less, still more preferably 10% by mass or less, particularly preferably 8% by mass or less. The proportion of the binder in the negative electrode active material higher than the above range may increase the proportion of the binder that does not contribute to the battery capacity, causing a low battery capacity. The proportion of the binder lower than the above range may cause decreased strength of the negative electrode.

In particular, in the case of using a rubbery polymer typified by SBR as a main component, the proportion of the binder in the negative electrode active material is usually 0.1% by mass or more, preferably 0.5% by mass or more, more preferably 0.6% by mass or more, while usually 5% by mass or less, preferably 3% by mass or less, more preferably 2% by mass or less. In the case of using a fluoropolymer typified by polyvinylidene fluoride as a main component, the proportion of the binder in the negative electrode active material is usually 1% by mass or more, preferably 2% by mass or more, more preferably 3% by mass or more, while usually 15% by mass or less, preferably 10% by mass or less, more preferably 8% by mass or less.

Examples of the thickening agent include the same thickening agents as those mentioned for the positive electrode. The proportion of the thickening agent in the negative electrode active material is usually 0.1% by mass or higher, preferably 0.5% by mass or higher, still more preferably 0.6% by mass or higher, while usually 5% by mass or lower, preferably 3% by mass or lower, still more preferably 2% by mass or lower. The proportion of the thickening agent in the negative electrode active material lower than the above range may cause significantly poor application properties. The proportion of the thickening agent higher than the above range may reduce the proportion of the negative electrode active material in the negative electrode active material layer, resulting in a low capacity of the battery and high resistance between the negative electrode active materials.

Examples of the conductive material of the negative electrode include metal materials such as copper and nickel; and carbon materials such as graphite and carbon black.

The solvent for forming slurry may be any solvent that can dissolve or disperse the negative electrode active material and the binder, as well as the thickening agent and the conductive material used as appropriate. The solvent may be either an aqueous solvent or an organic solvent.

Examples of the aqueous solvent include water and alcohols. Examples of the organic solvent include N-methylpyrrolidone (NMP), dimethyl formamide, dimethyl acetamide, methyl ethyl ketone, cyclohexanone, methyl acetate, methyl acrylate, diethyl triamine, N,N-dimethyl aminopropyl amine, tetrahydrofuran (THF), toluene, acetone, diethyl ether, dimethyl acetamide, hexamethylphosphoramide, dimethyl sulfoxide, benzene, xylene, quinoline, pyridine, methyl naphthalene, and hexane.

Examples of the material of the current collector for a negative electrode include copper, nickel, and stainless steel. In view of ease of processing into a thin film, and in view of cost, copper foil is preferred.

The current collector usually has a thickness of 1 pm or greater, preferably 5 μm or greater, while usually 100 μm or smaller, preferably 50 um or smaller. An overly thick negative electrode current collector may cause an excessive reduction in the capacity of the entire battery, while an overly thin current collector may cause difficulty in handling.

The negative electrode may be produced by a usual method. An example of the production method is a method in which the negative electrode material is mixed with the aforementioned binder, thickening agent, conductive material, solvent, and other components to form a mixture in the form of slurry, and this mixture is then applied to a current collector, dried, and pressed so as to be densified. In the case of using an alloyed material, a thin film layer containing the negative electrode active material (negative electrode active material layer) may be formed by vapor deposition, sputtering, plating, or the like.

The electrode formed from the negative electrode active material may have any structure. The negative electrode active material existing on the current collector preferably has a density of 1 $g \cdot cm^{-3}$ or higher, more preferably 1.2 $g \cdot cm^{-3}$ or higher, particularly preferably 1.3 $g \cdot cm^{-3}$ or higher, while preferably 2.2 $g \cdot cm^{-3}$ or lower, more preferably 2.1 $g \cdot cm^{-3}$ or lower, still more preferably 2.0 $g \cdot cm^{-3}$ or lower, particularly preferably 1.9 $g \cdot cm^{-3}$ or lower. The negative electrode active material existing on the current collector having a density higher than the above range may cause destruction of the negative electrode active material particles, resulting in a high initial irreversible capacity and poor high-current-density charge and discharge characteristics due to reduction in permeability of the electrolyte solution toward the vicinity of the interface between the current collector and the negative electrode active material. The negative electrode active material having a density below the above range may cause poor conductivity between the negative electrode active materials, high battery resistance, and a low capacity per unit volume.

The thickness of the negative electrode plate is a design matter determined in accordance with the positive electrode plate to be used, and may be any value. The thickness of the mixture layer excluding the thickness of the base metal foil is usually 15 pm or greater, preferably 20 pm or greater, more preferably 30 pm or greater, while usually 300 um or smaller, preferably 280 μm or smaller, more preferably 250 μm or smaller.

To a surface of the negative electrode plate may be attached a substance having a formulation different from the negative electrode plate. Examples of the substance attached to the surface include oxides such as aluminum oxide, silicon oxide, titanium oxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; and carbonates such as lithium carbonate, calcium carbonate, and magnesium carbonate.

Separator

The lithium ion secondary battery of the present disclosure preferably further includes a separator.

The separator may be formed from any known material and may have any known shape as long as the resulting separator is stable to the electrolyte solution and is excellent in liquid-retaining ability. The separator is preferably in the form of a porous sheet or a nonwoven fabric that is formed from a material stable to the electrolyte solution of the present disclosure, such as resin, glass fiber, or inorganic matter, and that has an excellent liquid-retaining ability.

Examples of the material of a resin or glass-fiber separator include polyolefins such as polyethylene and polypropylene, aromatic polyamide, polytetrafluoroethylene, polyether sulfone, and glass filters. One of these materials may be used alone or two or more thereof may be used in any combination at any ratio, for example, in the form of a polypropylene/polyethylene bilayer film or a polypropylene/polyethylene/polypropylene trilayer film. In view of good permeability of the electrolyte solution and a good shutdown effect, the separator is preferably a porous sheet or a nonwoven fabric formed from a polyolefin such as polyethylene or polypropylene.

The separator may have any thickness, and the thickness is usually 1 pm or greater, preferably 5 pm or greater, more preferably 8 pm or greater, while usually 50 um or smaller, preferably 40 pm or smaller, more preferably 30 pm or smaller. A separator thinner than the above range may have poor insulation and mechanical strength. A separator thicker than the above range may cause not only poor battery performance such as rate characteristics, but also low energy density of the entire electrolyte battery.

When the separator is porous, such as a porous sheet or a nonwoven fabric, the porosity of the separator may be any value. The porosity is usually 20% or higher, preferably 35% or higher, more preferably 45% or higher, while usually 90% or lower, preferably 85% or lower, more preferably 75% or lower. The separator having a porosity lower than the above range tends to have high film resistance, causing poor rate characteristics. A separator having a porosity higher than the above range tends to have low mechanical strength, causing poor insulation.

The separator may also have any average pore size. The average pore size is usually 0.5 μm or smaller, preferably 0.2 um or smaller, while usually 0.05 μm or larger. A separator having an average pore size larger than the above range easily causes short circuits. A separator having an average pore size smaller than the above range may have high film resistance, causing poor rate characteristics.

Examples of the material of the inorganic matter include oxides such as alumina and silicon dioxide, nitrides such as aluminum nitride and silicon nitride, and sulfates such as barium sulfate and calcium sulfate, each in the form of particles or fibers.

The separator is in the form of a thin film such as a nonwoven fabric, a woven fabric, or a microporous film. The thin film for use preferably has a pore size of 0.01 to 1 um and a thickness of 5 to 50 um. Instead of the individual

thin film, the separator may be in the form of a composite porous layer containing particles of the inorganic matter, the composite porous layer being formed on the surface layer of the positive electrode and/or the negative electrode using a resin binder. For example, alumina particles having a 90% particle size of smaller than 1 um may be applied to both surfaces of the positive electrode using a fluororesin as a binder to form a porous layer.

Battery Design

The electrode group may have a laminate structure including the positive and negative electrode plates with the separator in between, or a wound structure including the positive and negative electrode plates in spiral with the separator in between. The proportion of the volume of the electrode group in the battery internal volume (hereinafter, referred to as an electrode group proportion) is usually 40% or higher, preferably 50% or higher, while usually 90% or lower, preferably 80% or lower.

An electrode group proportion lower than the above range reduces the battery capacity. An electrode group proportion higher than the above range reduces the void space in the battery. Thus, when the battery temperature rises to a high temperature, causing the battery components to undergo swelling or causing the vapor pressure of the liquid components of the electrolyte to increase, the internal pressure may increase, which possibly results in deterioration of battery characteristics, such as charge and discharge repeatability and high-temperature storageability, and further results in actuation of a gas-releasing valve for releasing the internal pressure toward the outside.

The current collecting structure may be any structure. In order to more effectively improve the high-current-density charge and discharge characteristics by the electrolyte solution of the present disclosure, the current collecting structure is preferably a structure that reduces the resistances at wiring portions and jointing portions. When the internal resistance is reduced accordingly, the effects achieved with the use of the electrolyte solution of the present disclosure are obtained in a particularly excellent manner.

In an electrode group having the laminate structure, the metal core portions of the electrode layers are preferably bundled and welded to a terminal. If an electrode has a large area, the internal resistance is high. Thus, multiple terminals may preferably be disposed in the electrode so as to reduce the resistance. In an electrode group having the wound structure, multiple lead structures may be disposed on each of the positive electrode and the negative electrode and bundled to a terminal. This can reduce the internal resistance.

The external case may be made of any material that is stable to the electrolyte solution for use. Specific examples thereof include metals such as nickel-plated steel plates, stainless steel, aluminum and aluminum alloys, and magnesium alloys, and a layered film (laminate film) of resin and aluminum foil. In order to reduce the weight, a metal such as aluminum or an aluminum alloy or a laminate film is preferably used.

An external case made of metals may have a sealed-up structure formed by welding the metals by laser welding, resistance welding, or ultrasonic welding, or a caulking structure using the metals with a resin gasket in between. An external case made of a laminate film may have a sealed-up structure formed by hot-melting resin layers. In order to improve the sealability, a resin which is different from the resin of the laminate film may be disposed between the resin layers. In particular, in the case of forming a sealed-up structure by hot-melting the resin layers with current collecting terminals in between, metal and resin are to be bonded. Thus, the resin to be disposed between the resin layers is preferably a resin having a polar group or a modified resin having a polar group introduced therein.

The lithium ion secondary battery of the present disclosure may have any shape, such as a cylindrical shape, a square shape, a laminate shape, a coin shape, or a large shape. The shapes and the structures of the positive electrode, the negative electrode, and the separator may be changed for use in accordance with the shape of the battery.

A module including the lithium ion secondary battery of the present disclosure is also one aspect of the present disclosure.

In a preferred embodiment, the lithium ion secondary battery comprises a positive electrode, a negative electrode, and the electrolyte solution described above, the positive electrode including a positive electrode current collector and a positive electrode active material layer containing a positive electrode active material, the positive electrode active material containing Mn. The lithium ion secondary battery comprising a positive electrode active material layer that contains a positive electrode active material containing Mn can have much better high-temperature storage characteristics.

To provide a high-power lithium ion secondary battery having a high energy density, the positive electrode active material containing Mn is preferably $LiMn_{1.5}Ni_{0.5}O_4$, $LiNi_{0.5}Co_{0.2}Mn_{0.3}O_2$, or $LiNi_{0.6}Co_{0.2}Mn_{0.2}O_2$.

The amount of the positive electrode active material in the positive electrode active material layer is preferably 80% by mass or more, more preferably 82% by mass or more, particularly preferably 84% by mass or more. The upper limit of the amount thereof is preferably 99% by mass or less, more preferably 98% by mass or less. An overly small amount of the positive electrode active material in the positive electrode active material layer may lead to an insufficient electric capacity. In contrast, an overly large amount thereof may lead to insufficient strength of the positive electrode.

The positive electrode active material layer may further contain a conductive material, a thickening agent, and

a binder.

The binder may be any material that is safe against the solvent and the electrolyte solution used in the production of the electrode. Examples thereof include polyvinylidene fluoride, polytetrafluoroethylene, polyethylene, polypropylene, SBR (styrene-butadiene rubber), isoprene rubber, butadiene rubber, ethylene-acrylic acid copolymers, ethylene-methacrylic acid copolymers, polyethylene terephthalate, polymethyl methacrylate, polyimide, aromatic polyamide, cellulose, nitro cellulose, NBR (acrylonitrile-butadiene rubber), fluororubber, ethylene-propylene rubber, styrene-butadiene-styrene block copolymers and hydrogenated products thereof, EPDM (ethylene-propylene-diene terpolymers), styrene-ethylene-butadiene-ethylene copolymers, styrene-isoprene-styrene block copolymers and hydrogenated products thereof, syndiotactic-1,2-polybutadiene, polyvinyl acetate, ethylene-vinyl acetate copolymers, propylene-$\alpha$-olefin copolymers, fluorinated polyvinylidene fluoride, tetrafluoroethylene-ethylene copolymers, and polymer compositions having ion conductivity of alkali metal ions (in particular, lithium ions). One of these substances may be used alone or two or more thereof may be used in any combination at any ratio.

The amount of the binder, which is expressed as the proportion of the binder in the positive electrode active material layer, is usually 0.1% by mass or more, preferably 1% by mass or more, more preferably 1.5% by mass or more. The proportion is also usually 80% by mass or less, preferably 60% by mass or less, still more preferably 40% by mass or less, most preferably 10% by mass or less. An overly low proportion of the binder may fail to sufficiently hold the positive electrode active material and cause insufficient mechanical strength of the positive electrode, impairing the battery performance such as cycle characteristics. In contrast, an overly high proportion thereof may cause reduction in battery capacity and conductivity.

Examples of the thickening agent include carboxymethyl cellulose, methyl cellulose, hydroxymethyl cellulose, ethyl cellulose, polyvinyl alcohol, oxidized starch, monostarch phosphate, casein, and salts thereof. One of these agents may be used alone or two or more thereof may be used in any combination at any ratio.

The proportion of the thickening agent relative to the active material is usually 0.1% by mass or higher, preferably 0.2% by mass or higher, more preferably 0.3% by mass or higher, while usually 5% by mass or lower, preferably 3% by mass or lower, more preferably 2% by mass or lower. The thickening agent at a proportion lower than the above range may result in significantly deteriorated application properties. The thickening agent at a proportion higher than the above range may cause a low proportion of the active material in the positive electrode active material layer, resulting in a low capacity of the battery and high resistance between the positive electrode active materials.

The conductive material may be any known conductive material. Specific examples thereof include metal materials such as copper and nickel, and carbon materials such as graphite, including natural graphite and artificial graphite, carbon black, including acetylene black, and amorphous carbon, including needle coke. One of these materials may be used alone or two or more thereof may be used in any combination at any ratio. The conductive material is used in an amount of usually 0.01% by mass or more, preferably 0.1% by mass or more, more preferably 1% by mass or more, while usually 50% by mass or less, preferably 30% by mass or less, more preferably 15% by mass or less, in the positive electrode active material layer. The conductive material in an amount less than the above range may cause insufficient conductivity. In contrast, the conductive material in an amount more than the above range may cause a low battery capacity.

To further improve the high-temperature storage characteristics, the positive electrode current collector is preferably formed from a valve metal or an alloy thereof. Examples of the valve metal include aluminum, titanium, tantalum, and chromium. The positive electrode current collector is more preferably formed from aluminum or an alloy of aluminum.

To further improve the high-temperature storage characteristics of the lithium ion secondary battery, a portion in contact with the electrolyte solution among portions electrically coupled with the positive electrode current collector is also preferably formed from a valve metal or an alloy thereof. In particular, the external case of the battery and a portion that is electrically coupled with the positive electrode current collector and is in contact with the non-aqueous electrolyte solution among components accommodated in the external case of the battery, such as leads and a safety valve, are preferably formed from a valve metal or an alloy thereof. Stainless steel coated with a valve metal or an alloy thereof may also be used.

The positive electrode may be produced by the method described above. An example of the production method is a method in which the positive electrode active material is mixed with the aforementioned binder, thickening agent, conductive material, solvent, and other components to form a positive electrode mixture in the form of slurry, and this mixture is then applied to a positive electrode current collector, dried, and pressed so as to be densified.

The structure of the negative electrode is as described above.

The electric double layer capacitor may include a positive electrode, a negative electrode, and the electrolyte solution described above.

In the electric double layer capacitor, at least one selected from the group consisting of the positive electrode and the negative electrode is a polarizable electrode. Examples of polarizable electrodes and non-polarizable electrodes include the following electrodes specifically disclosed in JPH09-7896A.

The polarizable electrode mainly containing activated carbon for use in the present disclosure preferably contains inactivated carbon having a large specific surface area and a conductive material, such as carbon black, which imparts electronic conductivity. The polarizable electrode may be formed by a variety of methods. For example, a polarizable electrode including activated carbon and carbon black can be produced by mixing activated carbon powder, carbon black, and phenolic resin, press-molding the mixture, and then sintering and activating the mixture in an inert gas atmosphere and water vapor atmosphere. Preferably, this polarizable electrode is bonded to a current collector using a conductive adhesive, for example.

Alternatively, a polarizable electrode can also be formed by kneading activated carbon powder, carbon black, and a binder in the presence of an alcohol, forming the mixture into a sheet, and then drying the sheet. The binder for use may be polytetrafluoroethylene, for example. Alternatively, a polarizable electrode integrated with a current collector can be produced by mixing activated carbon powder, carbon black, a binder, and a solvent to form slurry, applying this slurry to metal foil of a current collector, and then drying the slurry.

The electric double layer capacitor may include polarizable electrodes mainly containing activated carbon as the electrodes. The electric double layer capacitor may also have a structure in which a non-polarizable electrode is used on one side. Examples of such a structure include a structure in which a positive electrode mainly containing an electrode active material such as a metal oxide is combined with a polarizable negative electrode mainly containing activated carbon; and a structure in which a negative electrode mainly containing a carbon material that can reversibly occlude and release lithium ions or a negative electrode of lithium metal or lithium alloy is combined with a polarizable positive electrode mainly containing activated carbon.

In place of or in combination with activated carbon, any carbonaceous material may be used, such as carbon black, graphite, expanded graphite, porous carbon, carbon nanotube, carbon nanohorn, and Ketjenblack.

The non-polarizable electrode is preferably an electrode mainly containing a carbon material that can reversibly occlude and release lithium ions, with this carbon material made to occlude lithium ions in advance. In this case, the electrolyte used is a lithium salt. The electric double layer capacitor having such a structure can achieve a much higher withstand voltage exceeding 4 V.

The solvent used in preparation of the slurry in production of electrodes is preferably a solvent that dissolves a binder. In accordance with the type of a binder, the solvent is appropriately selected from N-methylpyrrolidone, dimethyl formamide, toluene, xylene, isophorone, methyl ethyl ketone, ethyl acetate, methyl acetate, dimethyl phthalate, ethanol, methanol, butanol, and water.

Examples of the activated carbon used for the polarizable electrode include phenol resin-based activated carbon, coconut shell-based activated carbon, and petroleum coke-based activated carbon. To achieve a large capacity, petroleum coke-based activated carbon or phenol resin-based activated carbon is preferably used. Examples of methods of activating the activated carbon include steam activation and molten KOH activation. To achieve a larger capacity, activated carbon prepared by molten KOH activation is preferably used.

Preferred examples of the conductive agent used for the polarizable electrode include carbon black, Ketjenblack, acetylene black, natural graphite, artificial graphite, metal fiber, conductive titanium oxide, and ruthenium oxide. In view of excellent conductivity (i.e., low internal resistance), and because an overly large amount thereof may lead to decreased capacity of the product, the amount of the conductive agent such as carbon black used for the polarizable electrode is preferably 1 to 50% by mass in the sum of the amounts of the activated carbon and the conductive agent.

In order to provide an electric double layer capacitor having a large capacity and low internal resistance, the activated carbon used for the polarizable electrode preferably has an average particle size of 20 um or smaller and a specific surface area of 1500 to 3000 $m^2/g$. Preferred examples of the carbon material for providing an electrode mainly containing a carbon material that can reversibly occlude and release lithium ions include natural graphite, artificial graphite, graphitized mesocarbon microsphere, graphitized whisker, vapor-grown carbon fiber, sintered furfuryl alcohol resin, and sintered novolak resin.

The current collector may be any current collector that has corrosion resistance in terms of chemically and electrochemically. Preferred examples of the current collector used for the polarizable electrode mainly containing activated carbon include stainless steel, aluminum, titanium, and tantalum. Particularly preferred materials in terms of the characteristics and cost of the resulting electric double layer capacitor are stainless steel and aluminum.

Preferred examples of the current collector used for the electrode mainly containing a carbon material that can reversibly occlude and release lithium ions include stainless steel, copper, and nickel.

Examples of methods of allowing the carbon material that can reversibly occlude and release lithium ions to occlude lithium ions in advance include: (1) a method of mixing powdery lithium to a carbon material that can reversibly occlude and release lithium ions; (2) a method of placing lithium foil on an electrode including a carbon material that can reversibly occlude and release lithium ions and a binder so as to bring the lithium foil to be in electrical contact with the electrode, immersing this electrode in an electrolyte solution containing a lithium salt dissolved therein so as to ionize the lithium, and allowing the carbon material to take in the lithium ions; and (3) a method of placing an electrode including a carbon material that can reversibly occlude and release lithium ions and a binder on the minus side and placing a

lithium metal on the plus side, immersing the electrodes in a non-aqueous electrolyte solution containing a lithium salt as an electrolyte, and supplying a current so that the carbon material is allowed to electrochemically take in the ionized lithium.

Examples of typically known electric double layer capacitors include wound electric double layer capacitors, laminated electric double layer capacitors, and coin-type electric double layer capacitors. The electric double layer capacitor may also be any of these types.

For example, a wound electric double layer capacitor may be assembled as follows. A positive electrode and a negative electrode each of which includes a laminate (electrode) of a current collector and an electrode layer are wound with a separator in between to provide a wound element. This wound element is put into a case made of aluminum, for example. The case is filled with an electrolyte solution, preferably a non-aqueous electrolyte solution, and then sealed with a rubber sealant.

A separator formed from a conventionally known material and having a conventionally known structure may be used. Examples thereof include polyethylene porous membranes, polytetrafluoroethylene, and nonwoven fabric of polypropylene fiber, glass fiber, or cellulose fiber.

In accordance with any known method, the electric double layer capacitor may be prepared in the form of a laminated electric double layer capacitor in which sheet-like positive and negative electrodes are stacked with an electrolyte solution and a separator in between, or in the form of a coin-type electric double layer capacitor in which positive and negative electrodes are fixed in a coin shape with a gasket with an electrolyte solution and a separator in between.

The electrolyte solution of the present disclosure is useful as an electrolyte solution for large lithium ion secondary batteries for hybrid vehicles or distributed generation, and for electric double layer capacitors.

## Examples

The present disclosure will be described in more detail below with reference to Examples. However, the present disclosure is not limited to the Examples.

The term "yield" means molar yield unless otherwise stated.

### Symbols and Abbreviations in the Examples and the Table

Fluorinated Carboxylic Acid Salt Compound

Compound F: $HCF_2COOLi$
Compound G: $CF_3COOLi$
Compound H: $CFH_2COOLi$
Compound I: $CF_3CH_2COOLi$
Compound J: $HCF_2CF_2COOLi$

Carbonate

Chain Carbonate

(a): Dimethyl carbonate
(b): Ethyl methyl carbonate
(c): Diethyl carbonate
(d): $CF_3CH_2OCOOCH_3$

Cyclic Carbonate

EC: Ethylene carbonate
PC: Propylene carbonate
FEC: 4-Fluoro-1,3-dioxolan-2-one

Measurement of Water Content

The water content of lithium difluoroacetate of the Examples and Comparative Example 4 was measured by the Karl Fischer method in a dry room (dew point: -40°C or lower). In Comparative Example 4, a purchased product (lithium difluoroacetate, Apollo Scientific Ltd.) was used.

Example 1: Production of Compound F

Ethyl difluoroacetate (100 g, 0.8065 mol) was added to a reactor and stirred at room temperature. Lithium hydroxide (17.4 g, 0.7258 mol) was added portionwise and stirred at room temperature until the reaction completed. After completion of the reaction, the liquid medium was distilled off from the reaction mixture under reduced pressure to obtain compound F (lithium difluoroacetate) (64.9 g, yield: 88%) (water content: 2340 ppm) as a fluorinated carboxylic acid salt compound.

Example 2: Production of Compound F

Ethyl difluoroacetate (100 g, 0.8065 mol) and ethanol (200 g) were added to a reactor and stirred at room temperature. While the temperature was maintained at 70°C or lower, lithium hydroxide (17.4 g, 0.7258 mol) was added portionwise and stirred at room temperature until the reaction completed. After completion of the reaction, the liquid medium was distilled off from the reaction mixture under reduced pressure to obtain compound F (lithium difluoroacetate) (62.3 g, yield: 85%) (water content: 1462 ppm) as a fluorinated carboxylic acid salt compound.

Examples 3 to 16: Production of Compound F

Compounds F (lithium difluoroacetate) with various water contents as shown in Table 1 were obtained in the same manner as in Example 1. The water content was adjusted by performing azeotropic dehydration and/or addition of a trace amount of water.

Example 17: Production of Compound G

Compound G was produced in the same manner as in Example 2, except that ethyl trifluoroacetate was used instead of ethyl difluoroacetate.

Example 18: Production of Compound H

Compound H was produced in the same manner as in Example 2, except that ethyl monofluoroacetate was used instead of ethyl difluoroacetate.

Example 19: Production of Compound I

Compound I was produced in the same manner as in Example 2, except that ethyl 3-trifluoropropionate was used instead of ethyl difluoroacetate, and tetrahydrofuran was used instead of ethanol.

Example 20: Production of Compound J

Compound J was produced in the same manner as in Example 2, except that ethyl 2-difluoro-3-difluoropropionate was used instead of ethyl difluoroacetate, and acetonitrile was used instead of ethanol.

Test Example

The water content of lithium difluoroacetate of Examples 1 to 21 and Comparative Example 4 was measured by the Karl Fischer method in a dry room (dew point: -40°C or lower). In Comparative Example 4, a purchased product (lithium difluoroacetate, Apollo Scientific Ltd.) was used. The results are shown in the "Water content mass" column in Table 1.

Experiment 1: Lithium Battery Evaluation

The electrolyte solutions of Examples 1 to 21 and the electrolyte solutions of Comparative Examples 1 to 4

were prepared as described below (as shown in Table 1, for Comparative Examples 1 to 3 (reference examples), no fluorinated carboxylic acid salt compound was added), and lithium ion secondary batteries were produced using the obtained electrolyte solutions. Then, the resistance increase rate and cycle capacity retention rate of each of the produced lithium ion secondary batteries were evaluated.

Preparation of Electrolyte Solution

Under a dry argon atmosphere, a chain carbonate and a cyclic carbonate were mixed in the ratios shown in Table 1. To the obtained solution, the fluorinated carboxylic acid salt compound obtained in each of the Examples was added in the amount shown in Table 1. Further, dry $LiPF_6$ was added thereto to achieve a concentration of 1.2 mol/L to obtain a non-aqueous electrolyte solution. The mixing proportion of the fluorinated carboxylic acid salt compound is expressed in percent by mass relative to the electrolyte solution.

Production of Negative Electrode

An artificial graphite powder as a negative electrode active material, an aqueous dispersion of sodium carboxymethyl cellulose (concentration of sodium carboxymethyl cellulose: 1% by mass) as a thickening agent, and an aqueous dispersion of styrene-butadiene rubber (concentration of styrene-butadiene rubber: 50% by mass) as a binder were mixed in an aqueous solvent to prepare a negative electrode mixture in the form of slurry. The ratio on a solids basis of negative electrode active material to thickening agent to binder was 98.1/0.9/1.0 (percent by mass ratio). The obtained slurry was uniformly applied to 20-μm thick copper foil, dried, and then compressed by a press to form a negative electrode.

Production of Positive Electrode

$LiNi_{0.6}Mn_{0.2}Co_{0.2}O_2$ as a positive electrode active material, acetylene black as a conductive material, and a N-methyl-2-pyrrolidone dispersion of polyvinylidene fluoride (PVdF) as a binder were mixed to prepare a positive electrode mixture in the form of slurry. The ratio on a solids basis of positive electrode active material to conductive material to binder was 97/1.5/1.5 (percent by mass ratio). The obtained slurry of positive electrode mixture was uniformly applied to a 20-μm thick aluminum foil current collector, dried, and compressed by a press to form a positive electrode.

Production of Lithium Ion Secondary Battery

The negative electrode and positive electrode produced as described above, as well as a polyethylene separator, were stacked in the order of the negative electrode, the separator, and the positive electrode to produce a battery element.

The battery element was inserted into a bag formed of a laminate film obtained by coating both sides of an aluminum sheet (thickness: 40 um) with a resin layer, with the terminals of the positive electrode and negative electrode protruding. Subsequently, each of the electrolyte solutions of Examples 1 to 22 and Comparative Examples 1 to 3 was injected into the bag, and the bag was vacuum-sealed to produce sheet-like lithium ion secondary batteries.

High-temperature Cycle Capacity Retention Rate

The secondary battery produced above in a state of being sandwiched between plates under pressure was subjected to constant current-constant voltage charge (referred to below as "CC-CV charge") (0.1 C cut off) to 4.2 V at a current corresponding to 0.2 C at 45°C, and then discharged to 3 V at a constant current of 0.2 C. This procedure was counted as one cycle, and the initial discharge capacity was determined based on the discharge capacity at the third cycle. Here, 1 C represents the current value for discharging the reference battery capacity in one hour. For example, 0.2 C represents 1/5 of the current value. The cycles were performed again, and the discharge capacity after the 600th cycle was defined as the capacity after the cycles. The ratio of the discharge capacity after the 600th cycle to the initial discharge capacity was determined, which was defined as the cycle capacity retention rate (%).

(Discharge capacity after 600th cycle)/(Initial discharge capacity)×100= Cycle capacity retention (%)

Resistance Increase Rate

A charge-discharge cycle performed under predetermined charge-discharge conditions (charging at 0.2 C at

a predetermined voltage until the charging current reached 1/10 C and discharging to 3.0 V at a current corresponding to 1 C) was counted as one cycle, and the resistance after the third cycle and the resistance after the 600th cycle were measured. The measurement temperature was 25°C. The resistance increase rate after the 600th cycle was determined according to the following formula.

Table 1 shows the results.

Resistance increase rate after 600th cycle

(%)=Resistance after 600th cycle (Ω)/resistance after third cycle (Ω)×100

Table 1

| | Compound mixed with basic electrolyte solution | | | | | | | Cycle capacity retention (%) | Resistance increase rate (%) |
|---|---|---|---|---|---|---|---|---|---|
| | Fluorinated carboxylic acid salt compound | | | Chain carbonate | | Cyclic carbonate | | | |
| | Compound name or structure | Water content mass (ppm) | Mixing proportion (mass%) | Structure (component) | Mixing proportion (vol%) | Structure (component) | Mixing proportion (vol%) | | |
| Ex 1 | F | 2340 | 1.00 | Component (a) +Component (b) | 30+40 | EC | 30 | 87 | 132 |
| Ex 2 | F | 1462 | 1.00 | Component (a) +Component (b) | 30+40 | EC | 30 | 89 | 133 |
| Ex 3 | F | 25 | 1.00 | Component (a) +Component (b) | 30+40 | EC | 30 | 71 | 170 |
| Ex 4 | F | 4890 | 1.00 | Component (a) +Component (b) | 30+40 | EC | 30 | 86 | 129 |
| Ex 5 | F | 6800 | 1.00 | Component (a) +Component (b) | 30+40 | EC | 30 | 83 | 135 |
| Ex 6 | F | 8900 | 1.00 | Component (a) +Component (b) | 30+40 | EC | 30 | 80 | 151 |
| Ex 7 | F | 15500 | 1.00 | Component (a) +Component (b) | 30+40 | EC | 30 | 77 | 163 |
| Ex 8 | F | 48890 | 1.00 | Component (a) +Component (b) | 30+40 | EC | 30 | 74 | 169 |
| Ex 9 | F | 4890 | 0.05 | Component (a) +Component (b) | 30+40 | EC | 30 | 77 | 145 |
| Ex. 10 | F | 4890 | 0.30 | Component (a) +Component (b) | 30+40 | EC | 30 | 81 | 143 |
| Ex. 11 | F | 4890 | 0.50 | Component (a) +Component (b) | 30+40 | EC | 30 | 85 | 141 |
| Ex. 12 | F | 4890 | 0.70 | Component (a) +Component (b) | 30+40 | EC | 30 | 88 | 130 |
| Ex. 13 | F | 4890 | 1.50 | Component (a) +Component (b) | 30+40 | EC | 30 | 86 | 133 |

(continued)

| | Compound mixed with basic electrolyte solution | | | | | | | Cycle capacity retention (%) | Resistance increase rate (%) |
|---|---|---|---|---|---|---|---|---|---|
| | Fluorinated carboxylic acid salt compound | | | Chain carbonate | | Cyclic carbonate | | | |
| | Compound name or structure | Water content mass (ppm) | Mixing proportion (mass%) | Structure (component) | Mixing proportion (vol%) | Structure (component) | Mixing proportion (vol%) | | |
| Ex. 14 | F | 4890 | 2.00 | Component (a) +Component (b) | 30+40 | EC | 30 | 84 | 138 |
| Ex. 15 | F | 4890 | 1.50 | Component (a) +Component (b) | 30+40 | EC | 30 | 86 | 133 |
| Ex. 16 | F | 2340 | 0.50 | Component (c) +Component (d) | 60+10 | EC+FEC | 25+5 | 92 | 141 |
| Ex. 17 | G | 2460 | 0.50 | Component (c) +Component (d) | 60+10 | EC+FEC | 25+5 | 68 | 152 |
| Ex. 18 | H | 2650 | 0.50 | Component (c) +Component (d) | 60+10 | EC+FEC | 25+5 | 69 | 165 |
| Ex. 19 | I | 2440 | 0.50 | Component (c) +Component (d) | 60+10 | EC+FEC | 25+5 | 74 | 148 |
| Ex. 20 | J | 2910 | 0.50 | Component (c) +Component (d) | 60+10 | EC+FEC | 25+5 | 88 | 149 |
| Ex. 21 | F | 1462 | 1.00 | Component (b) +Component (c) | 40+40 | EC+PC | 16+4 | 81 | 131 |
| Comp. Ex. 1 | - | - | 0.00 | Component (a) +Component (b) | 30+40 | EC | 30 | 61 | 201 |
| Comp. Ex. 2 | - | - | 0.00 | Component (b) +Component (c) | 40+40 | EC+PC | 16+4 | 58 | 223 |
| Comp. Ex. 3 | - | - | 0.00 | Component (c) +Component (d) | 60+10 | EC+FEC | 25+5 | 55 | 211 |
| Comp. Ex. 4 | F | 52000 | 1.00 | Component (a) +Component (b) | 30+40 | EC | 30 | 48 | 221 |

## Claims

1. A method for producing a compound represented by formula (P1): $(B^{1f})_{mp}(A^1)_{np}$, wherein

   $B^{1f}$ is RfCOO,
   Rf is a hydrocarbon having one or more fluorine atoms,
   $A^1$ is a group excluding H,
   mp is the number of (valence of $A^1$) x np/ (valence of $B^{1f}$) and is 1 or 2,
   np is the number of (valence of $B^{1f}$) x mp/ (valence of $A^1$) and is 1,
   $A^1$ has a valence of 1 or 2, and
   $B^{1f}$ has a valence of 1,
   the method comprising step A of reacting
   a compound represented by formula (S1) : $(B^{1f})(R^1)$,
   wherein

      $B^{1f}$ is as defined above, and
      $R^1$ is an organic group,
      with the proviso that $R^1$ is a different group from $A^1$, and

   a compound represented by formula (S2) : $(A^1)_{ms2}(B^2)_{ns2}$,
   wherein

      $A^1$ is as defined above,
      $B^2$ is OH, $CO_3$, or $HCO_3$,
      ms2 is the number of (valence of $B^2$) x ns2/ (valence of $A^1$) and is 1 or 2, and
      ns2 is the number of (valence of $A^1$) x ms2/ (valence of $B^2$) and 1 or 2,

   or a hydrate thereof.

2. The production method according to claim 1, wherein $A^1$ is a metal atom or ammonium.

3. The production method according to claim 1 or 2, wherein the method further comprises step B of removing a compound represented by formula (P2): $(R^1)_{op}(B^2)$, which has been produced as a by-product in step A, wherein

   $B^2$ and $R^1$ are as defined above, and
   op is the number of (valence of $B^2$)/(valence of $R^1$) and
   is 1 or 2.

4. The production method according to any one of claims 1 to 3, wherein Rf is a $C_{1-6}$ alkyl group having one or more fluorine atoms.

5. The production method according to any one of claims 1 to 4, wherein Rf is a $C_{1-3}$ alkyl group having two or more fluorine atoms.

6. The production method according to any one of claims 1 to 5, wherein $A^1$ is an alkali metal atom.

7. The production method according to any one of claims 1 to 6, wherein $A^1$ is Li.

8. The production method according to any one of claims 1 to 7, wherein $B^2$ is OH.

9. An additive for an electrochemical device, comprising a compound represented by formula (P1A): $RfCOOA^d$, wherein

   Rf is an organic group having one or more fluorine atoms, and
   $A^d$ is a metal atom, and

water in an amount of 0 to 50000 mass ppm relative to the compound represented by formula (P1A).

**10.** An electrolyte solution comprising the additive for an electrochemical device according to claim 9.

**11.** An electrochemical device comprising the electrolyte solution according to claim 10.

**12.** A lithium ion secondary battery comprising the electrolyte solution according to claim 10.

**13.** A compound represented by formula (P1A): $RfCOOA^d$, wherein

Rf is an organic group having one or more fluorine atoms, and $A^d$ is a Li atom,
the compound having a water content of 50000 mass ppm or less.

**14.** A composition comprising

a compound represented by formula (P1A): $RfCOOA^d$, wherein
Rf is an organic group having one or more fluorine atoms, and $A^d$ is a Li atom, and
water in an amount of 50000 mass ppm or less relative to the compound represented by formula (P1).

**15.** The compound according to claim 13, which is produced by the production method according to any one of claims 1 to 8.

**16.** The composition according to claim 14, which is produced by the production method according to any one of claims 1 to 8.

## INTERNATIONAL SEARCH REPORT

International application No.
**PCT/JP2021/032577**

**A. CLASSIFICATION OF SUBJECT MATTER**

***C07C 51/09***(2006.01)i; ***C07C 53/18***(2006.01)i; ***C07C 53/21***(2006.01)i; ***H01G 9/035***(2006.01)i; ***H01G 9/20***(2006.01)i; ***H01G 11/06***(2013.01)i; ***H01G 11/64***(2013.01)i; ***H01M 6/16***(2006.01)i; ***H01M 8/02***(2016.01)i; ***H01M 10/052***(2010.01)i; ***H01M 10/0567***(2010.01)i

FI: C07C51/09; C07C53/18; C07C53/21; H01G9/035; H01G9/20 107C; H01G11/06; H01G11/64; H01M6/16 A; H01M8/02; H01M10/052; H01M10/0567

According to International Patent Classification (IPC) or to both national classification and IPC

**B. FIELDS SEARCHED**

Minimum documentation searched (classification system followed by classification symbols)

C07C51/09; C07C53/18; C07C53/21; H01G9/035; H01G9/20; H01G11/06; H01G11/64; H01M6/16; H01M8/02; H01M10/052; H01M10/0567

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN); CASREACT (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2004-22379 A (NEC CORP) 22 January 2004 (2004-01-22)<br>claims, examples | 9-16<br>1-8, 15, 16 |
| X<br>Y | WO 2008/078626 A1 (DAIKIN IND LTD) 03 July 2008 (2008-07-03)<br>paragraph [0055], examples, claims | 9-16<br>1-8, 15, 16 |
| Y | JP 53-146708 A (THE PROCTER & GAMBLE COMPANY) 20 December 1978 (1978-12-20)<br>claims | 1-8, 15, 16 |
| Y | JP 56-125500 A (UNILEVER NV) 01 October 1981 (1981-10-01)<br>claims | 1-8, 15, 16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 October 2021** | **19 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No. **PCT/JP2021/032577**

**C. DOCUMENTS CONSIDERED TO BE RELEVANT**

| Category* | Citation of document, with indication, where appropriate, of the relevant passages | Relevant to claim No. |
|---|---|---|
| A | JP 2011-93808 A (CENTRAL GLASS CO LTD) 12 May 2011 (2011-05-12) claims, paragraphs [0056], [0057] | 1-16 |
| A | WO 2011/000804 A1 (RHODIA OPERATIONS) 06 January 2011 (2011-01-06) REVENDICATIONS | 1-16 |

Form PCT/ISA/210 (second sheet) (January 2015)

**INTERNATIONAL SEARCH REPORT**
**Information on patent family members**

International application No.
**PCT/JP2021/032577**

| Patent document cited in search report | Publication date (day/month/year) | Patent family member(s) | Publication date (day/month/year) |
|---|---|---|---|
| JP 2004-22379 A | 22 January 2004 | (Family: none) | |
| WO 2008/078626 A1 | 03 July 2008 | CN 101584075 A<br>KR 10-2009-0102821 A | |
| JP 53-146708 A | 20 December 1978 | US 4129520 A<br>claims<br>GB 1589314 A<br>DE 2745367 A<br>FR 2367723 A<br>AU 2956477 A | |
| JP 56-125500 A | 01 October 1981 | US 4337209 A<br>claims<br>EP 34047 A1 | |
| JP 2011-93808 A | 12 May 2011 | (Family: none) | |
| WO 2011/000804 A1 | 06 January 2011 | JP 2012-531476 A<br>US 2012/0123159 A1<br>claims<br>EP 2448902 A1<br>FR 2947264 A1<br>CN 102471208 A<br>KR 10-2012-0024850 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004022379 A
- JP 2011093808 A
- JP H11233141 A
- JP H11283669 A
- JP H08222270 A
- JP 2002100405 A
- JP H04506726 T
- JP H08507407 T
- JP H10294131 A
- JP H1135765 A
- JP H1186630 A
- JP 2004301934 A
- JP H097896 A